# DEMANDE DE BREVET EUROPEEN

(11) **EP 0 998 908 A2**
(43) Date de publication de la demande: **10.05.2000**
(21) Numéro de dépôt: 99402549.2
(22) Date de dépôt: 15.10.1999
(51) Int. Cl.: A61K 7/13

(54) **Composition tinctoriale contenant un colorant direct cationique et une pyrazolo-(1,5-a)-pyrimidine à titre de base d'oxydation, et procédés de teinture**

(30) Priorité: 04.11.1998 FR 9813866
(71) Demandeur: L'OREAL, 75008 Paris (FR)
(72) Inventeur: Audousset, Marie-Pascale, 92600 Asnieres (FR)
(74) Mandataire: Goulard, Sophie

(57) **Abrégé**

L'invention a pour objet une composition pour la teinture d'oxydation des fibres kératiniques contenant au moins un colorant direct cationique et au moins une pyrazolo-[1,5-a]-pyrimidine à titre de base d'oxydation; ainsi que le procédé de teinture d'oxydation mettant en oeuvre cette composition.

## Description

L'invention a pour objet une composition pour la teinture d'oxydation des fibres kératiniques contenant au moins un colorant direct cationique et au moins une pyrazolo-[1,5-a]-pyrimidine à titre de base d'oxydation; ainsi que le procédé de teinture d'oxydation mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bis-phénylalkylènediamines ou bien encore des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.
Il a déjà été proposé, en particulier dans la demande de brevet FR-A-2 750 048, d'utiliser des pyrazolo-[1,5-a]-pyrimidines à titre de base d'oxydation, seules ou en association avec un ou plusieurs coupleurs. Cependant, les colorations obtenues ne sont pas toujours assez puissantes, chromatiques ou résistantes aux différentes agressions que peuvent subir les cheveux.

Or, la Demanderesse vient maintenant de découvrir, de façon totalement inattendue et surprenante, que l'association d'au moins une pyrazolo-[1,5-a]-pyrimidine à titre de base d'oxydation et d'au moins un colorant direct cationique ci-après défini permettait d'obtenir des colorations puissantes présentant de plus des propriétés de résistance améliorées vis à vis des diverses agressions que peuvent subir les cheveux (shampooings, lumière, intempéries, ondulations permanentes, transpiration, frottements, etc...).

Ces découvertes sont à la base de la présente invention.

L'invention a donc pour premier objet une composition pour la teinture d'oxydation des fibres kératiniques, et en particulier des fibres kératiniques humaines telles que les cheveux, caractérisée par le fait qu'elle contient, dans un milieu approprié pour la teinture :
- au moins une pyrazolo-[1,5-a]-pyrimidine à titre de base d'oxydation ;
- et au moins un colorant direct cationique ;
ladite composition étant exempte de tout système enzymatique susceptible de provoquer l'oxydation de la ou des pyrazolo-[1,5-a]-pyrimidines.

Comme indiqué précédemment, la composition tinctoriale conforme à l'invention conduit à des colorations puissantes qui présentent de plus d'excellentes propriétés de résistance vis à vis de l'action des différents agents extérieurs (lumière, intempéries, lavage, ondulation permanente, transpiration, frottements).

L'invention a également pour objet un procédé de teinture d'oxydation des fibres kératiniques mettant en oeuvre cette composition tinctoriale.

Les pyrazolo-[1,5-a]-pyrimidines utilisables à titre de base d'oxydation dans la composition tinctoriale conforme à l'invention sont de préférence choisies parmi les composés de formule (V) suivante, et leurs sels d'addition avec un acide ou avec une base : 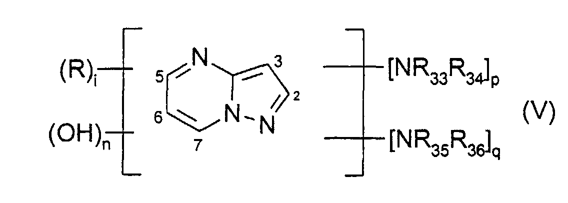 dans laquelle :
- R₃₃, R₃₄ R₃₅ et R₃₆ désignent , identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'aminé pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl-ou di-[hydroxy(C₁-C₄) alkyl]-amino alkyle en C₁-C₄ ;
- les radicaux R, identiques ou différents, désignent un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- (i) la somme p + q est différente de 0 ;
- (ii) lorsque p + q est égal à 2, alors n vaut 0 et les groupes NR₃₃R₃₄ et NR₃₅R₃₆ occupent les positions (2,3) ; (5,6) ; (6,7) ; (3,5) ou (3,7) ;
- (iii) lorsque p + q est égal à 1 alors n vaut 1 et le groupe NR₃₃R₃₄ (ou NR₃₅R₃₆) et le groupe OH occupent les positions (2,3) ; (5,6) ; (6,7) ; (3,5) ou (3,7).

Les pyrazolo-[1,5-a]-pyrimidines de formule (V) ci-dessus sont des composés connus et décrits notamment dans la demande de brevet FR-A-2 750 048 dont le contenu fait partie intégrante de la présente demande.

Parmi les pyrazolo-[1,5-a]-pyrimidines de formule (V), utilisables à titre de base d'oxydation dans les compositions tinctoriales conformes à l'invention, on peut notamment citer :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
- la 3-amino-5-méthyl-7-imidazolylpropylamino pyrazolo-[1,5-a]-pyrimidine ;
et leurs sels d'addition avec un acide ou avec une base.

Le ou les colorants directs cationiques pouvant être utilisés dans la composition tinctoriale conforme à l'invention sont de préférence choisis parmi les amino-anthraquinoniques cationiques, les mono- ou di-azoïques cationiques, et les naphtoquinones cationiques.

A titre de d'exemple, on peut notamment citer le chlorure de [8-[(p-aminophényl)azol]-7-hydroxy-2-naphtyl]triméthylammonium (également appelé Basic Brown 16 ® ou Arianor Mahogany 306002 ® dans le Color Index), l'association du chlorure de 3-[(4-amino-6-bromo-5,8-dihydro-1-hydroxy-8-imino-5-oxo-2-naphtalényl)-amino]-N,N,N-triméthylbenzénaminium et du chlorure de 3-[(2,6-dibromo-5,8-dihydro-1-hydroxy-8-imino-5-oxo-3-naphtyl)-amino]-N,N,N-triméthyl-benzénaminium (également dénommé Basic Blue 99 ® ou Arianor Steel Blue 306004 ® dans le Color Index), le chlorure de 7-hydroxy-8-[(2-méthoxyphényl)azo]-N,N,N-triméthyl-2-naphtalènaminium (également appelé le Basic Red 76 ® ou Arianor Madder Red ® dans le Color Index), le chlorure de [8-[(4-amino-2-nitrophényl)azo]-7-hydroxy-2-naphtyl]triméthylammonium (également appelé Basic Red 118 ® ou Arianor Bordeaux 306006 ® dans le Color Index), l'association du chlorure de [8-[(4-amino-3-nitrophényl)azo]-7-hydroxy-2-naphtyl]triméthylammonium et du chlorure de [8-[(4-amino-2-nitrophényl)azo]-7-hydroxy-2-naphtyl]triméthylammonium (également appelé Basic Brown 17 ® ou Arianor Sienna Brown 306001 ® dans le Color Index), le chlorure de 3-[(4,5-dihydro-3-méthyl-5-oxo-1-phényl-1H-pyrazol-4-yl)azo]-N,N,N-triméthyl-benzènaminium (également appelé Basic Yellow 57 ® ou Arianor Straw Yellow 306005 ® dans le Color Index), le chlorhydrate de 1-(γ-aminopropyl)amino anthraquinone, le méthylsulfate de 1-N-(méthyl-morpholinium-propyl)amino 4-hydroxy anthraquinone, et le Basic Orange 69 ® (dénomination du Color Index).

Le ou les colorants directs cationiques utilisés dans la composition tinctoriale conforme à l'invention peuvent également être choisis parmi les composés de formules (I), (II), (III), (III'), et (IV) suivantes :
**a) composés de formule (I):** 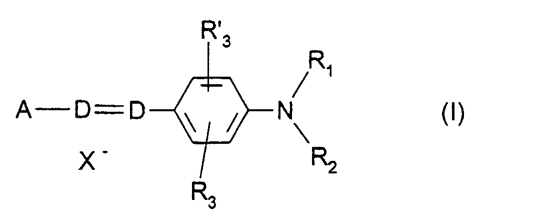 dans laquelle :
   - D représente un atome d'azote ou le groupement -CH,
   - R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   - R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
   - X⁻ représente un anion de préférence choisi parmi le chlorure, le méthyl sulfate et l'acétate,
   - A représente un groupement choisi par les structures A1 à A19 suivantes : 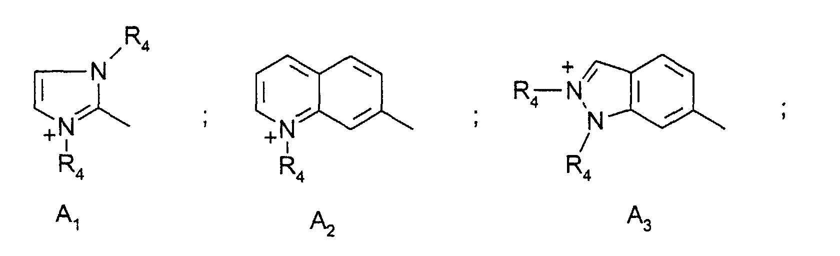 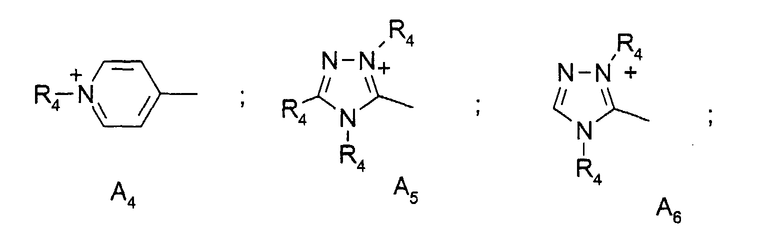 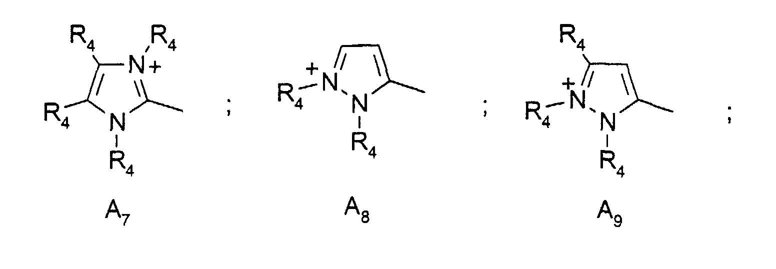 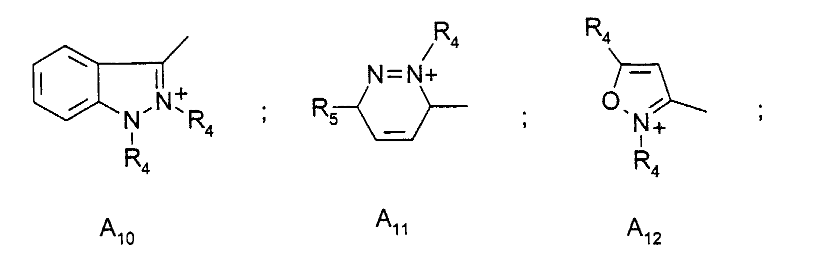 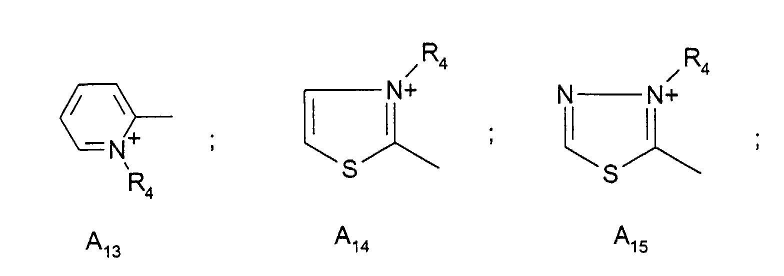 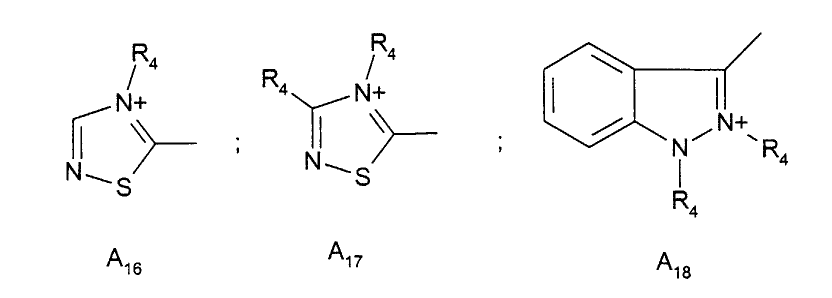 et 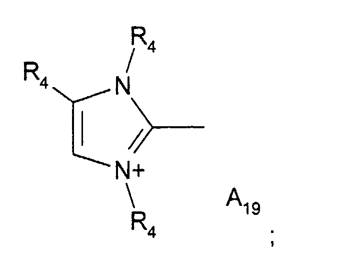 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) composés de formule (II) :** 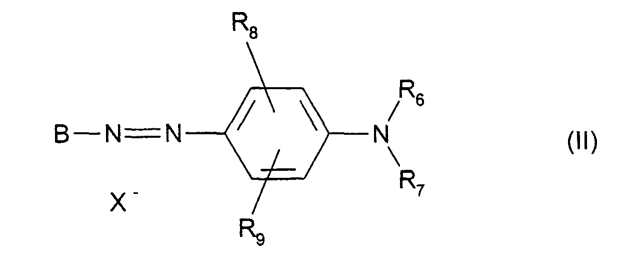 dans laquelle :
   - R₆ représente un atome d'hydrogène ou un radical alkyle en C₁-C₄,
   - R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
   - R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
   - X⁻ représente un anion de préférence choisi parmi le chlorure, le méthyl sulfate et l'acétate,
   - B représente un groupement choisi par les structures B1 à B6 suivantes : 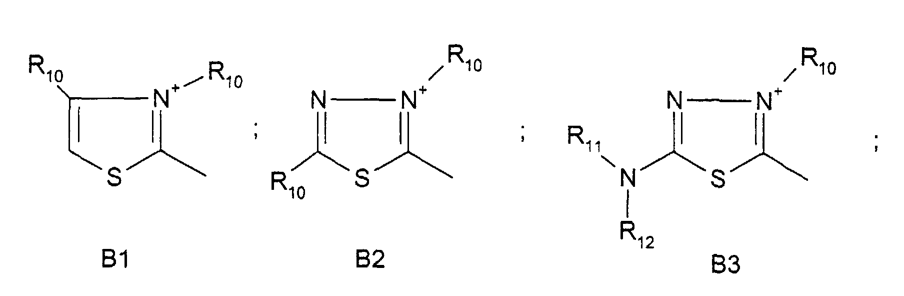 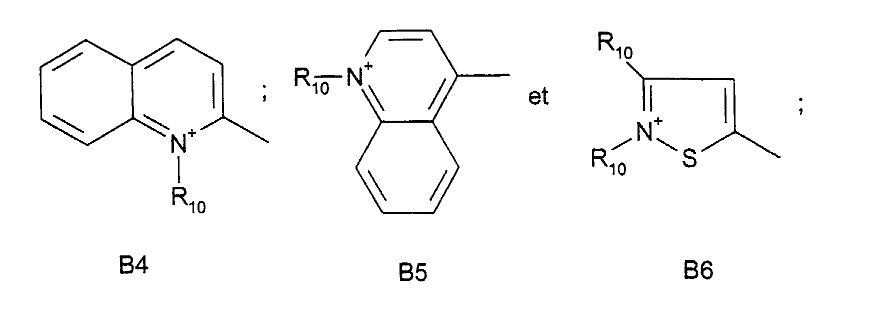 dans lesquelles R₁₀ représente un radical alkyle en C₁-C₄, R₁₁ et R₁₂, identiques ou différents, représentent un atome d'hydrogène ou un radical alkyle en C₁-C₄ ;
**c) composés de formules (III) et (III'):** 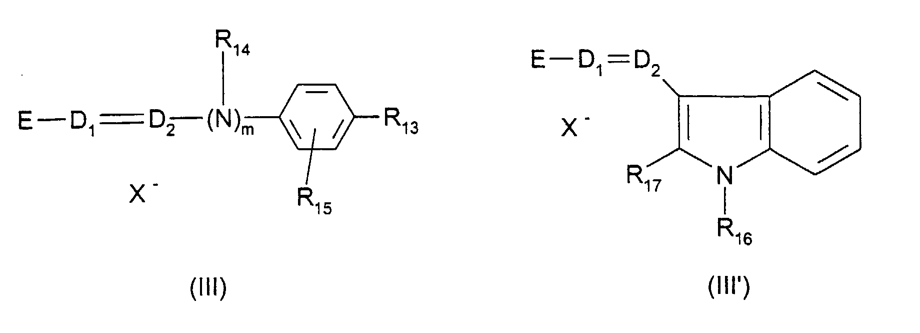 dans lesquelles :
   - R₁₃ représente un atome d'hydrogène, un radical alcoxy en C₁-C₄, un atome d'halogène tel que le brome, le chlore, l'iode ou le fluor ou un radical amino,
   - R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   - R₁₅ représente un atome d'hydrogène ou d'halogène tel que le brome, le chlore, l'iode ou le fluor,
   - R₁₆ et R₁₇, identiques ou différents, représentent un atome d'hydrogène ou un radical alkyle en C₁-C₄,
   - D₁ et D₂, identiques ou différents, représentent un atome d'azote ou le groupement -CH,
   - m = 0 ou 1,
   étant entendu que lorsque R₁₃ représente un groupement amino non substitué, alors D₁ et D₂ représentent simultanément un groupement -CH et m = 0,
   - X⁻ représente un anion de préférence choisi parmi le chlorure, le méthyl sulfate et l'acétate,
   - E représente un groupement choisi par les structures E1 à E8 suivantes : 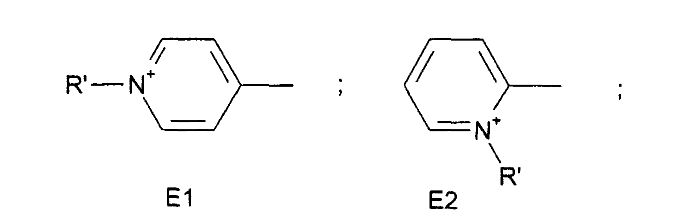 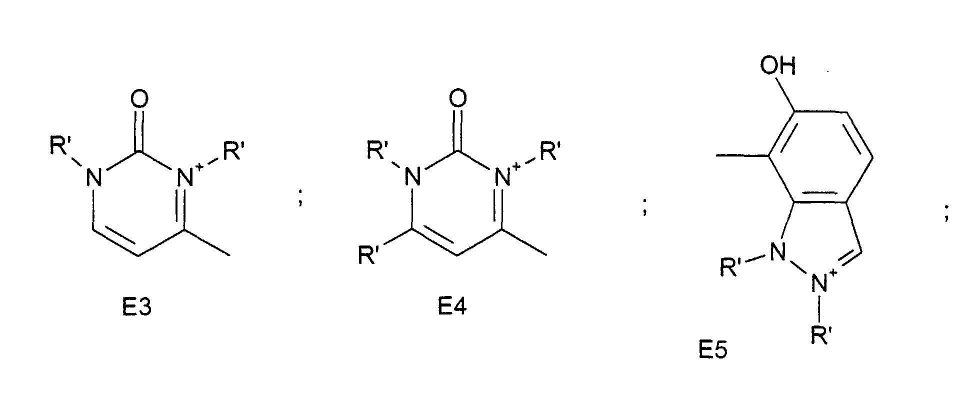 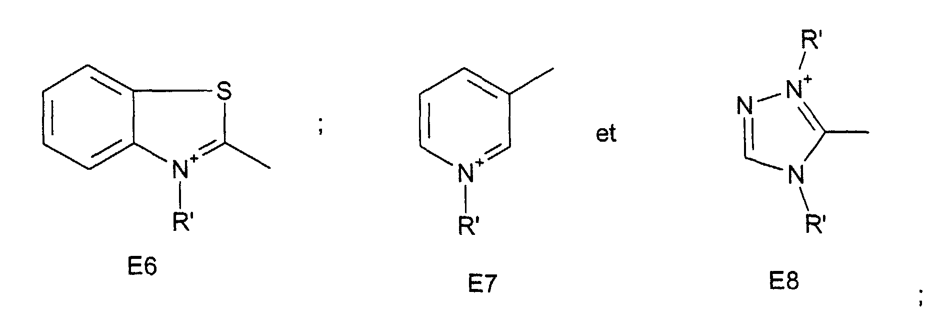 dans lesquelles R' représente un radical alkyle en C₁-C₄ ; lorsque m = 0 et que D₁ représente un atome d'azote, alors E peut également désigner un groupement de structure E9 suivante : 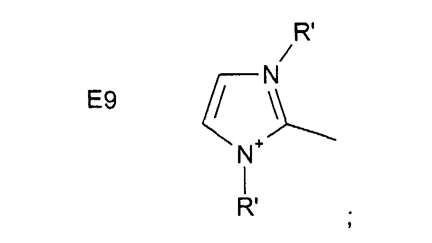 dans laquelle R' représente un radical alkyle en C₁-C₄.
**d) composés de formule (IV) :**

   G―N=N―J (IV)

   dans laquelle :
   - **G** représente un groupement choisi parmi les structures G₁ à G₃ suivantes : 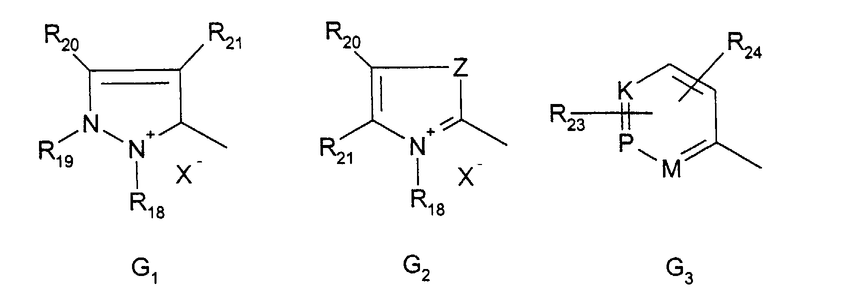 dans lesquelles :
   - R₁₈ désigne un radical alkyle en C₁-C₄, un radical phényle, un radical phényle substitué par un radical alkyle en C₁-C₄, ou un atome d'halogène choisi parmi le chlore, le brome, l'iode et le fluor ;
   - R₁₉ désigne un radical alkyle en C₁-C₄ ou un radical phényle ;
   - R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂ ; R₂₀ peut également désigner un atome d'hydrogène ;
   - Z désigne un atome d'oxygène, de soufre ou un groupement -NR₁₉ ;
   - M représente un groupement -CH, -CR" (R" désignant alkyle en C₁-C₄), ou -NR₂₂(X⁻)ᵣ ;
   - K représente un groupement -CH, -CR" (R" désignant alkyle en C₁-C₄), ou -NR₂₂(X⁻)ᵣ;
   - P représente un groupement -CH, -CR" (R" désignant alkyle en C₁-C₄), ou -NR₂₂(X⁻)ᵣ ; r désigne zéro ou 1 ;
   - R₂₂ représente un atome O⁻, un radical alcoxy en C₁-C₄, ou un radical alkyle en C₁-C₄ ;
   - R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂ ;
   - X⁻ représerte un anion de préférence choisi parmi le chlorure, l'iodure, le méthyl sulfate, l'éthyl sulfate, l'acétate et le perchlorate ;
sous réserve que :
   - lorsque R₂₂ désigne O⁻, alors r désigne zéro ;
   - lorsque K ou P ou M désignent un groupement -N-alkyle en C₁-C₄ X⁻, alors l'un au moins des radicaux R₂₃ et R₂₄ est différent d'un atome d'hydrogène ;
   - lorsque K désigne -NR₂₂(X⁻)ᵣ, alors M = P = -CH ou -CR";
   - lorsque M désigne -NR₂₂(X⁻)ᵣ, alors K = P = -CH ou -CR";
   - lorsque P désigne -NR₂₂(X⁻)ᵣ, alors K = M et désignent -CH ou -CR ;
   - lorsque Z désigne un atome de soufre et que R₂₁ désigne un radical alkyle en C₁-C₄, alors R₂₀ est différent d'un atome d'hydrogène ;
   - lorsque Z désigne -NR₂₂, et que R₁₉ désigne un radical alkyle en C₁-C₄, alors au moins un des radicaux R₁₈, R₁₉ ou R₂₀ du groupement de structure G₂ est différent d'un radical alkyle en C₁-C₄ ;
   - **J** représente :
      - soit un groupement de structure J₁ suivante : 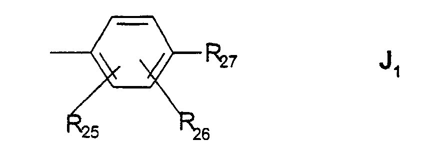 dans laquelle :
         - R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène et le soufre ;
         - R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre ;
         - R₂₇ représente un atome d'hydrogène, un radical -OH, un radical -NHR₂₈, ou un radical -NR₂₉R₃₀ ;
         - R₂₈ représente un atome d'hydrogène, un radical alkyle en C₁-C₄, un radical monohydroxyalkyle en C₁-C₄, polyhydroxyalkyle en C₂-C₄, ou un radical phényle ;
         - R₂₉ et R₃₀, identiques ou différents, représentent un radical alkyle en C₁-C₄, un radical monohydroxyalkyle en C₁-C₄, ou polyhydroxyalkyle en C₂-C₄ ;
      - soit un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 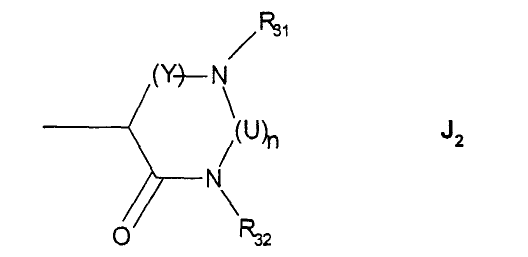 dans laquelle :
         - R₃₁ et R₃₂, identiques ou différents, représentent un atome d'hydrogène, un radical alkyle en C₁-C₄, ou un radical phényle ;
         - Y désigne un radical -CO- ou le radical 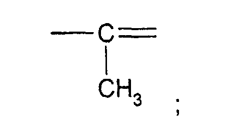
         - n = 0 ou 1, étant entendu que lorsque n = 1, alors U désigne le radical -CO- .

Dans les structures (I) à (IV) définies ci-dessus le groupement alkyle ou alcoxy en C₁-C₄ désigne de préférence méthyle, éthyle, butyle, méthoxy, ou éthoxy.

Les colorants directs cationiques de formules (I), (II), (III) et (III') utilisables dans les compositions tinctoriales conformes à l'invention, sont des composés connus et sont décrits par exemple dans les demandes de brevets WO 95/01772, WO 95/15144 et EP-A-0 714 954. Les colorants directs cationiques de formule (IV) utilisables dans les compositions tinctoriales conformes à l'invention, sont également des composés connus et sont décrits par exemple dans les demandes de brevets FR-A-2 189 006, FR-A-2 285 851, FR-A-2 140 205 et ses certificats d'addition.

Parmi les colorants directs cationiques de formule (I) utilisables dans les compositions tinctoriales conformes à l'invention, on peut plus particulièrement citer les composés répondant aux structures (I1) à (I52) suivantes : 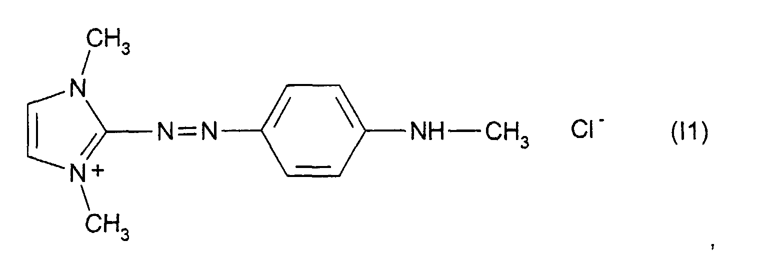 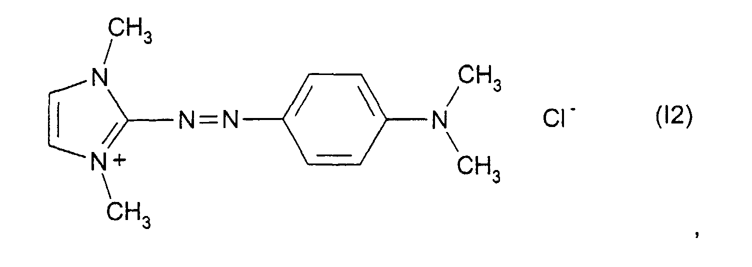 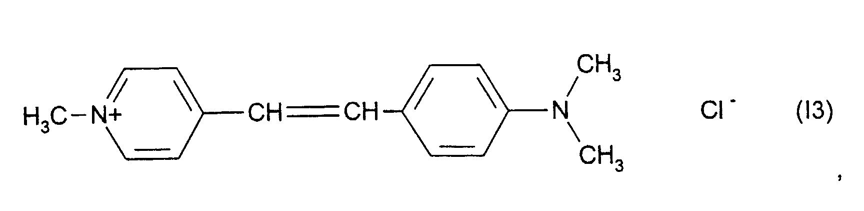 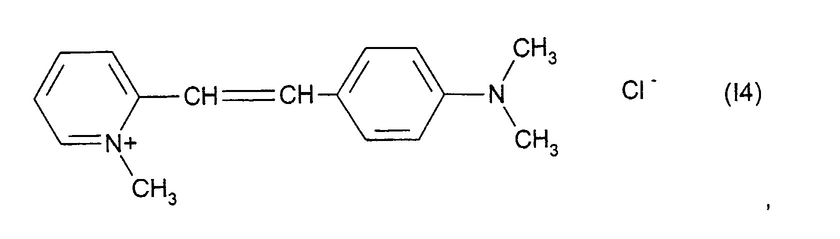 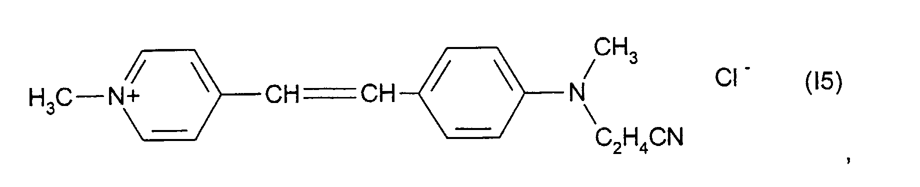 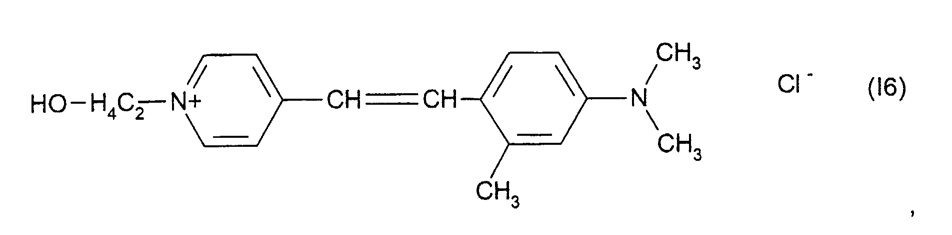 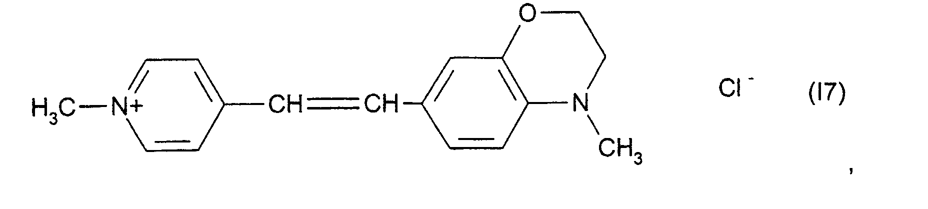 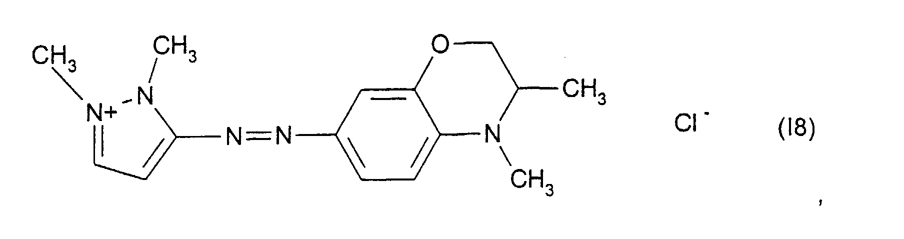 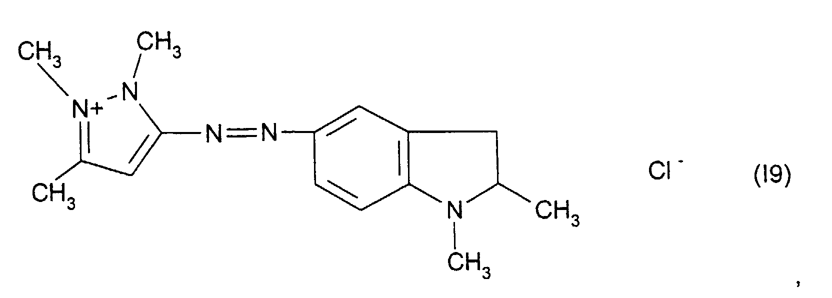 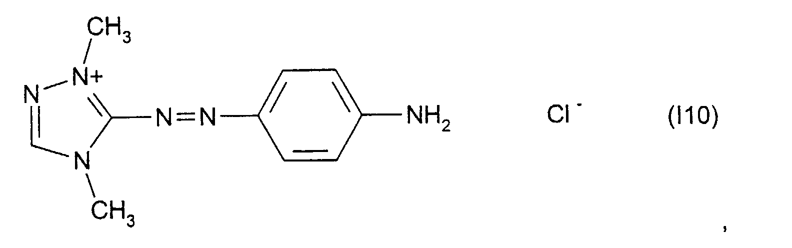 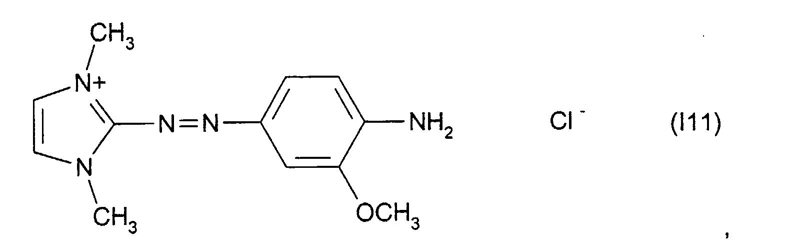 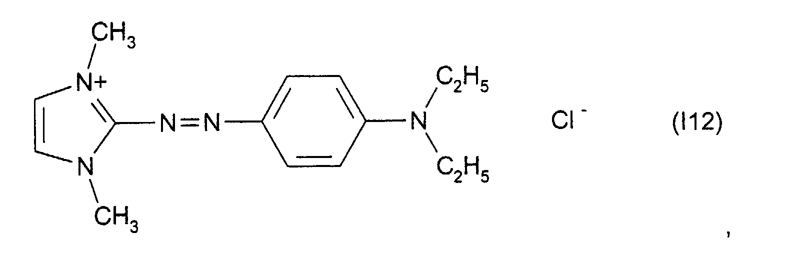 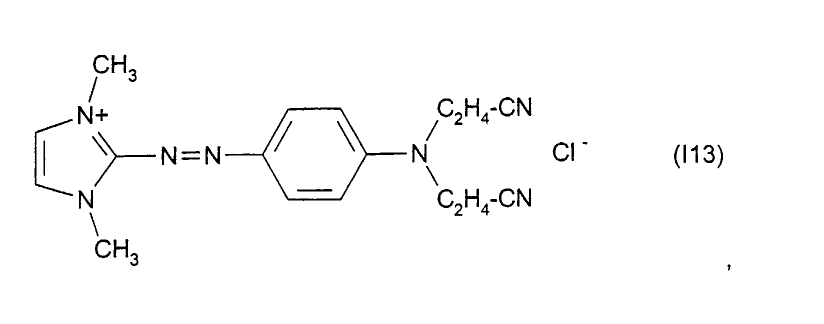 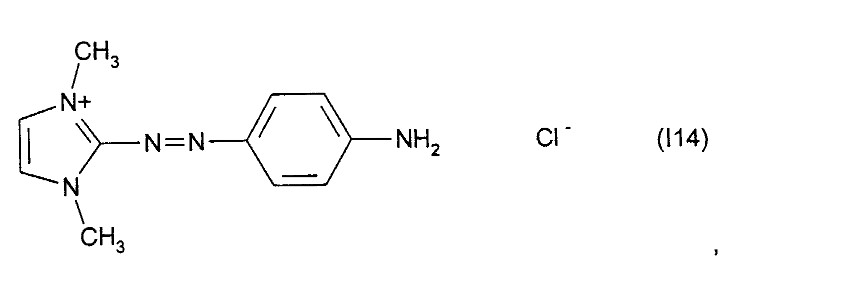 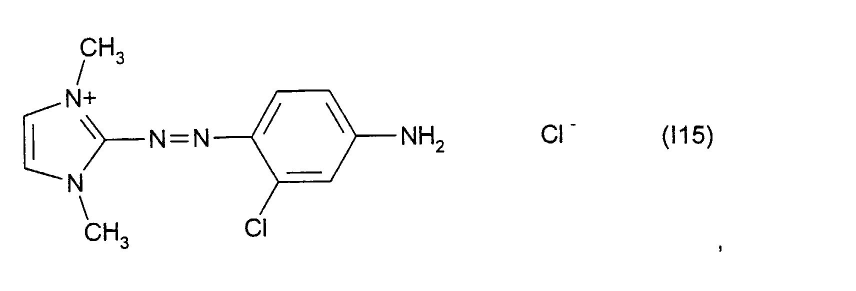 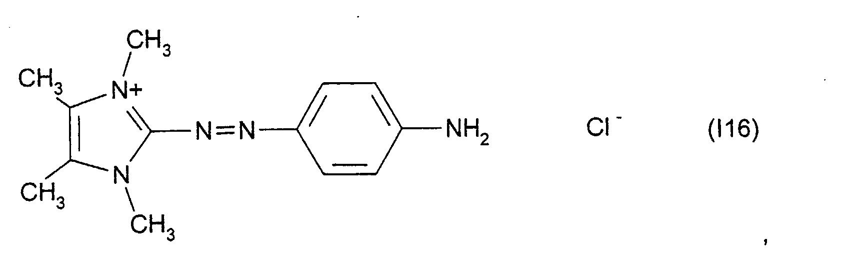 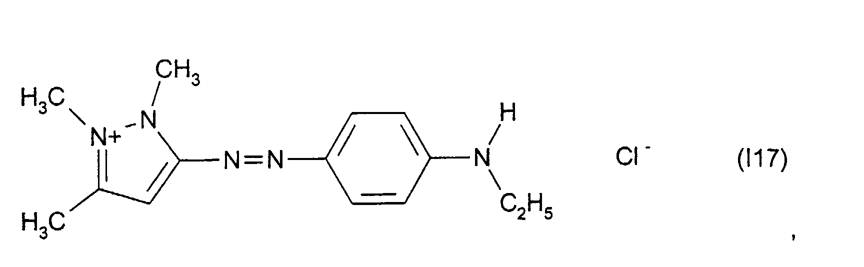 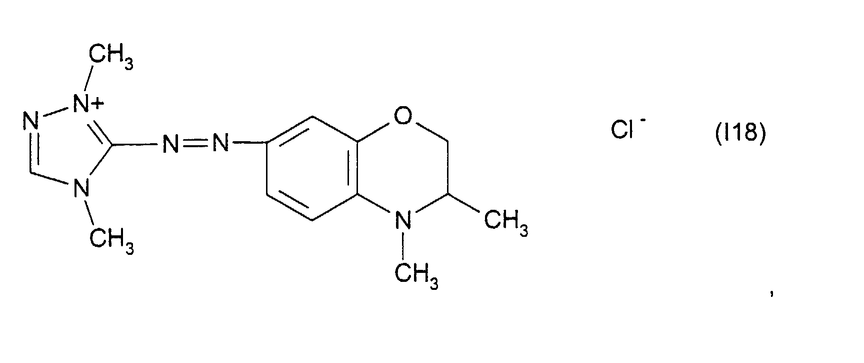 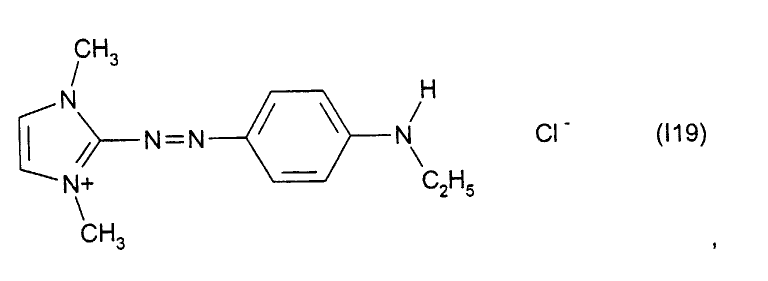 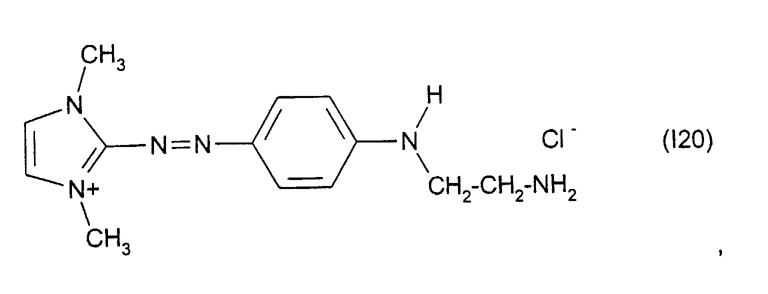 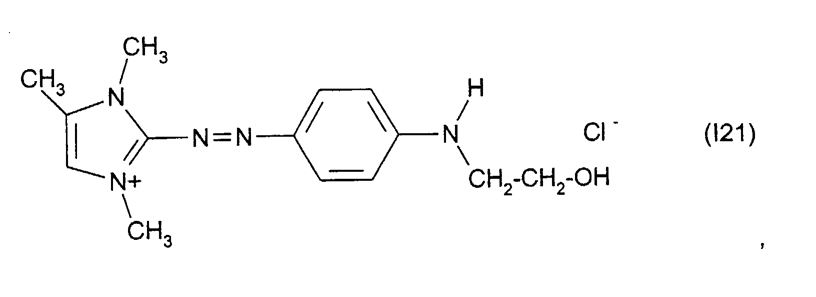 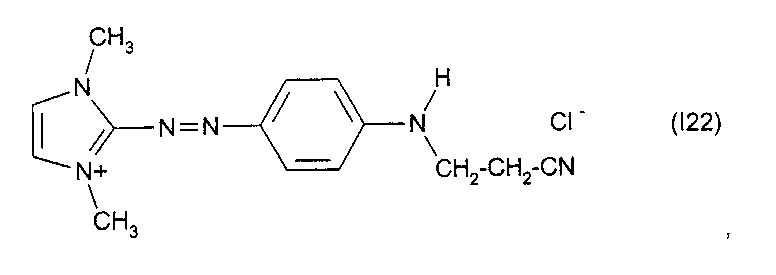 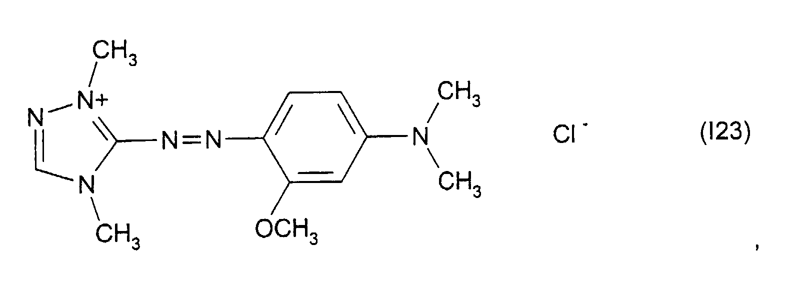 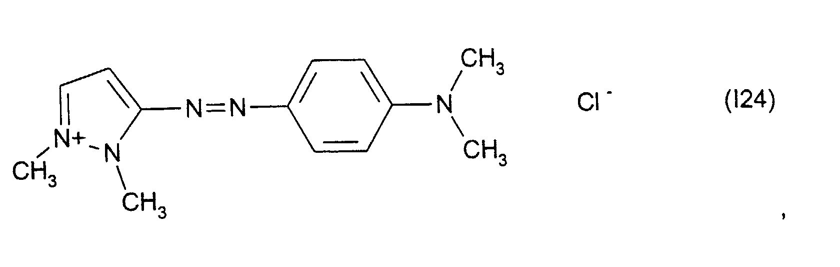 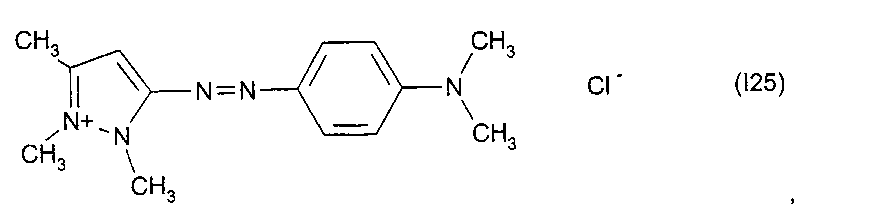 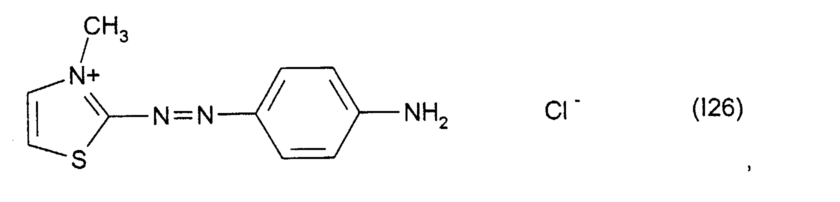 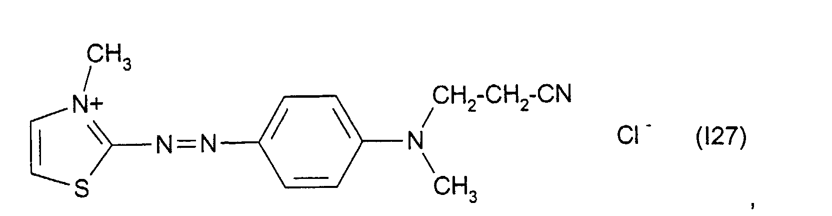 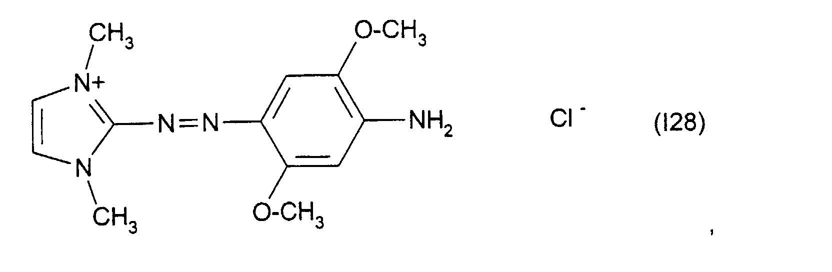 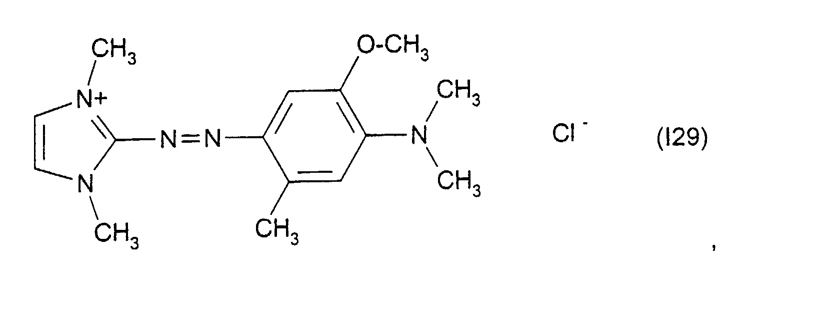 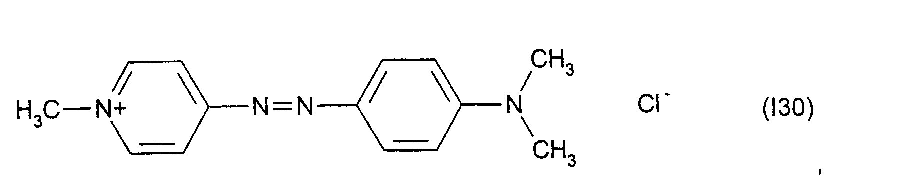 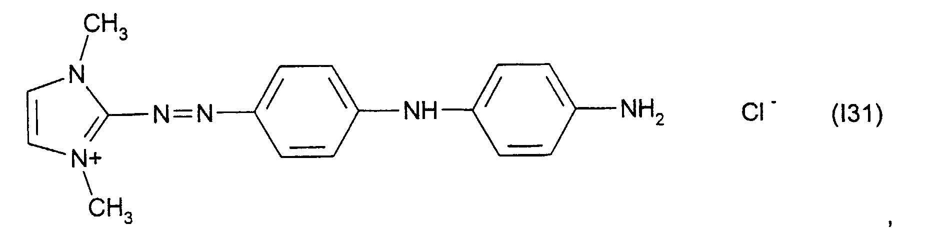 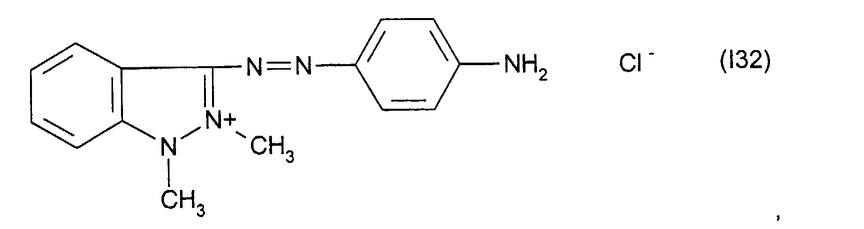 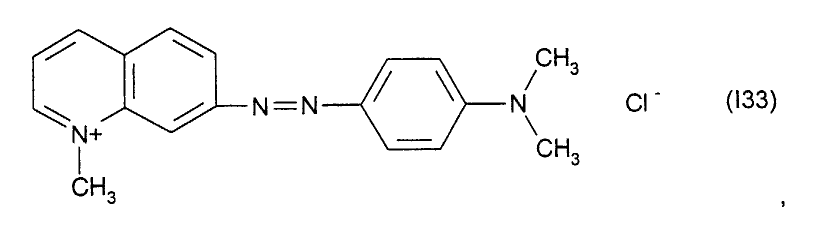 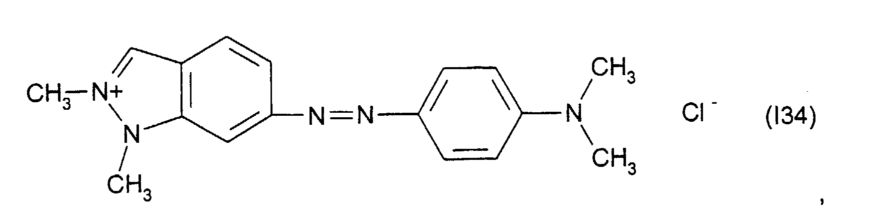 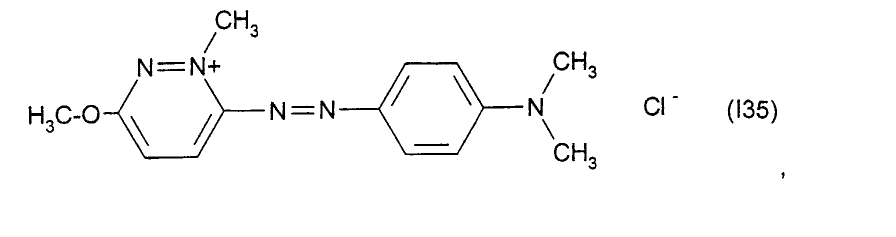 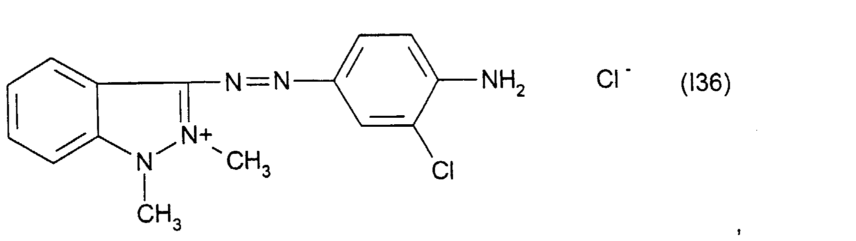 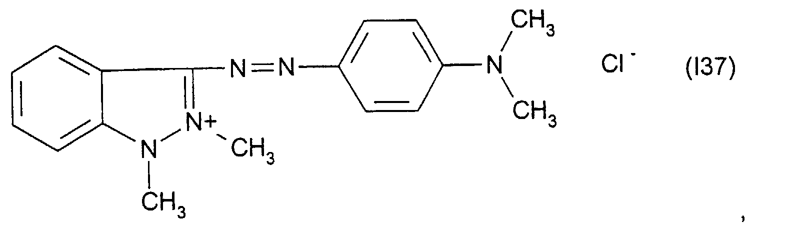 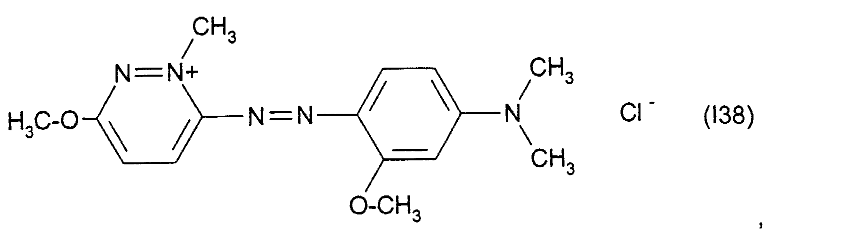 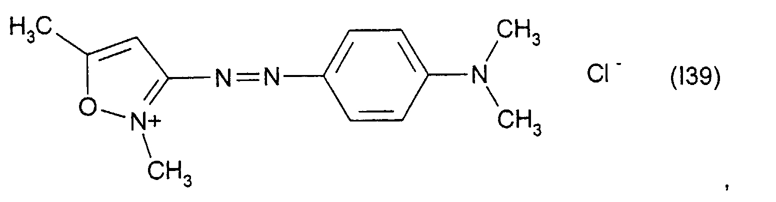 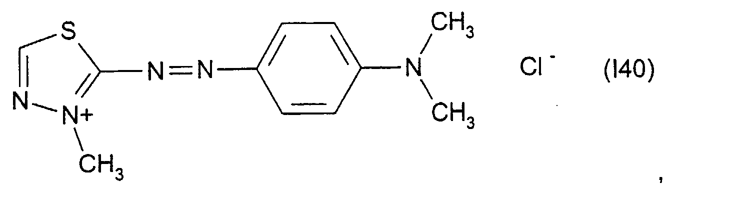 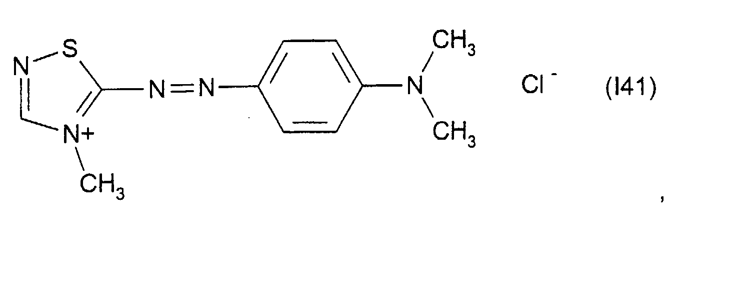 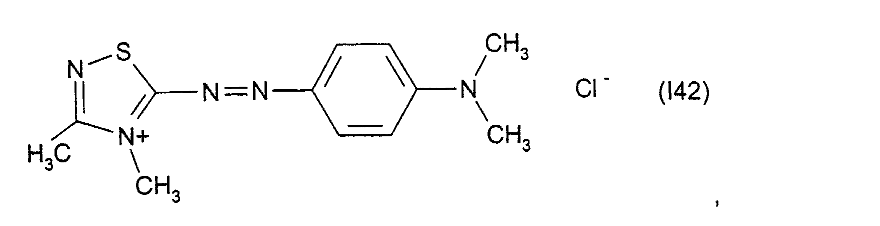 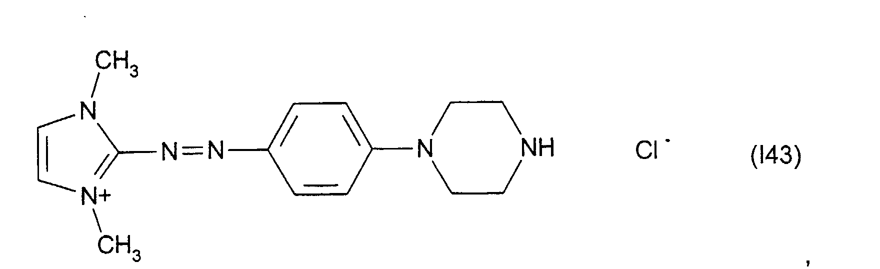 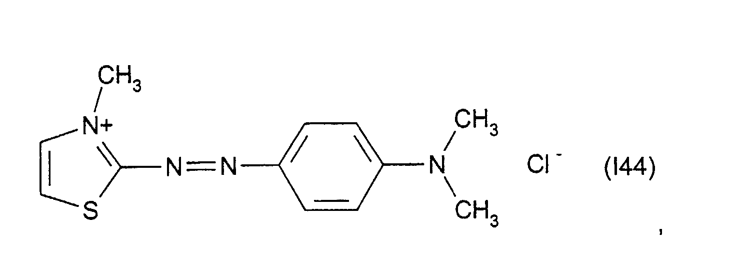 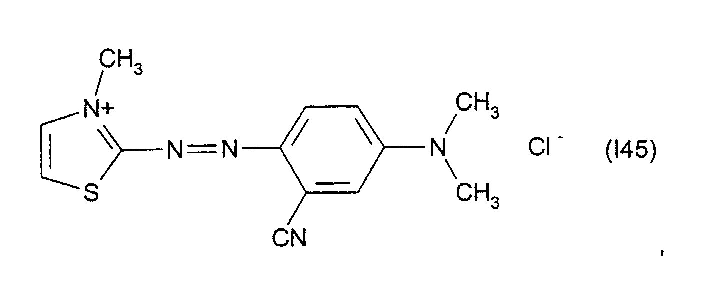 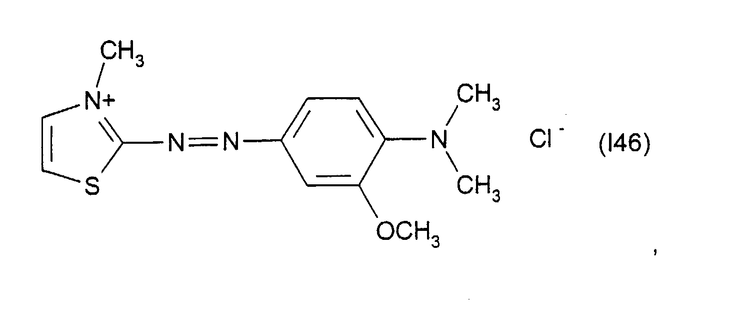 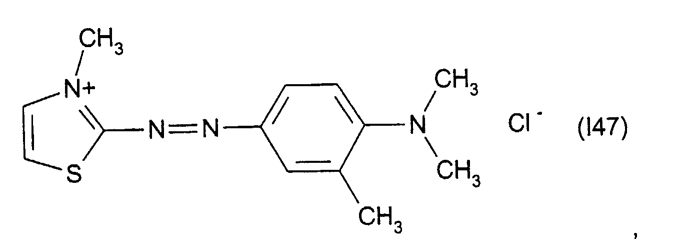 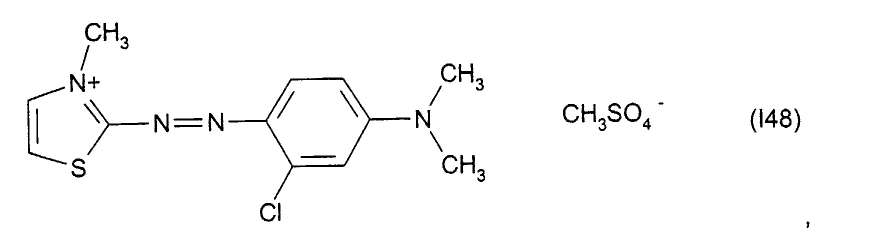 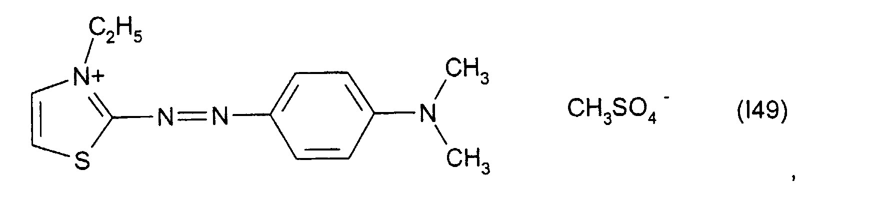 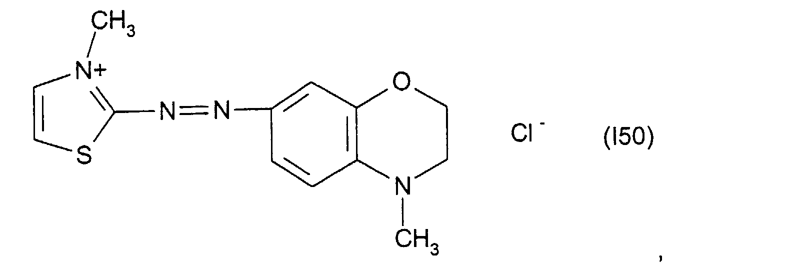 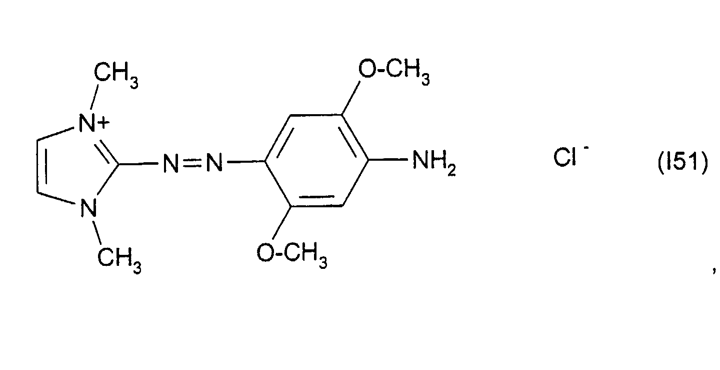 et

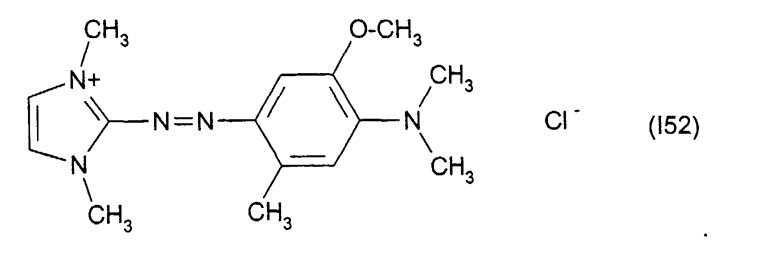

Parmi les composés de structures (I1) à (I52) décrits ci-dessus, on préfère tout particulièrement les composés répondant aux structures (I1), (I2), (I14) et (I31).

Parmi les colorants directs cationiques de formule (II) utilisables dans les compositions tinctoriales conformes à l'invention, on peut plus particulièrement citer les composés répondant aux structures (II1) à (II12) suivantes : 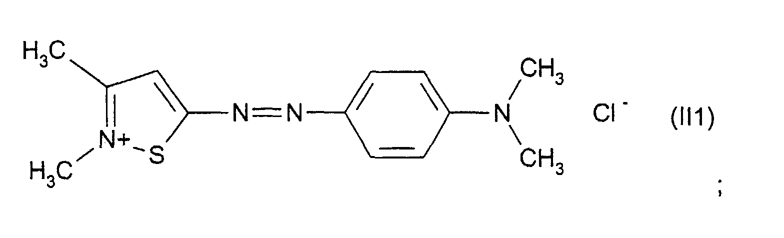 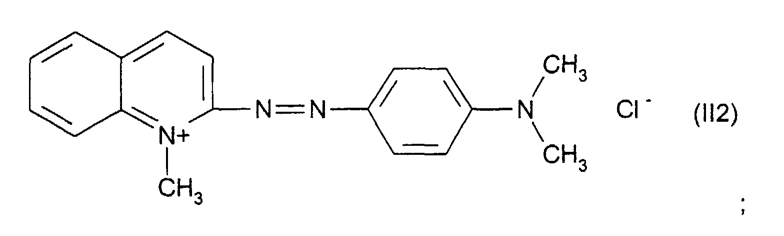 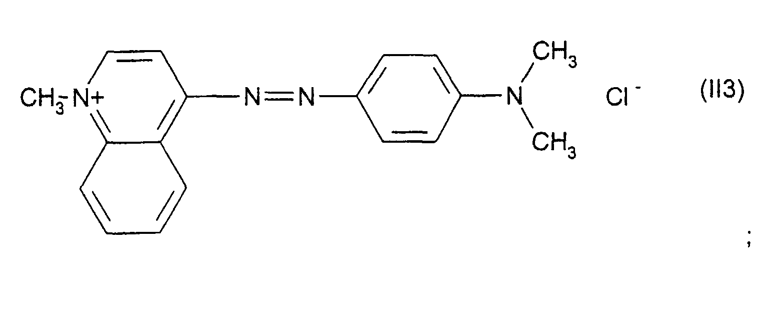 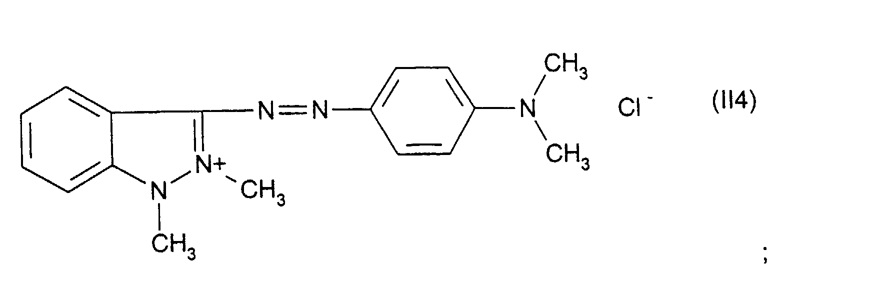 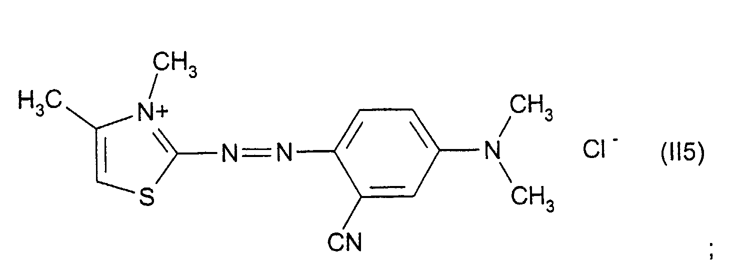 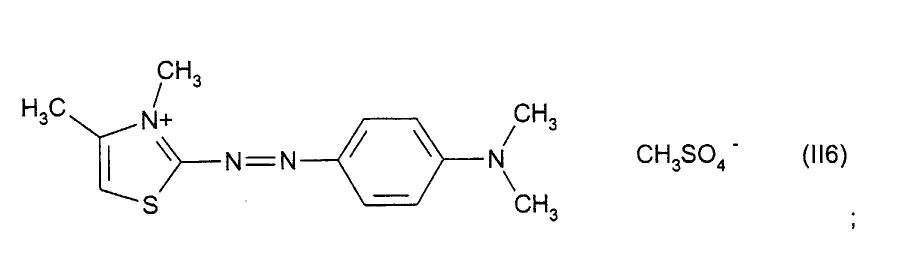 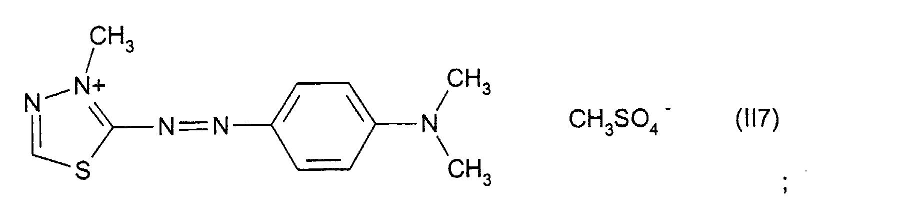 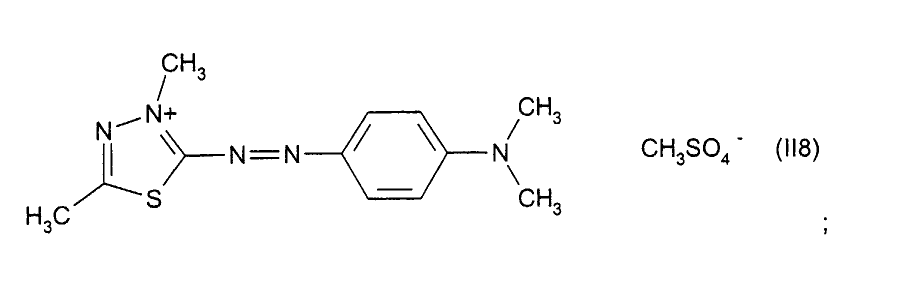 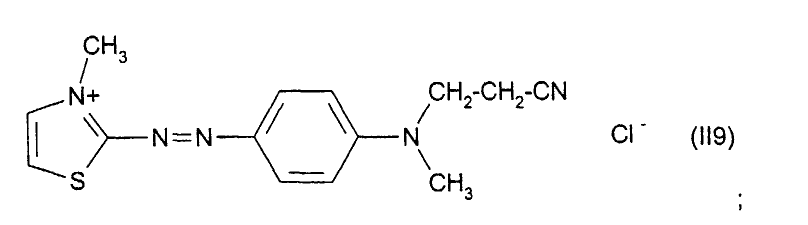 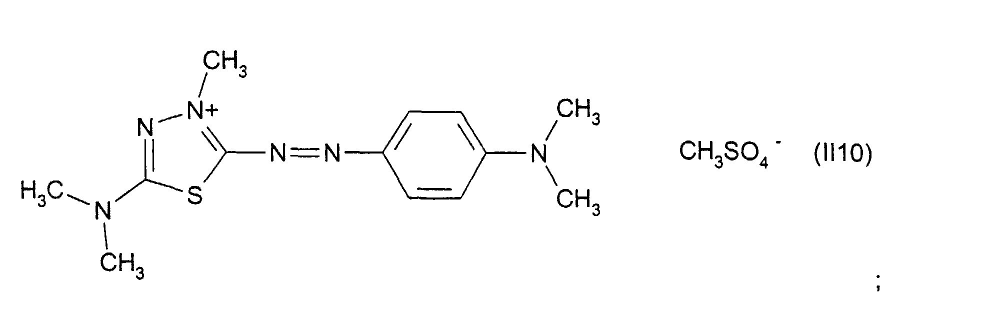 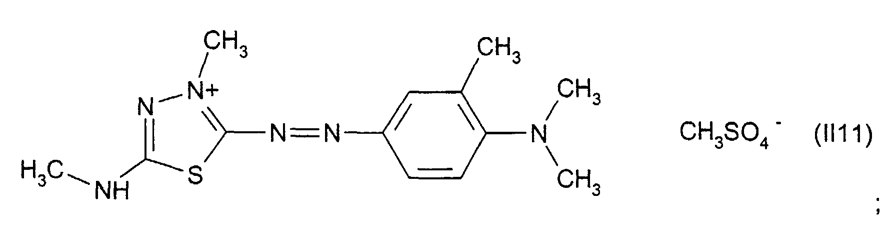 et

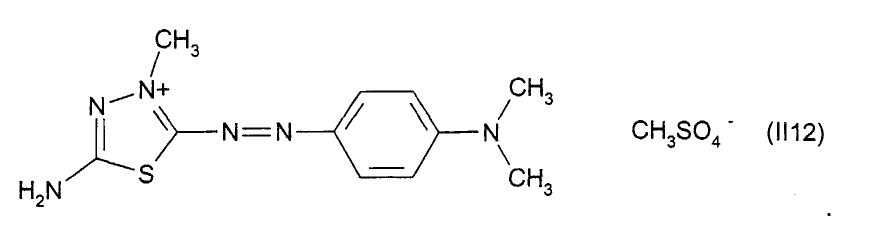

Parmi les colorants directs cationiques de formule (III), utilisables dans les compositions tinctoriales conformes à l'invention, on peut plus particulièrement citer les composés répondant aux structures (III1) à (III18) suivantes : 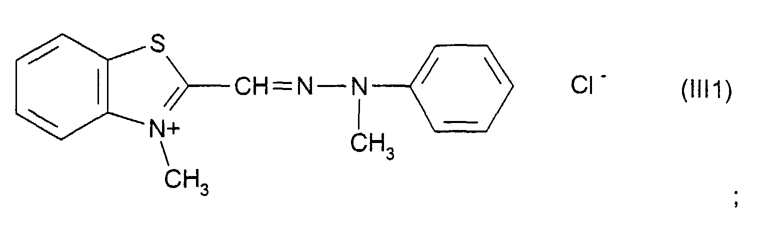 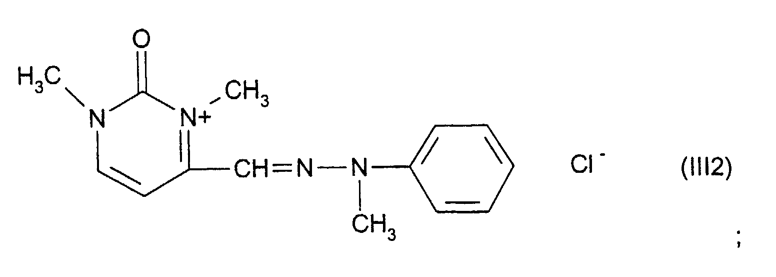 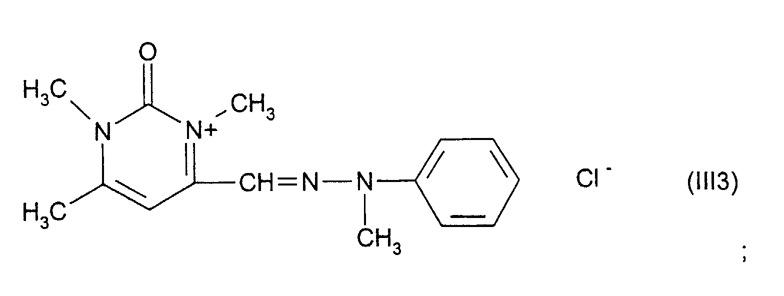 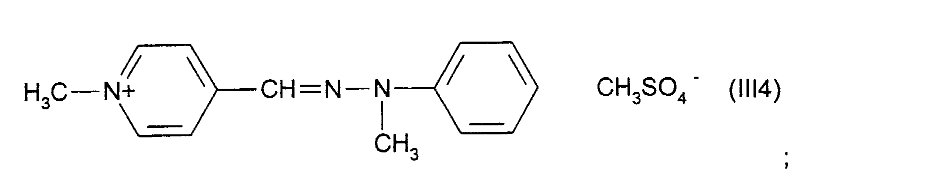 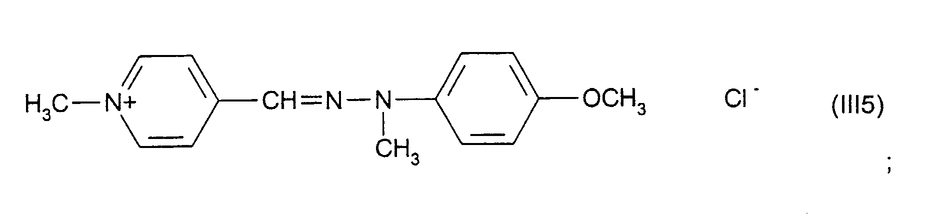 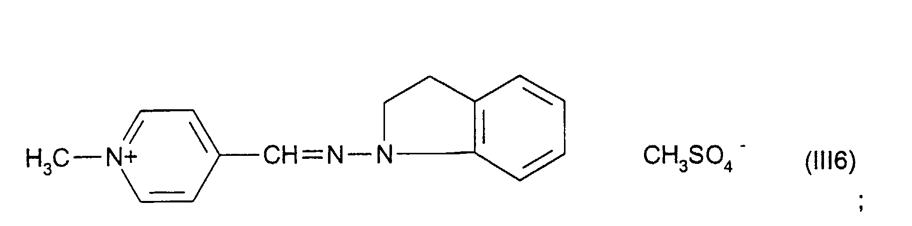 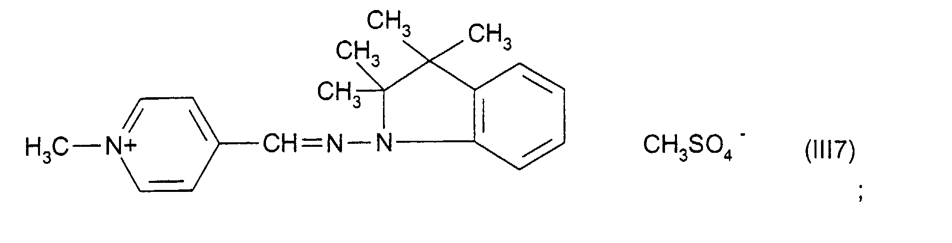 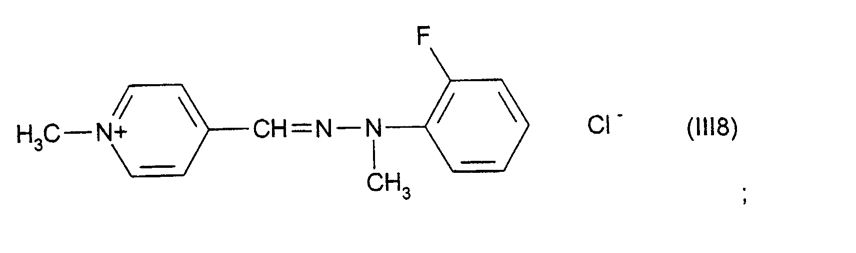 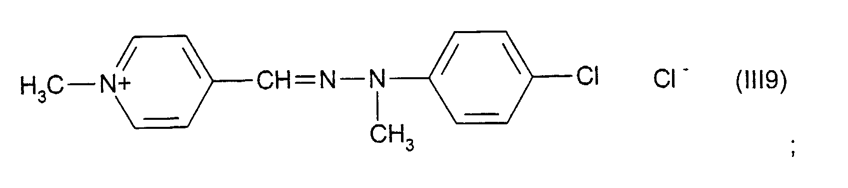 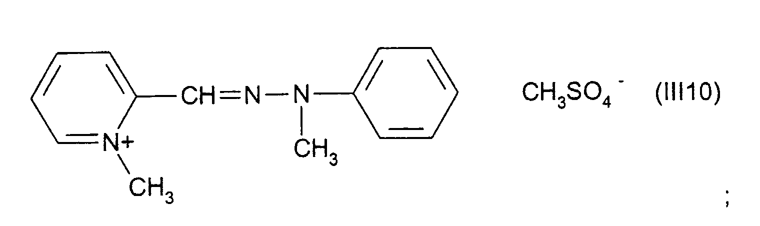 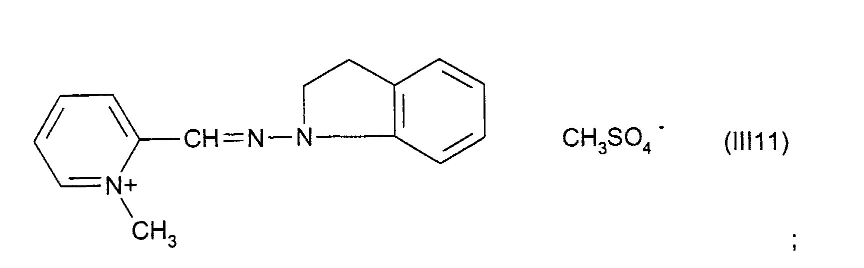 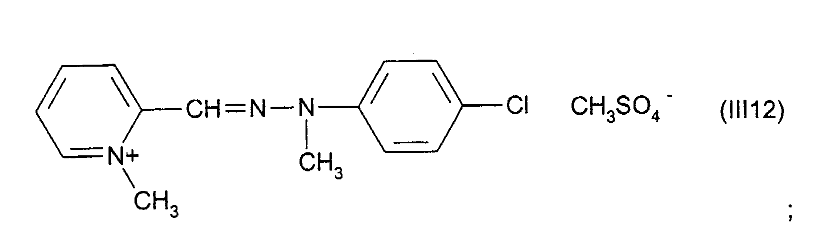 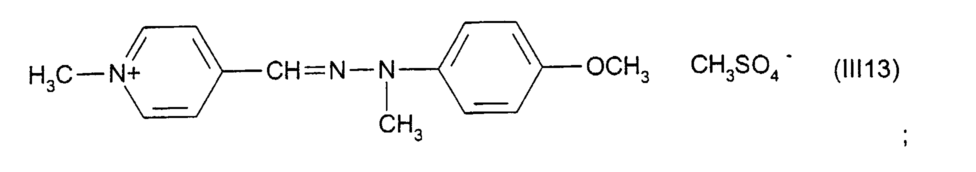 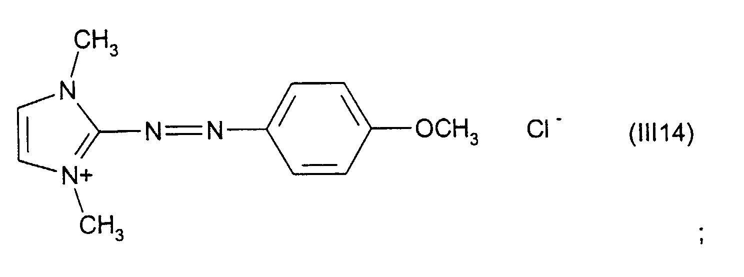 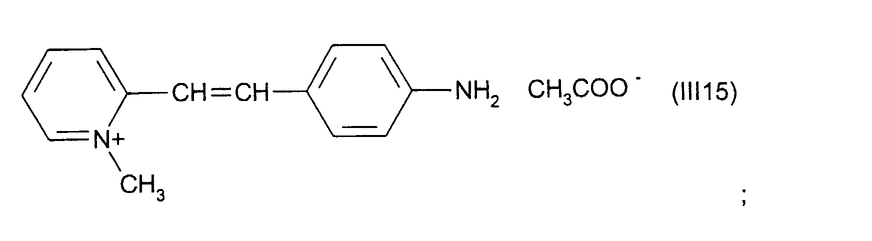 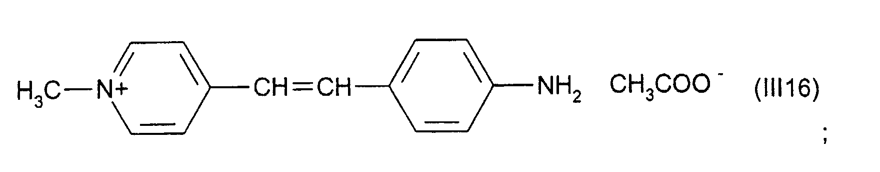 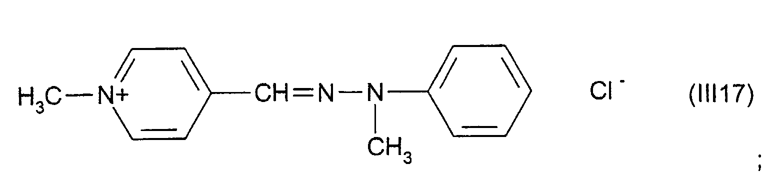 et

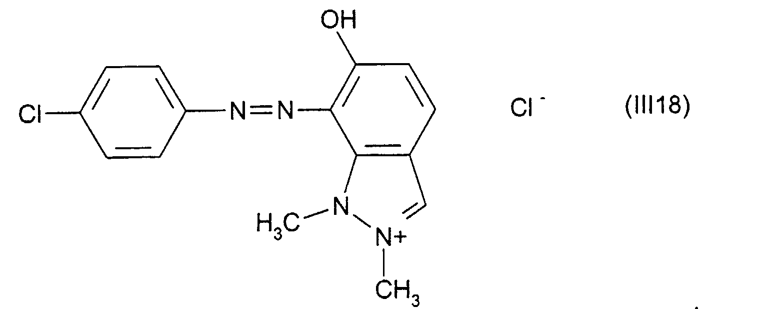

Parmi les composés de structures (III1) à (III18) décrits ci-dessus, on préfère tout particulièrement les composés répondant aux structures (III4), (III5) et (III13). Parmi les colorants directs cationiques de formule (III'), utilisables dans les compositions tinctoriales conformes à l'invention, on peut plus particulièrement citer les composés répondant aux structures (III'1) à (III'3) suivantes : 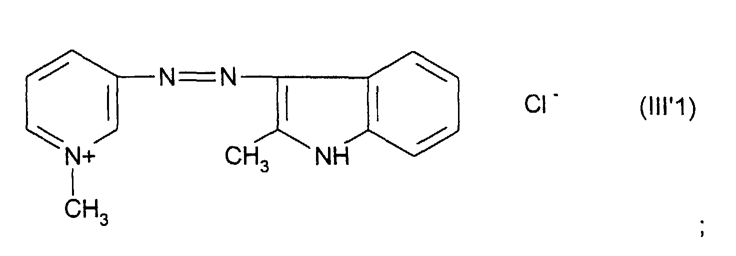 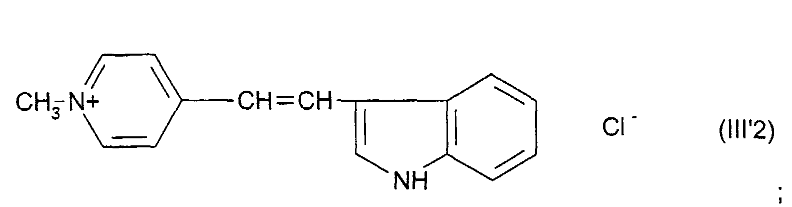 et

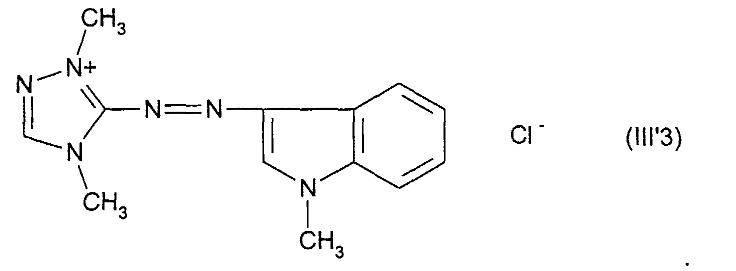

Parmi les colorants directs cationiques de formule (IV) utilisables dans les compositions tinctoriales conformes à l'invention, on peut citer plus particulièrement les composés répondant aux structures (IV)₁ à (IV)₇₇ suivantes : 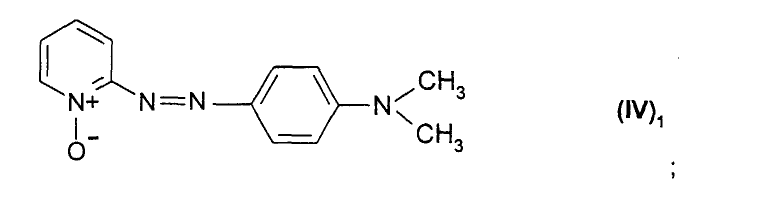 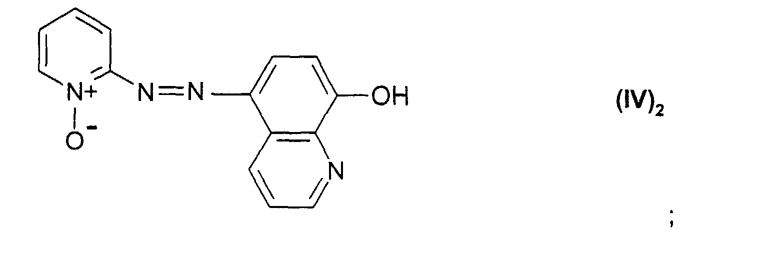 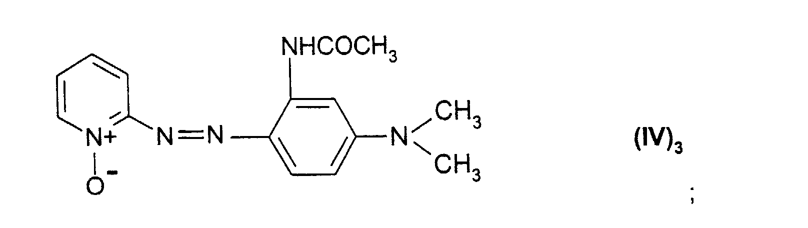 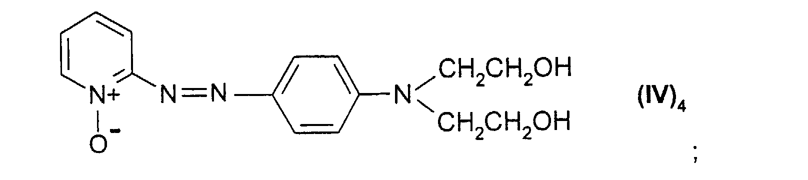 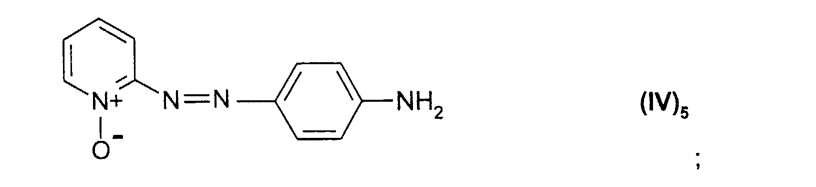 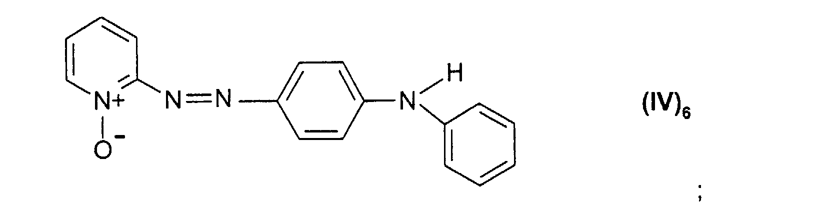 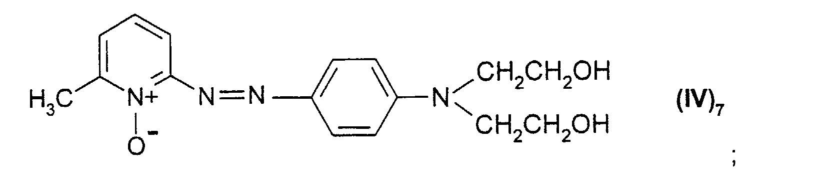 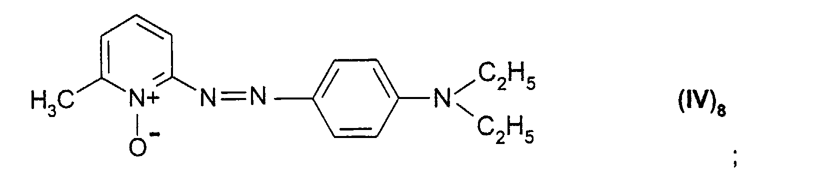 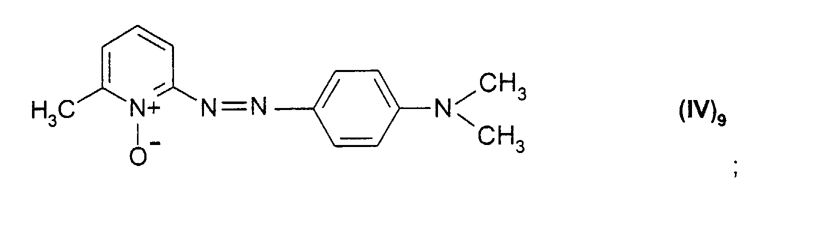 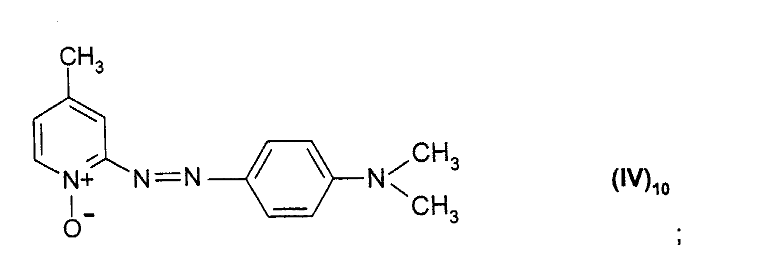 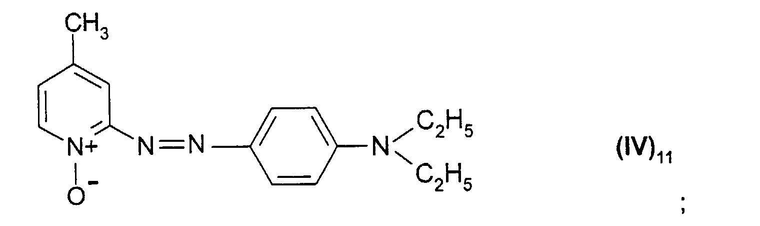 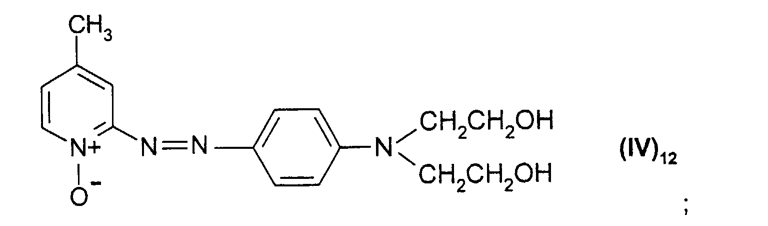 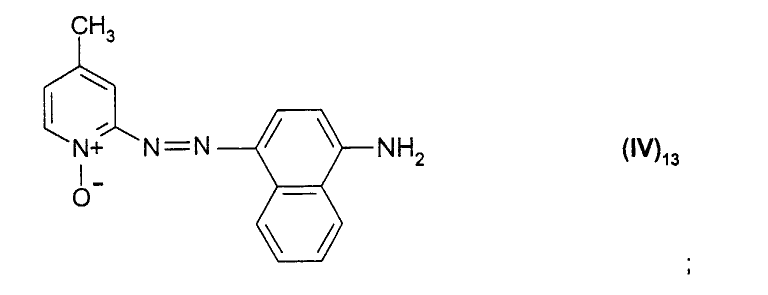 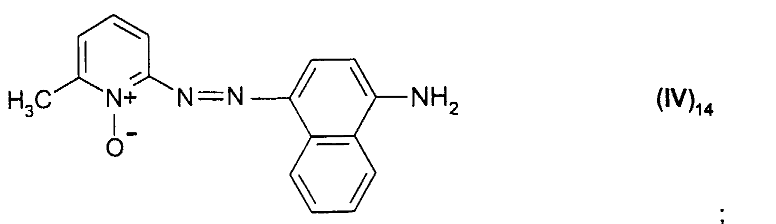 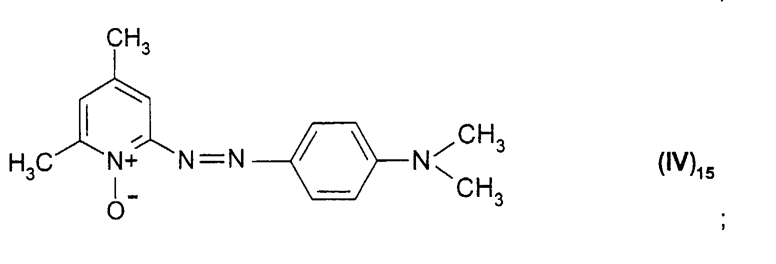 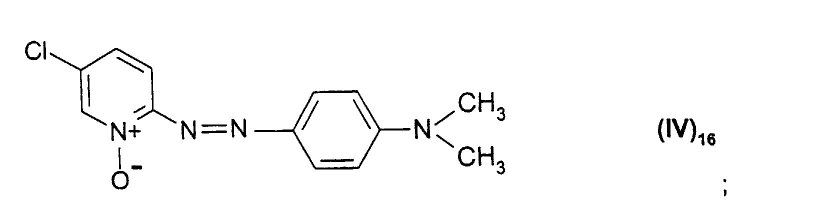 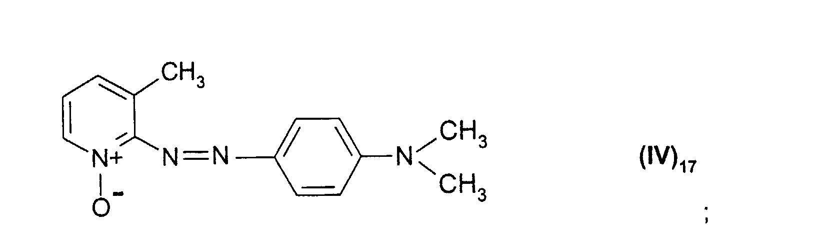 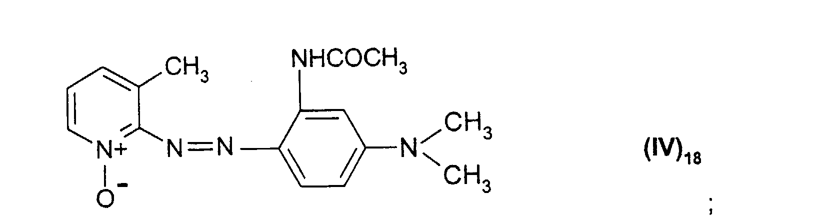 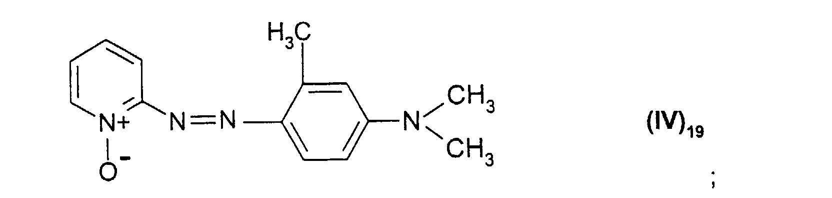 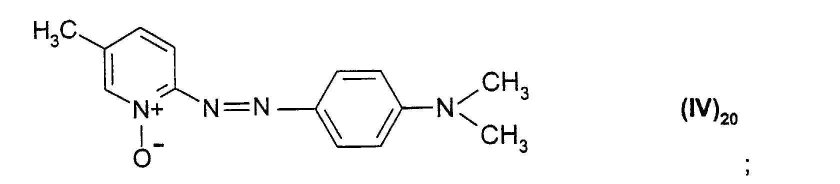 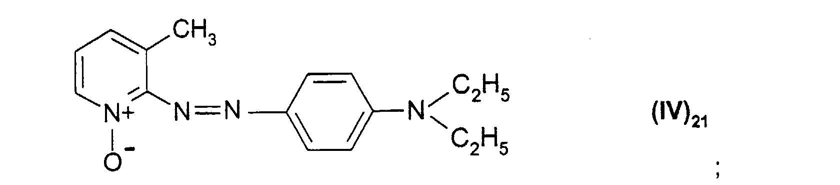 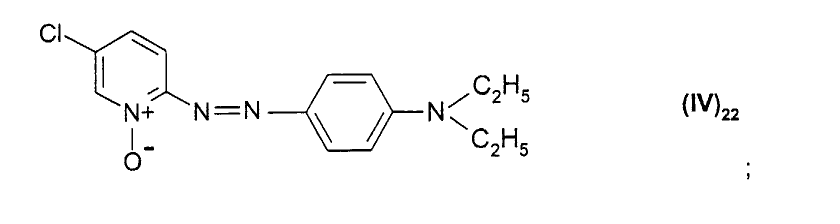 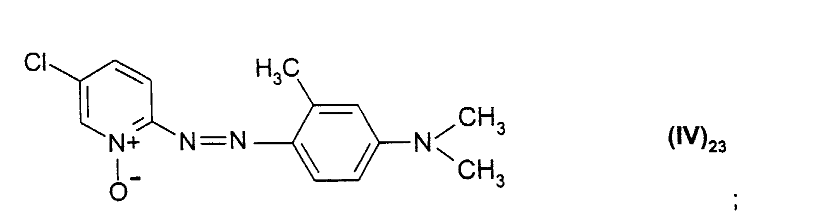 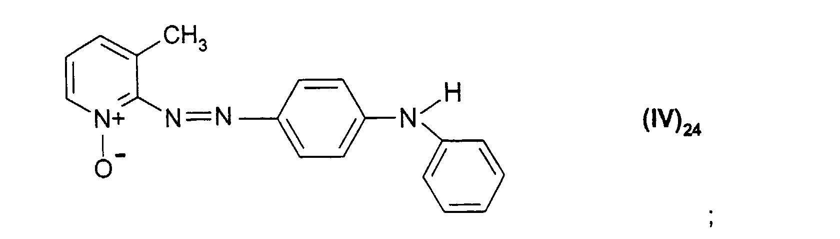 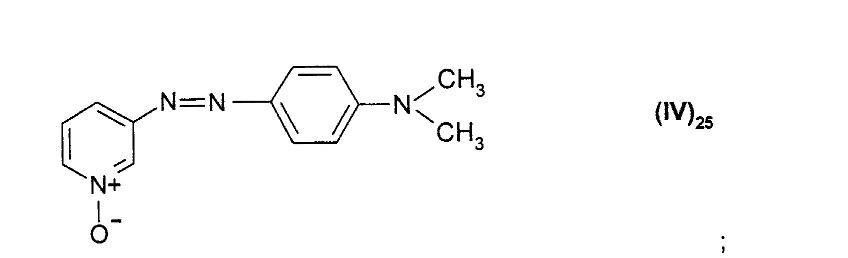 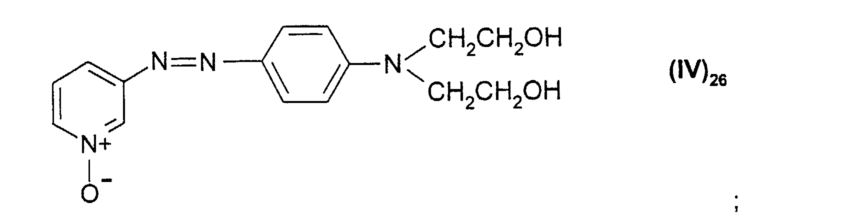 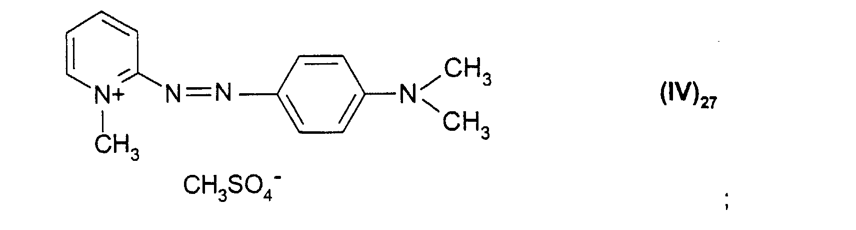 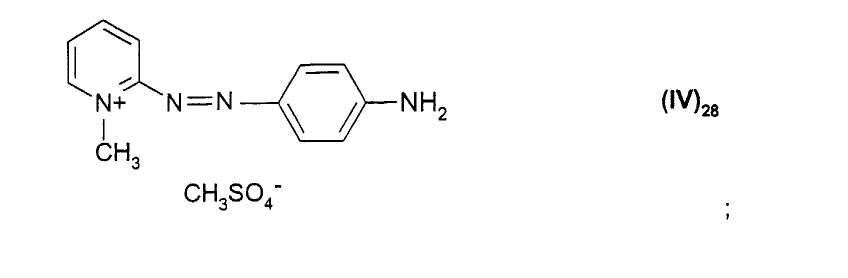 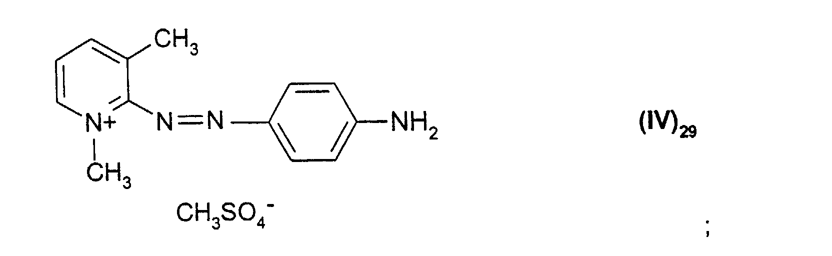 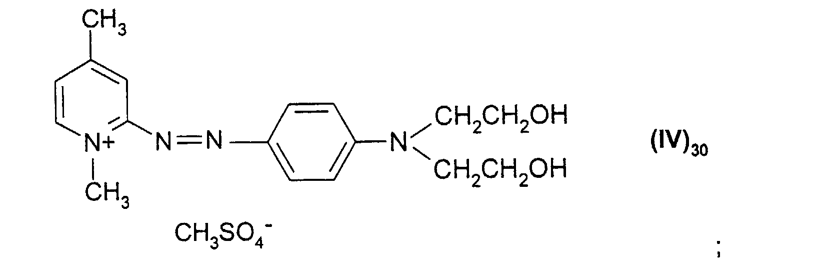 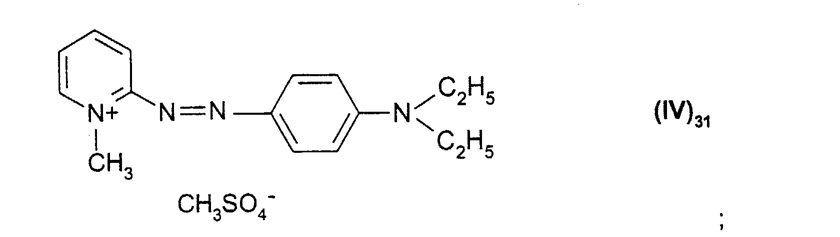 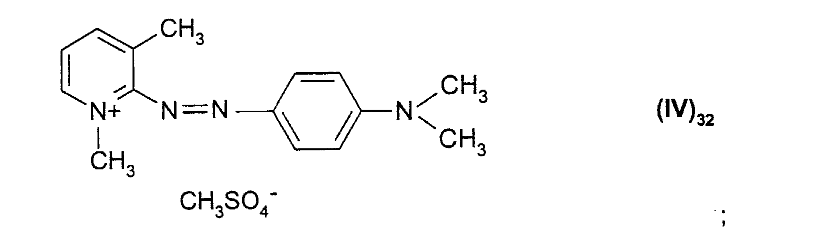 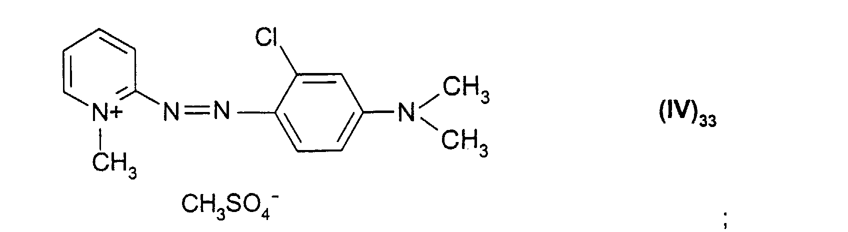 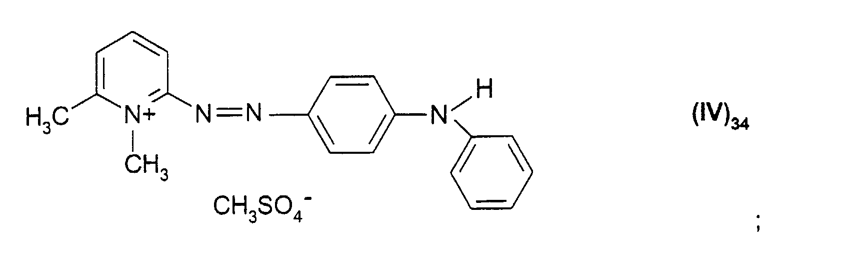 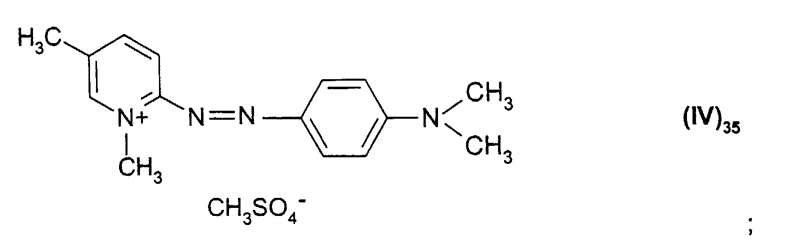 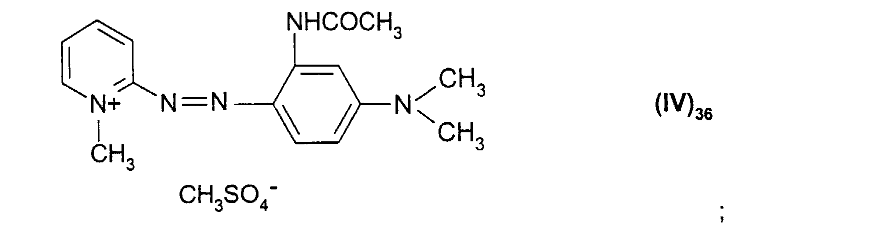 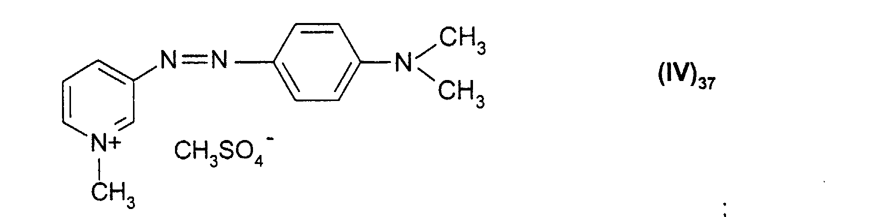 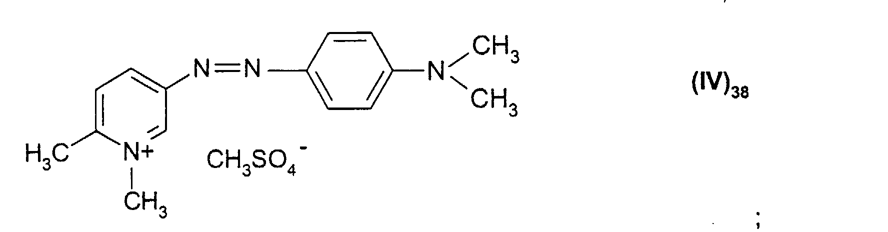 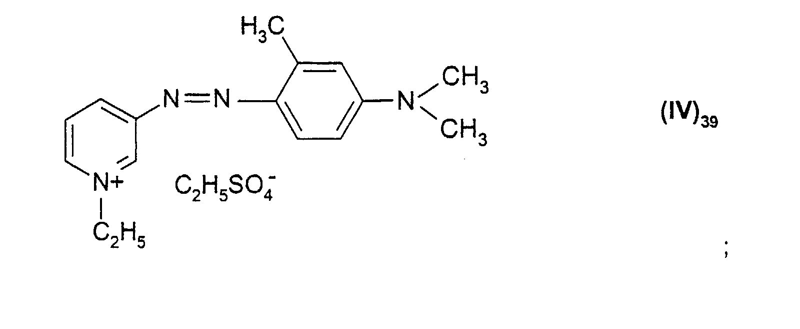 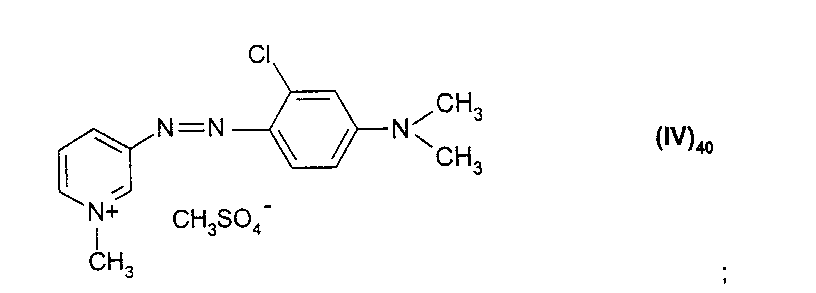 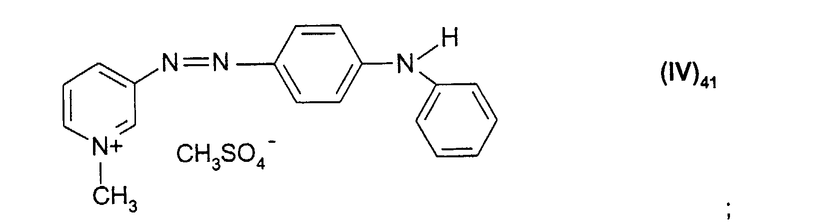 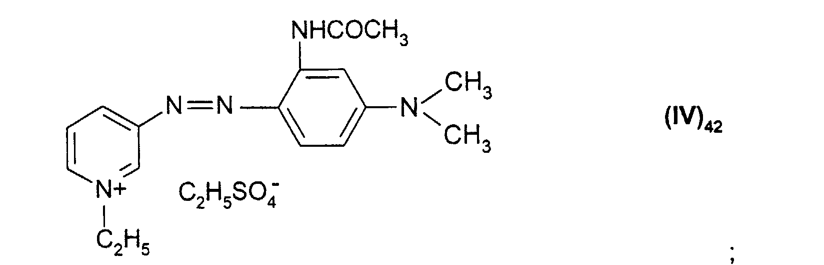 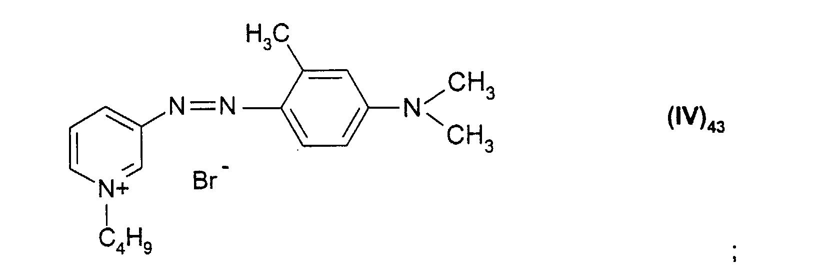 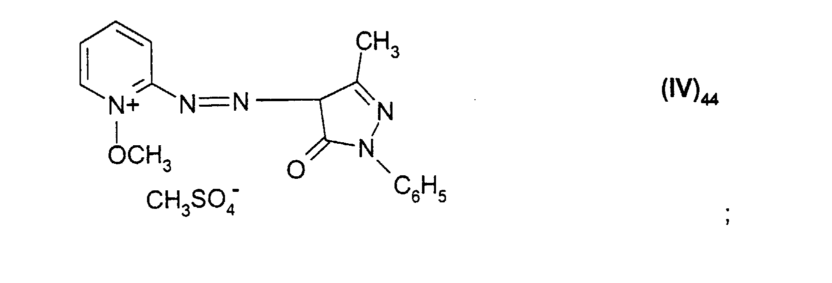 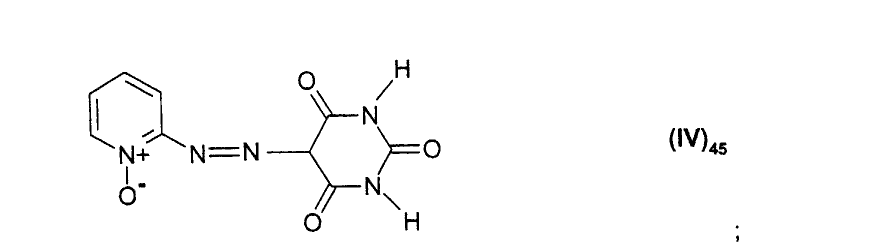 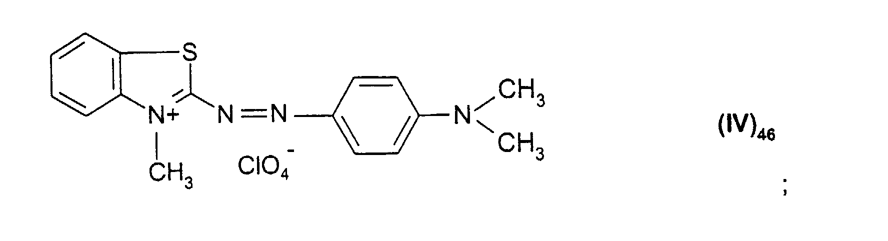 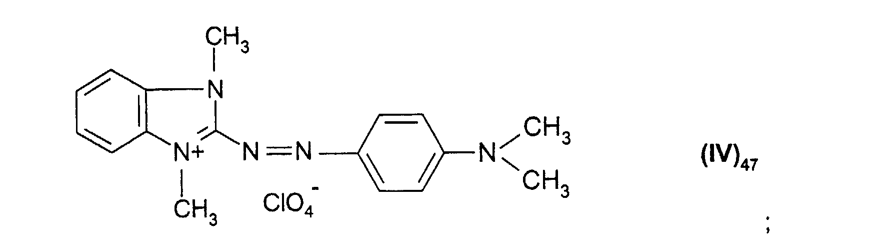 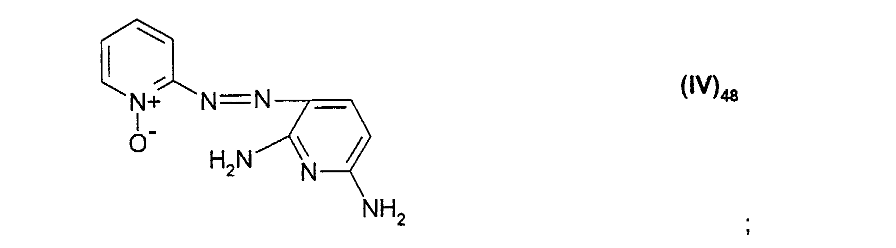 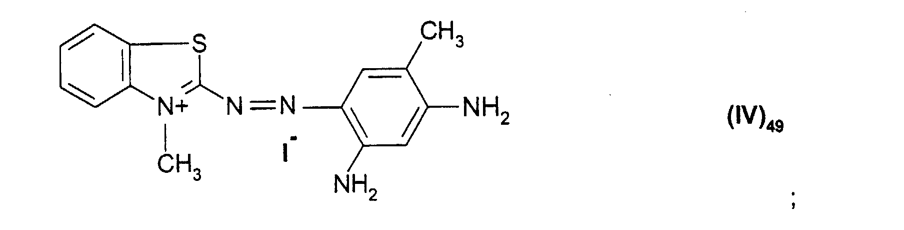 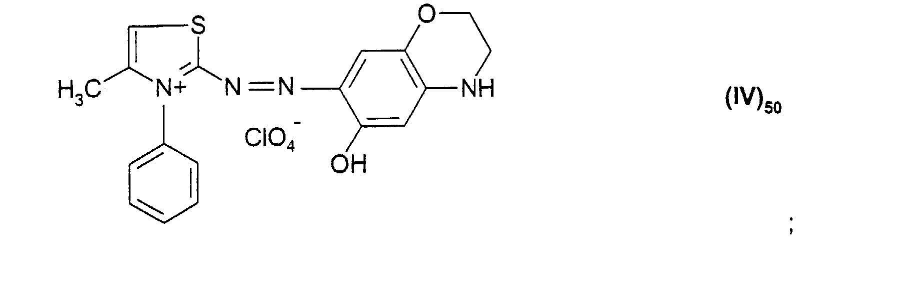 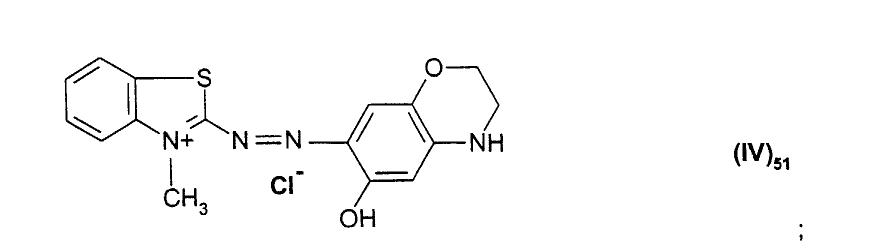 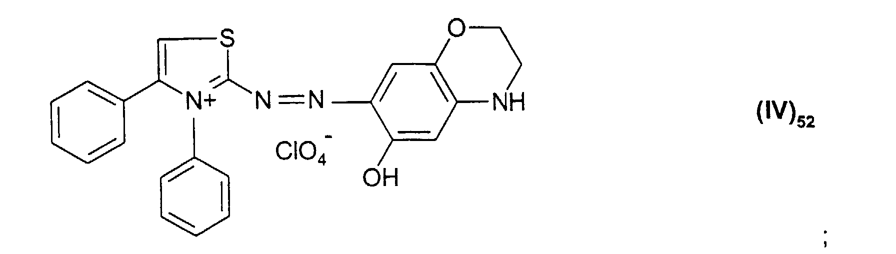 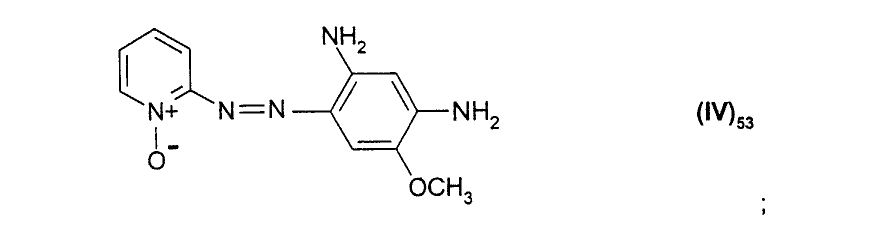 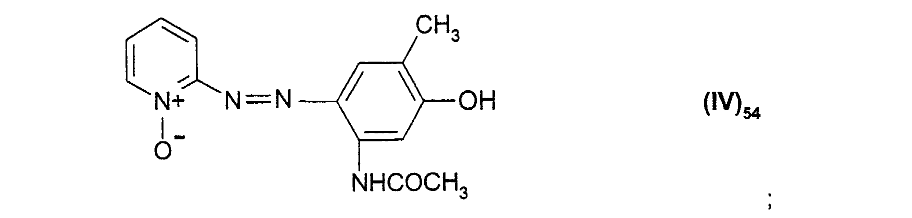 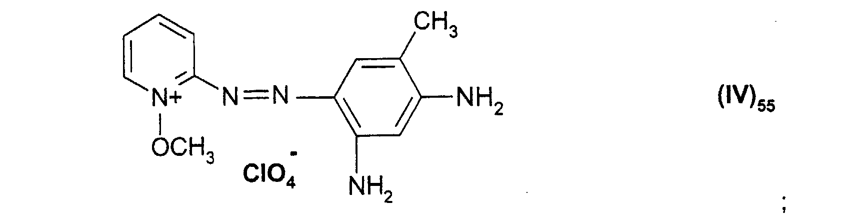 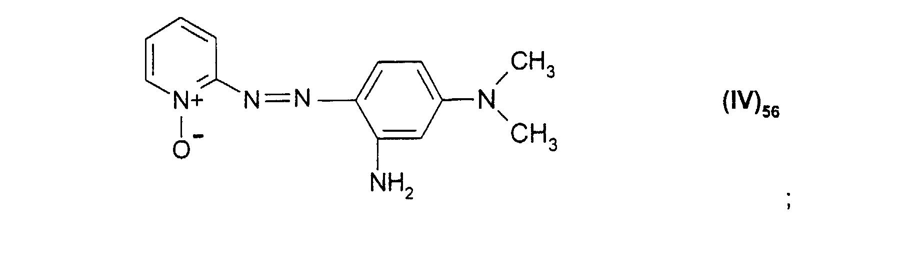 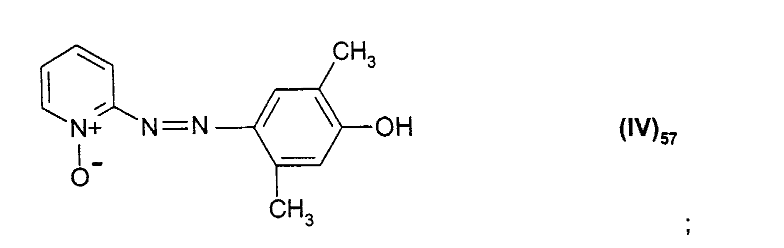 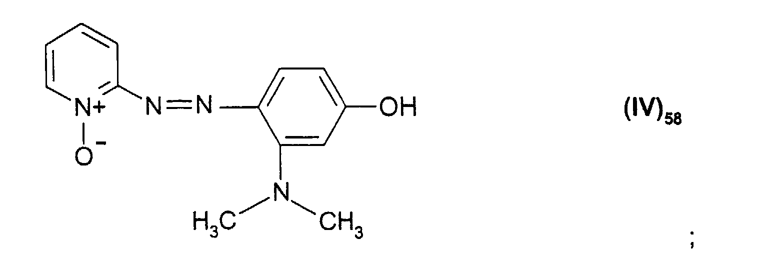 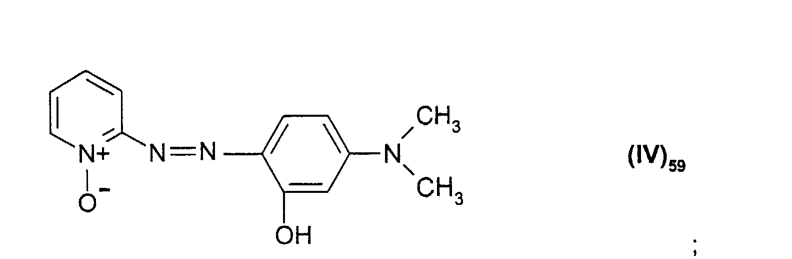 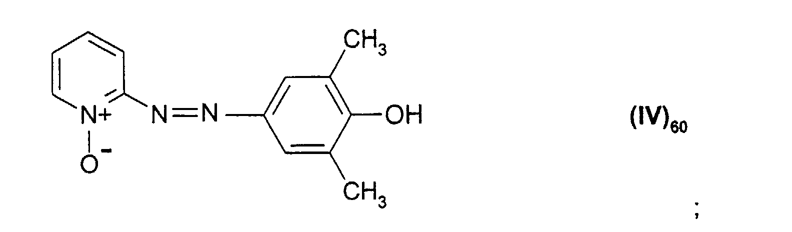 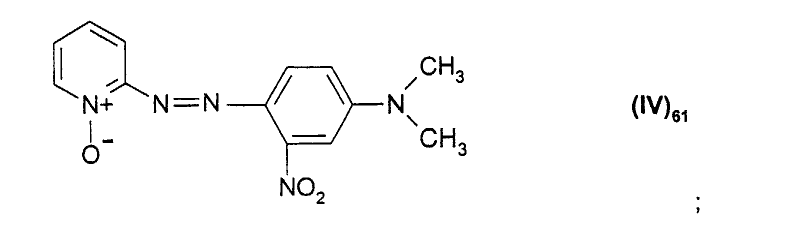 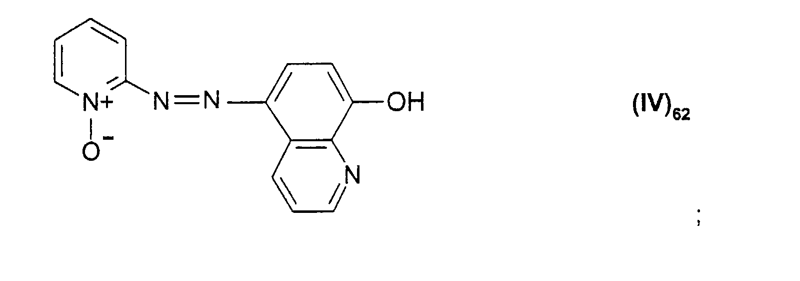 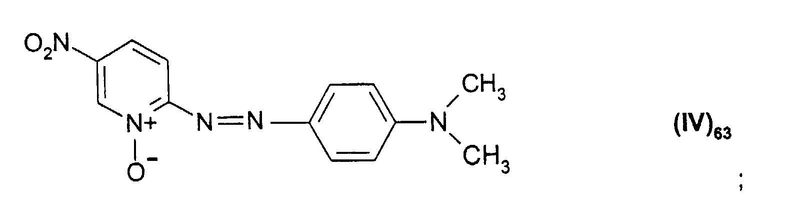 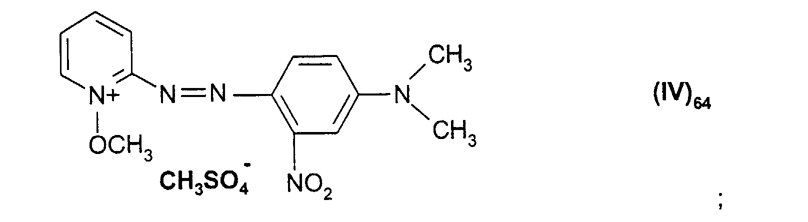 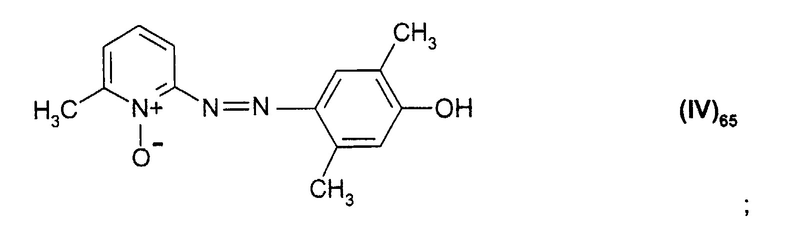 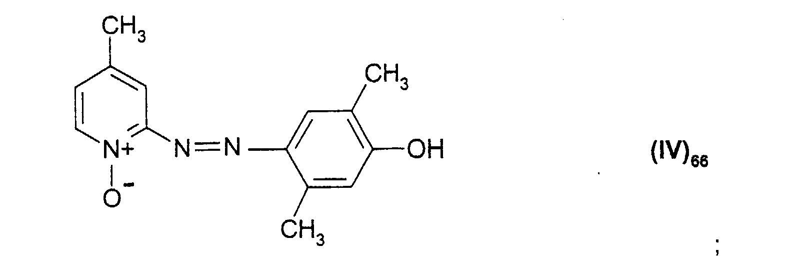 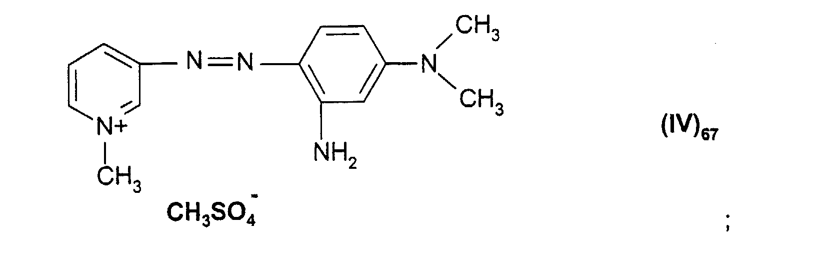 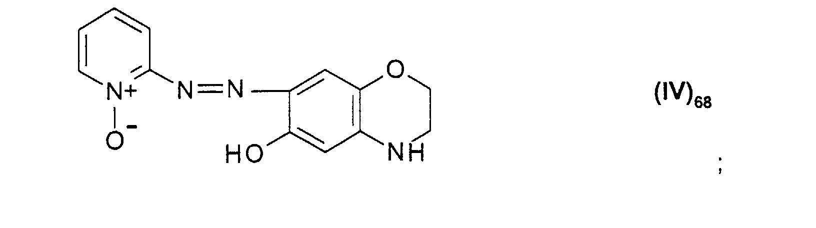 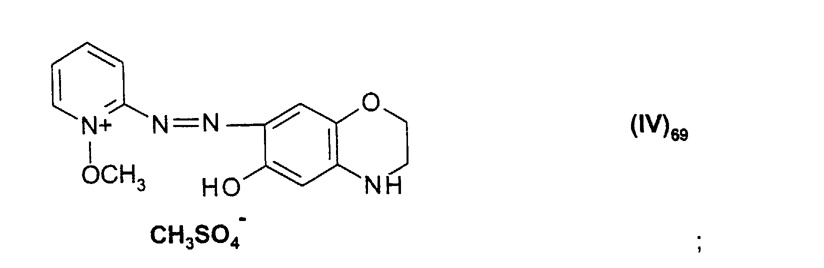 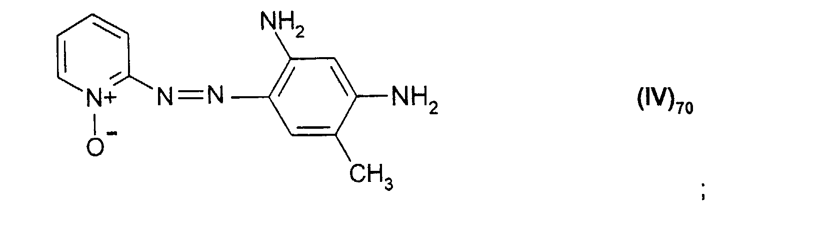 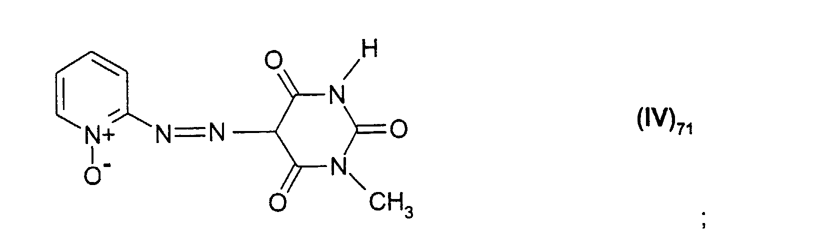 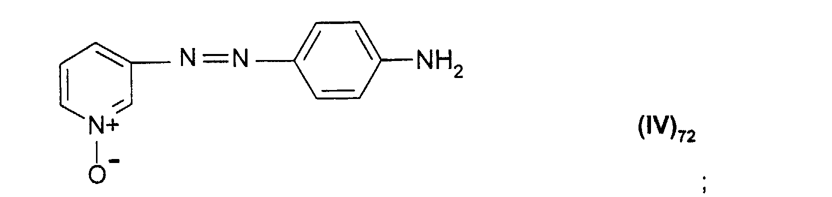 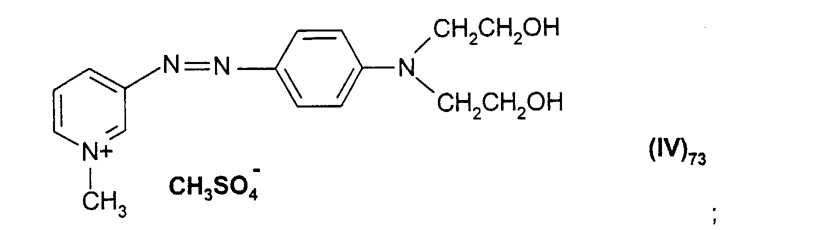 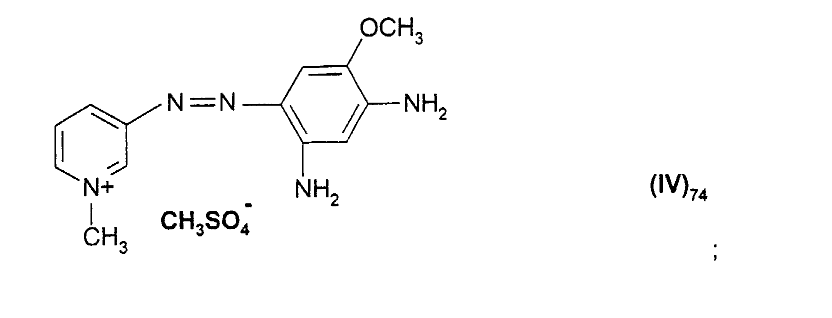 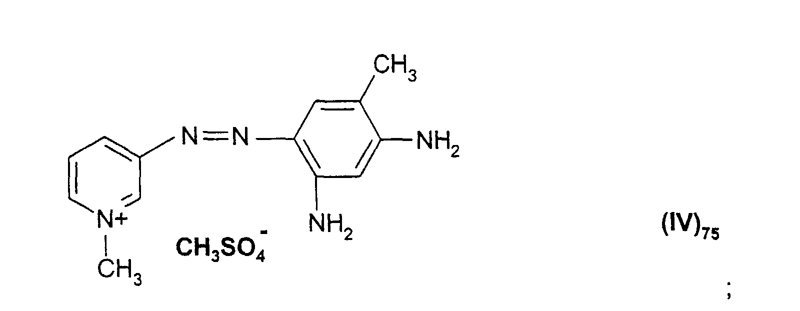 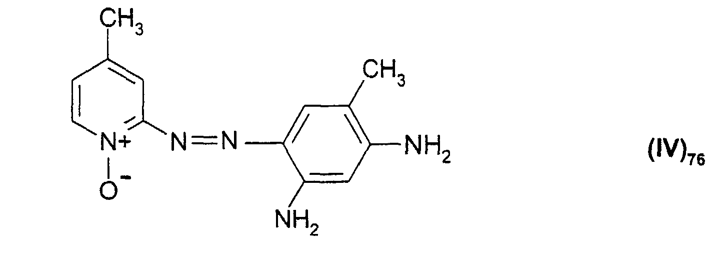 et

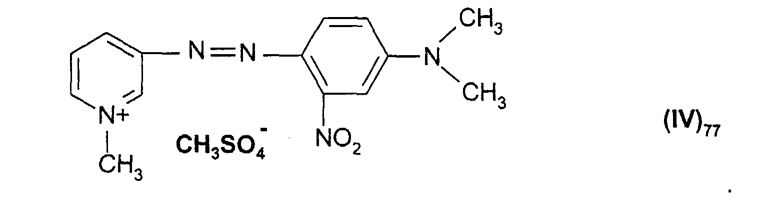

La ou les pyrazolo-[1,5-a]-pyrimidines de formule (V) conformes à l'invention et/ou le ou leurs sels d'addition avec un acide ou avec une base représentent de préférence de 0,0005 à 12 % en poids environ du poids total de la composition tinctoriale, et encore plus préférentiellement de 0,005 à 6 % en poids environ de ce poids.

Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.
Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (Vi) suivante : 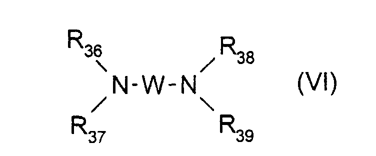 dans laquelle W est un reste propylène éventuellement substitué par un groupement hydroxyle ou un radical alkyle en C₁-C₆ ; R₃₆, R₃₇, R₃₈ et R₃₉, identiques ou différents, représentent un atome d'hydrogène, un radical alkyle en C₁-C₆ ou hydroxyalkyle en C₁-C₆.

La composition tinctoriale conforme à l'invention peut également renfermer un ou plusieurs coupleurs pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer une ou plusieurs bases d'oxydation additionnelles différentes d'une pyrazolo-[1,5-a]-pyrimidine et/ou un ou plusieurs colorants directs non cationiques, notamment pour modifier les nuances ou les enrichir en reflets.

Parmi les bases d'oxydation additionnelles pouvant être utilisées dans les compositions tinctoriales conformes à l'invention, on peut citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques différents des composés de formule (V) conformes à l'invention, et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, et la 2,5,6-triaminopyrimidine.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

D'une manière générale, les sels d'addition avec un acide utilisables dans le cadre de l'invention (composés de formule (V), bases d'oxydation additionnelles et coupleurs) sont notamment choisis parmi les chlorhydrates, les bromhydrates, les sulfates, les citrates, les succinates, les tartrates, les lactates et les acétates. Les sels d'addition avec une base utilisables dans le cadre des compositions tinctoriales de l'invention, (composés de formule (V)), sont notamment ceux obtenus avec la soude, la potasse, l'ammoniaque ou les amines.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques tels que par exemple des gommes de guar non-ioniques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de poudres, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant non enzymatique qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant non enzymatique présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les peracides et les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

L'exemple suivant est destiné à illustrer l'invention sans pour autant présenter un caractère limitatif.

### EXEMPLE DE TEINTURE EN MILIEU ALCALIN

On a préparé la composition tinctoriale conforme à l'invention suivante :
- Pyrazolo-[1,5-a]-pyrimidine-3,7-diamine, 2HCl (base d'oxydation) 0,333 g
- Colorant direct cationique de formule (12) 1 g
- Alcool éthylique à 96° 18 g
- Sel pentasodique de l'acide diéthylènetriaminopentacétique 1,1 g
- Ammoniaque à 20% de NH₃ 10,0 g
- Eau déminéralisée qsp 100 g

Au moment de l'emploi, on a mélangé poids pour poids la composition tinctoriale ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les cheveux ont été teints dans une nuance fuchsia puissant.

## Revendications

1. Composition pour la teinture d'oxydation des fibres kératiniques, et en particulier des fibres kératiniques humaines telles que les cheveux, caractérisée par le fait qu'elle contient, dans un milieu approprié pour la teinture :
- au moins une pyrazolo-[1,5-a]-pyrimidine à titre de base d'oxydation ;
- et au moins un colorant direct cationique.
ladite composition étant exempte de tout système enzymatique susceptible de provoquer l'oxydation de la ou des pyrazolo-[1,5-a]-pyrimidines.

2. Composition selon la revendication 1, caractérisée par le fait que la ou les pyrazolo-[1,5-a]-pyrimidines sont choisies parmi les composés de formule (V) suivante et leurs sels d'addition avec un acide ou avec une base : 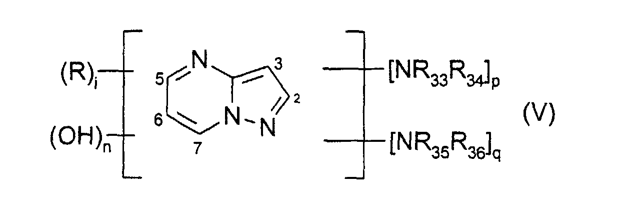 dans laquelle :
- R₃₃, R₃₄ R₃₅ et R₃₆ désignent, identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'aminé pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl-ou di-[hydroxy(C₁-C₄) alkyl]-amino alkyle en C₁-C₄ ;
- les radicaux R, identiques ou différents, désignent un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄(les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- (i) la somme p + q est différente de 0 ;
- (ii) lorsque p + q est égal à 2, alors n vaut 0 et les groupes NR₃₃R₃₄ et NR₃₅R₃₆ occupent les positions (2,3) ; (5,6) ; (6,7) ; (3,5) ou (3,7) ;
- (iii) lorsque p + q est égal à 1 alors n vaut 1 et le groupe NR₃₃R₃₄ (ou NR₃₅R₃₆) et le groupe OH occupent les positions (2,3) ; (5,6) ; (6,7) ; (3,5) ou (3,7).

3. Composition selon la revendication 2, caractérisée par le fait que les pyrazolo-[1,5-a]-pyrimidines de formule (V) sont choisies parmi :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine; - le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
- la 3-amino-5-méthyl-7-imidazolylpropylamino pyrazolo-[1,5-a]-pyrimidine ;
et leurs sels d'addition avec un acide ou avec une base.

4. Composition selon l'une quelconque des revendications précédentes, caractérisée par le fait que le ou les colorants directs cationiques sont choisis parmi les amino-anthraquinoniques cationiques, les mono- ou di-azoïques cationiques, et les naphtoquinones cationiques

5. Composition selon la revendication 4, caractérisée par le fait que les colorants directs cationiques sont choisis parmi le chlorure de [8-[(p-aminophényl)azol]-7-hydroxy-2-naphtyl]triméthylammonium, l'association du chlorure de 3-[(4-amino-6-bromo-5,8-dihydro-1-hydroxy-8-imino-5-oxo-2-naphtalényl)-amino]-N,N,N-triméthylbenzénaminium et du chlorure de 3-[(2,6-dibromo-5,8-dihydro-1-hydroxy-8-imino-5-oxo-3-naphtyl)-amino]-N,N,N-triméthyl-benzénaminium, le chlorure de 7-hydroxy-8-[(2-méthoxyphényl)azo]-N,N,N-triméthyl-2-naphtalènaminium, le chlorure de [8-[(4-amino-2-nitrophényl)azo]-7-hydroxy-2-naphtyl]triméthylammonium, l'association du chlorure de [8-[(4-amino-3-nitrophényl)azo]-7-hydroxy-2-naphtyl]triméthylammonium et du chlorure de [8-[(4-amino-2-nitrophényl)azo]-7-hydroxy-2-naphtyl]triméthylammonium, le chlorure de 3-[(4,5-dihydro-3-méthyl-5-oxo-1-phényl-1H-pyrazol-4-yl)azo]-N,N,N-triméthyl-benzènaminium, le chlorhydrate de 1-(γ-aminopropyl)amino anthraquinone, le méthylsulfate de 1-N-(méthyl-morpholinium-propyl)amino 4-hydroxy anthraquinone, et le Basic Orange 69 ®.

6. Composition selon l'une quelconque des revendications 1 à 3, caractérisée par le fait que le ou les colorants directs cationiques sont choisis parmi les composés de formules (I), (II), (III), (III'), et (IV) suivantes :
**a) composés de formule (I):** 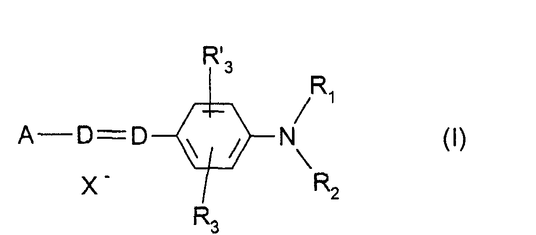 dans laquelle :
- D représente un atome d'azote ou le groupement -CH,
- R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
- R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
- X⁻ représente un anion de préférence choisi parmi le chlorure, le méthyl sulfate et l'acétate,
- A représente un groupement choisi par les structures A1 à A19 suivantes : 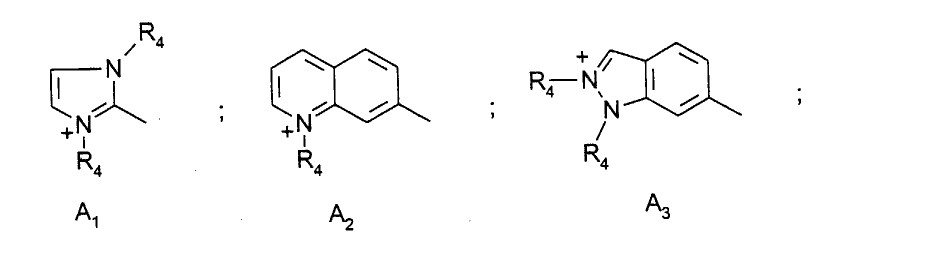 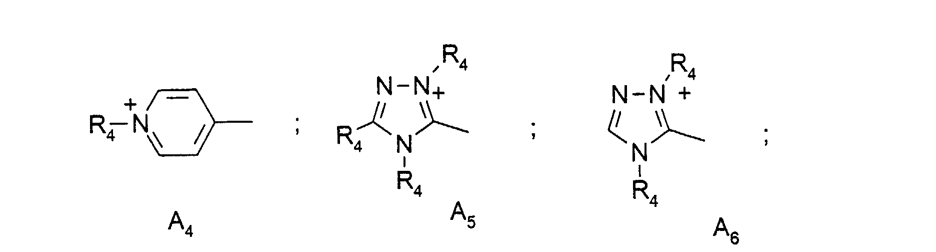 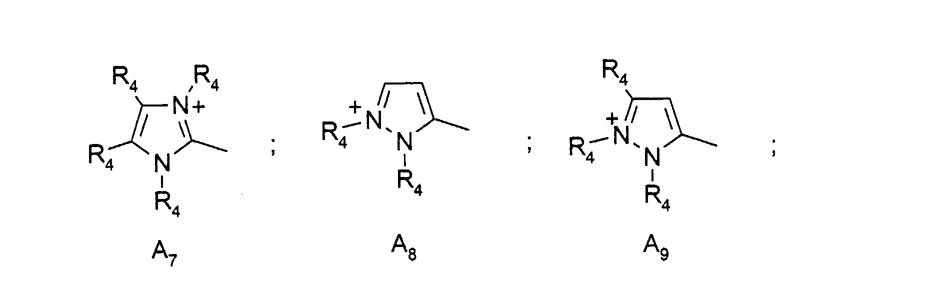 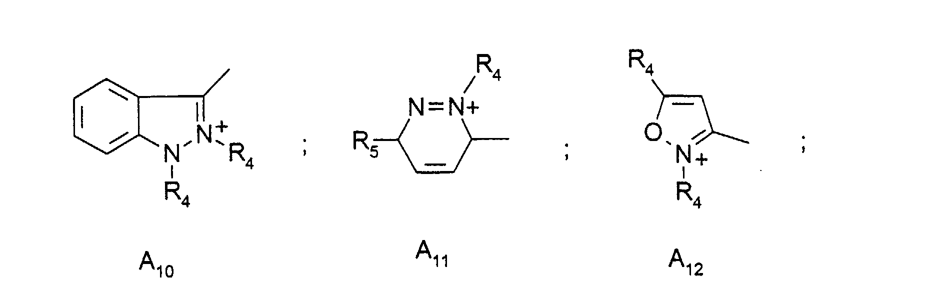 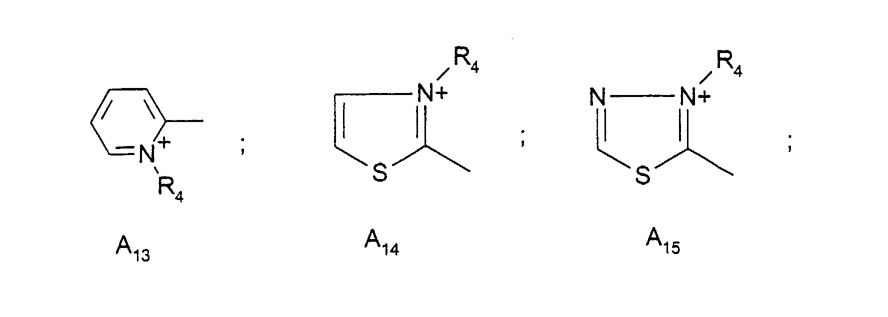 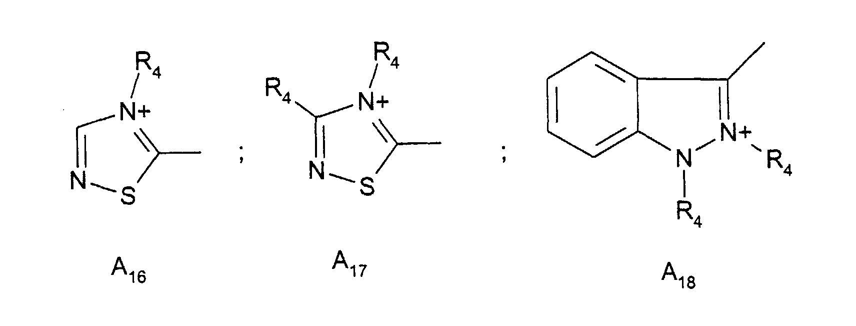 et 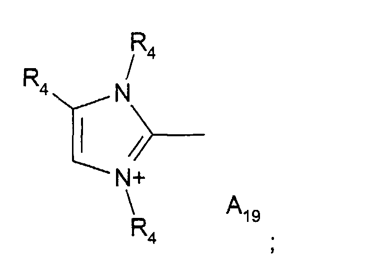 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) composés de formule (II) :** 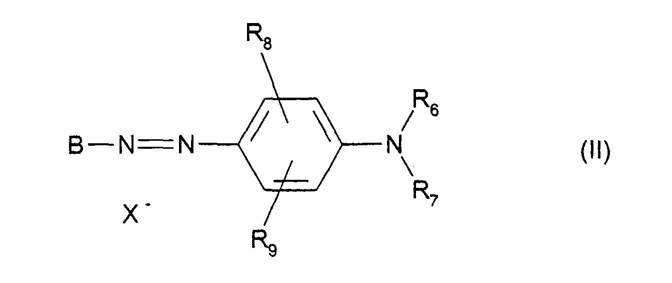 dans laquelle :
- R₆ représente un atome d'hydrogène ou un radical alkyle en C₁-C₄,
- R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
- R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
- X⁻ représente un anion de préférence choisi parmi le chlorure, le méthyl sulfate et l'acétate,
- B représente un groupement choisi par les structures B1 à B6 suivantes : 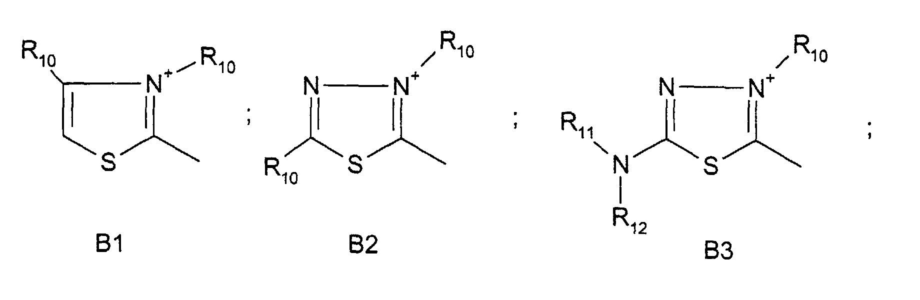 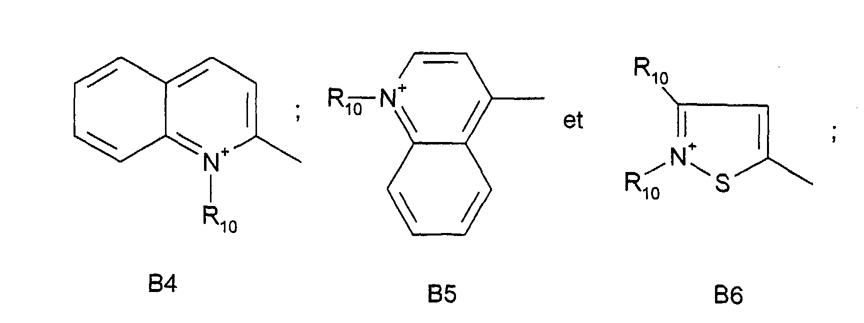 dans lesquelles R₁₀ représente un radical alkyle en C₁-C₄, R₁₁ et R₁₂, identiques ou différents, représentent un atome d'hydrogène ou un radical alkyle en C₁-C₄ ;
**c) composés de formules (III) et (III'):** 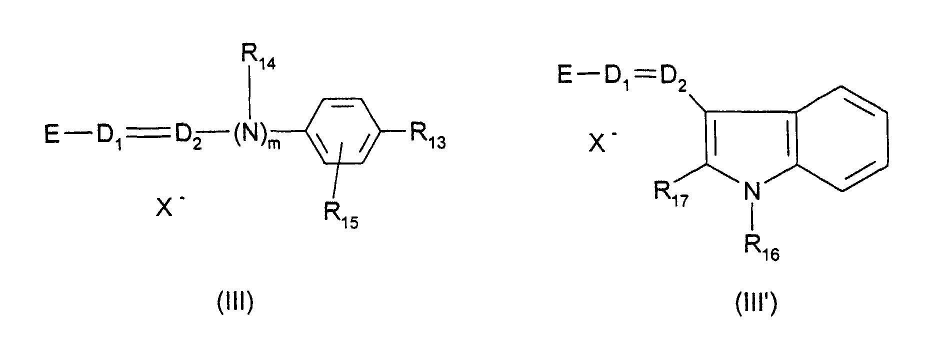 dans lesquelles :
- R₁₃ représente un atome d'hydrogène, un radical alcoxy en C₁-C₄, un atome d'halogène tel que le brome, le chlore, l'iode ou le fluor ou un radical amino,
- R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
- R₁₅ représente un atome d'hydrogène ou d'halogène tel que le brome, le chlore, l'iode ou le fluor,
- R₁₆ et R₁₇, identiques ou différents, représentent un atome d'hydrogène ou un radical alkyle en C₁-C₄,
- D₁ et D₂, identiques ou différents, représentent un atome d'azote ou le groupement -CH,
- m = 0 ou 1,
étant entendu que lorsque R₁₃ représente un groupement amino non substitué, alors D₁ et D₂ représentent simultanément un groupement -CH et m = 0,
- X⁻ représente un anion de préférence choisi parmi le chlorure, le méthyl sulfate et l'acétate,
- E représente un groupement choisi par les structures E1 à E8 suivantes : 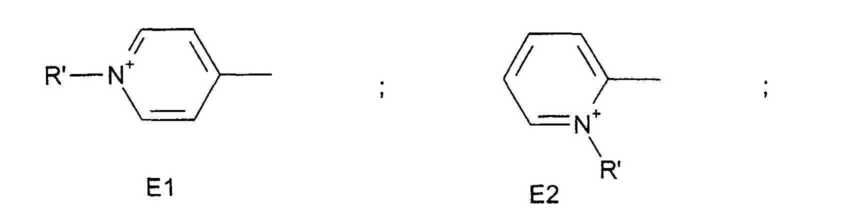 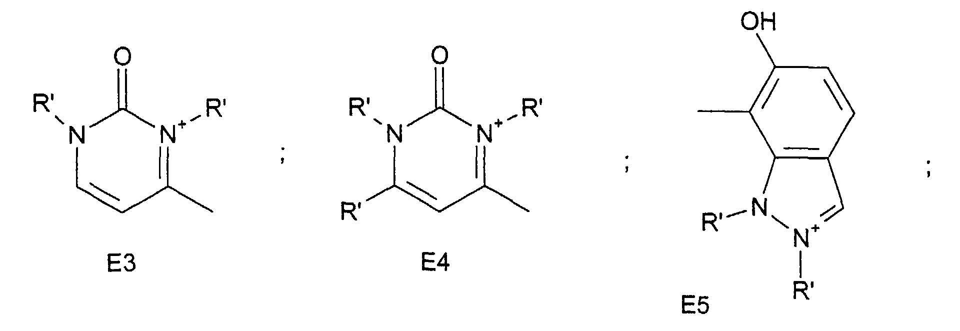 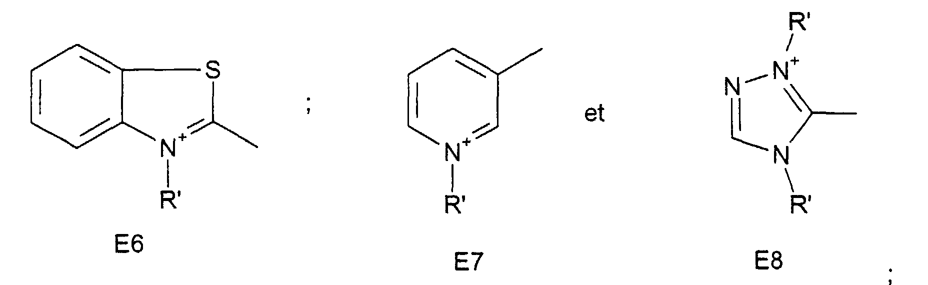 dans lesquelles R' représente un radical alkyle en C₁-C₄ ;
lorsque m = 0 et que D₁ représente un atome d'azote, alors E peut également désigner un groupement de structure E9 suivante : 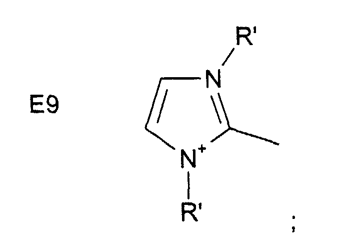 dans laquelle R' représente un radical alkyle en C₁-C₄.
**d) composés de formule (IV) :**
G―N=N―J (IV)
dans laquelle :
- **G** représente un groupement choisi parmi les structures G₁ à G₃ suivantes : 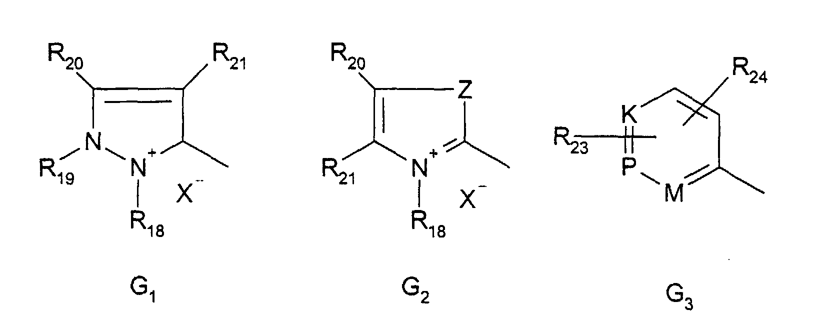
dans lesquelles :
- R₁₈ désigne un radical alkyle en C₁-C₄, un radical phényle, un radical phényle substitué par un radical alkyle en C₁-C₄, ou un atome d'halogène choisi parmi le chlore, le brome, l'iode et le fluor ;
- R₁₉ désigne un radical alkyle en C₁-C₄ ou un radical phényle ;
- R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂ ; R₂₀ peut également désigner un atome d'hydrogène ;
- Z désigne un atome d'oxygène, de soufre ou un groupement -NR₁₉ ;
- M représente un groupement -CH, -CR" (R" désignant alkyle en C₁-C₄), ou -NR₂₂(X⁻)ᵣ ;
- K représente un groupement -CH, -CR" (R" désignant alkyle en C₁-C₄), ou -NR₂₂(X⁻)ᵣ ;
- P représente un groupement -CH, -CR" (R" désignant alkyle en C₁-C₄), ou -NR₂₂(X⁻)ᵣ ; r désigne zéro ou 1 ;
- R₂₂ représente un atome O⁻, un radical alcoxy en C₁-C₄, ou un radical alkyle en C₁-C₄ ;
- R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂ ;
- X⁻ représente un anion de préférence choisi parmi le chlorure, l'iodure, le méthyl sulfate, l'éthyl sulfate, l'acétate et le perchlorate ;
sous réserve que :
- lorsque R₂₂ désigne O⁻, alors r désigne zéro ;
- lorsque K ou P ou M désignent un groupement -N-alkyle en C₁-C₄ X⁻, alors l'un au moins des radicaux R₂₃ et R₂₄ est différent d'un atome d'hydrogène ;
- lorsque K désigne -NR₂₂(X⁻)ᵣ, alors M = P = -CH ou -CR";
- lorsque M désigne -NR₂₂(X⁻)ᵣ, alors K = P = -CH ou -CR";
- lorsque P désigne -NR₂₂(X⁻)ᵣ, alors K = M et désignent -CH ou -CR ;
- lorsque Z désigne un atome de soufre et que R₂₁ désigne un radical alkyle en C₁-C₄, alors R₂₀ est différent d'un atome d'hydrogène ;
- lorsque Z désigne -NR₂₂, et que R₁₉ désigne un radical alkyle en C₁-C₄, alors au moins un des radicaux R₁₈, R₁₉ ou R₂₀ du groupement de structure G₂ est différent d'un radical alkyle en C₁-C₄ ;
- **J** représente :
• soit un groupement de structure J₁ suivante : 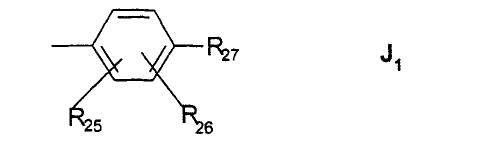 dans laquelle :
- R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène et le soufre ;
- R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre ;
- R₂₇ représente un atome d'hydrogène, un radical -OH, un radical -NHR₂₈, ou un radical -NR₂₉R₃₀ ;
- R₂₈ représente un atome d'hydrogène, un radical alkyle en C₁-C₄, un radical monohydroxyalkyle en C₁-C₄, polyhydroxyalkyle en C₂-C₄, ou un radical phényle ;
- R₂₉ et R₃₀, identiques ou différents, représentent un radical alkyle en C₁-C₄, un radical monohydroxyalkyle en C₁-C₄, ou polyhydroxyalkyle en C₂-C₄ ;
• soit un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 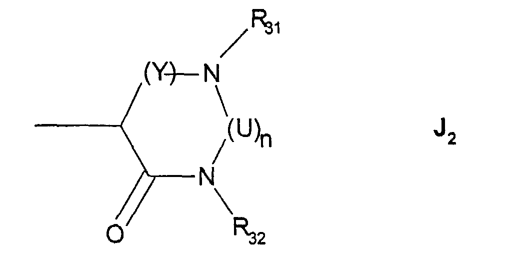 dans laquelle :
- R₃₁ et R₃₂, identiques ou différents, représentent un atome d'hydrogène, un radical alkyle en C₁-C₄, ou un radical phényle ;
- Y désigne un radical -CO- ou le radical 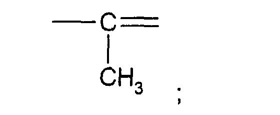
- n = 0 ou 1, étant entendu que lorsque n = 1, alors U désigne le radical -CO-.

7. Composition selon la revendication 6, caractérisée par le fait que les colorants directs cationiques de formule (I) sont choisis parmi les composés répondant aux structures (I1) à (I52) suivantes : 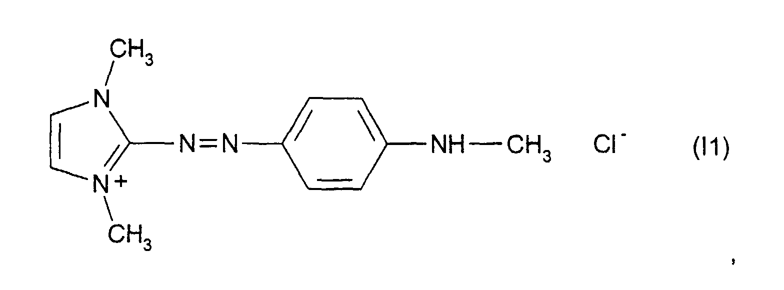 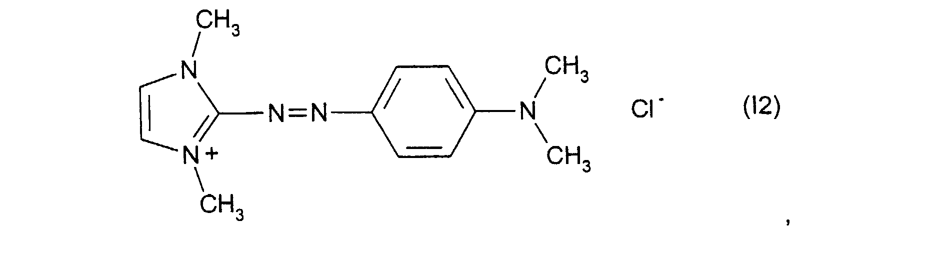 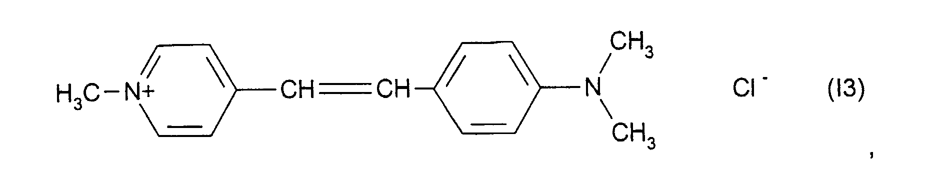 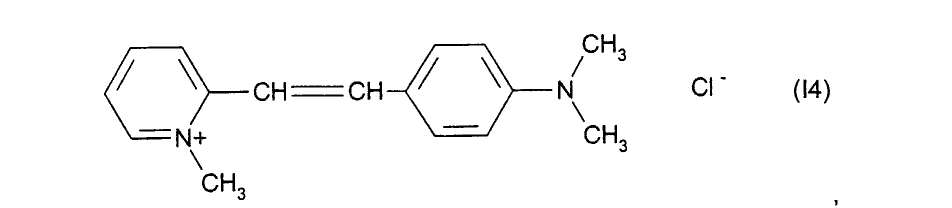 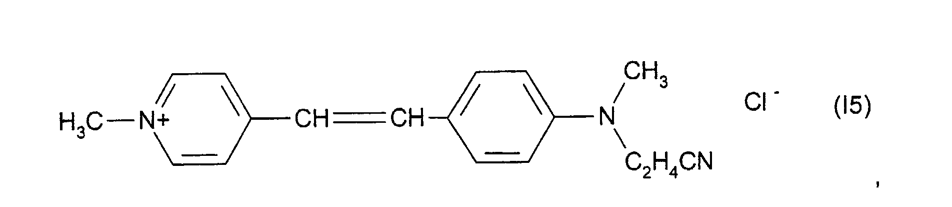 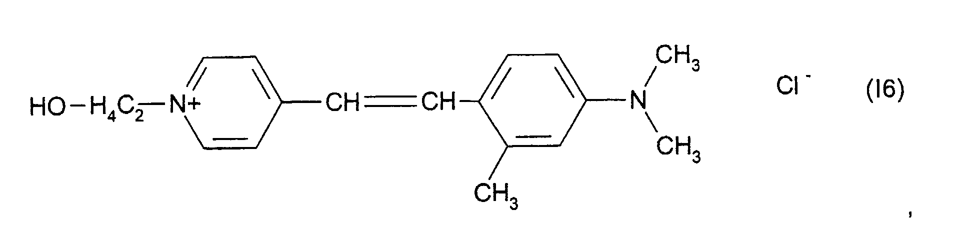 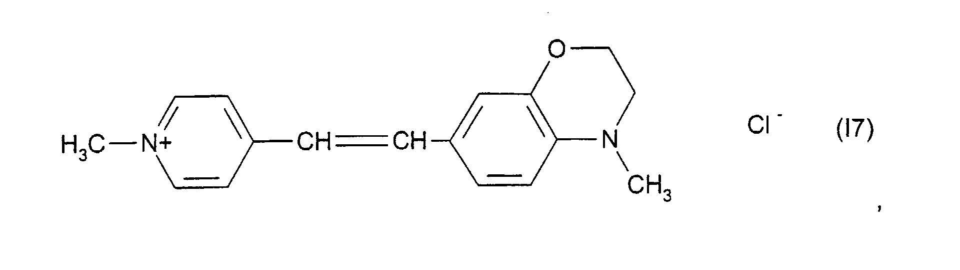 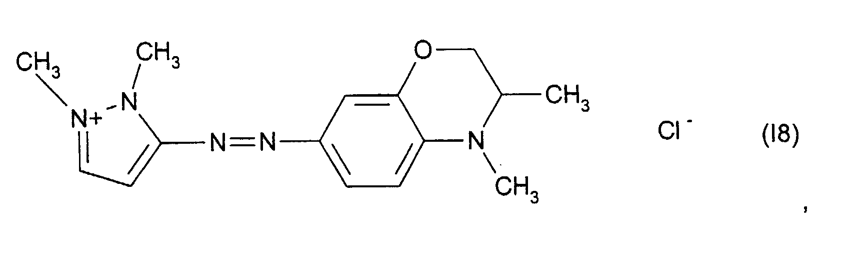 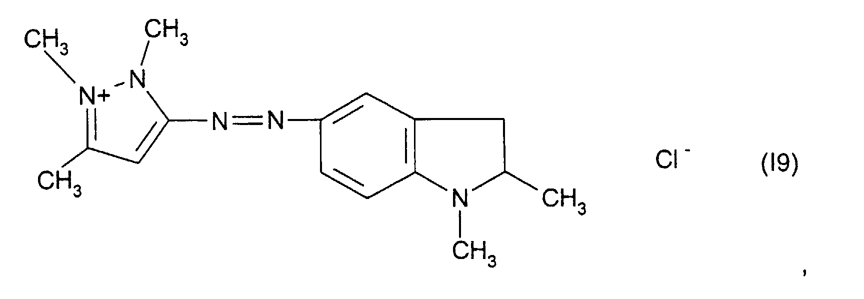 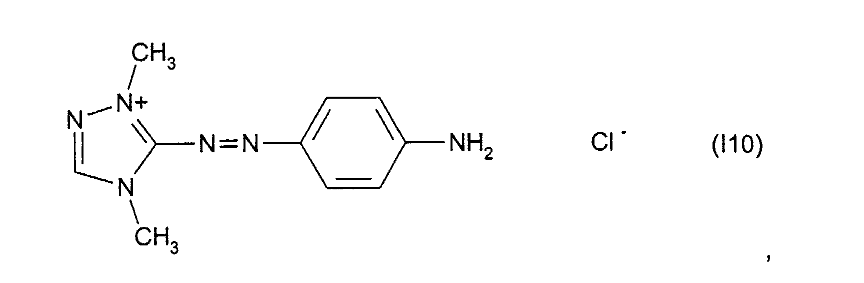 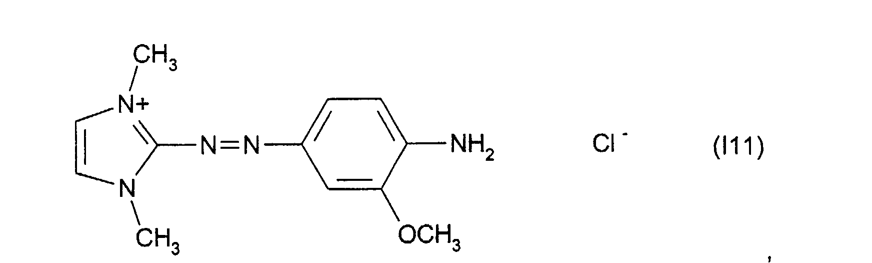 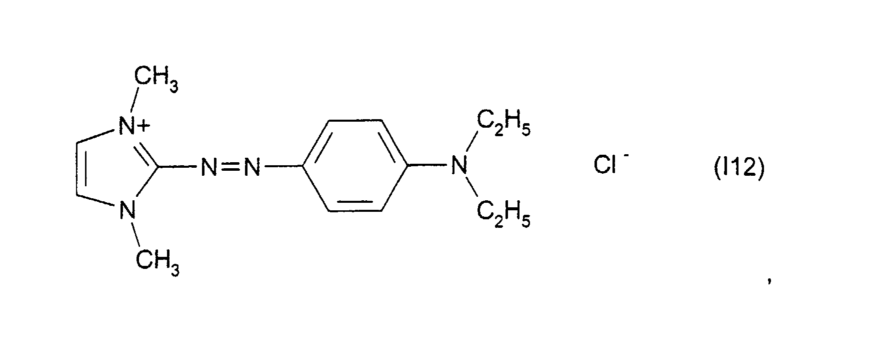 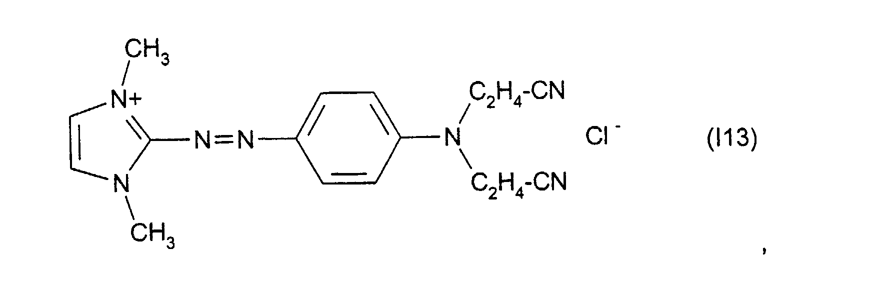 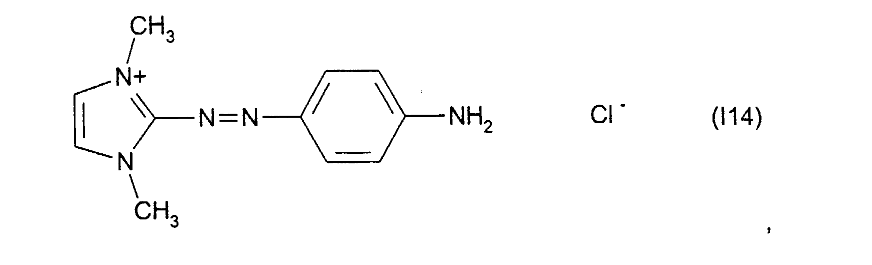 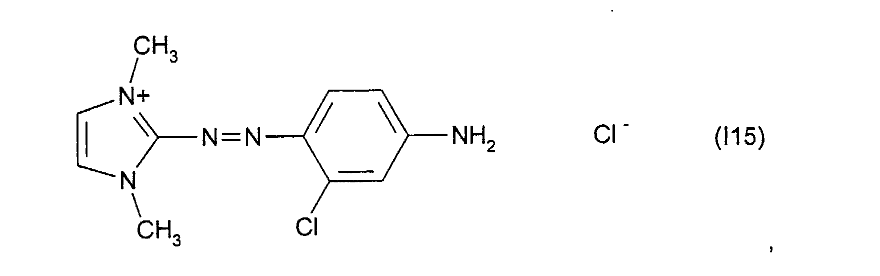 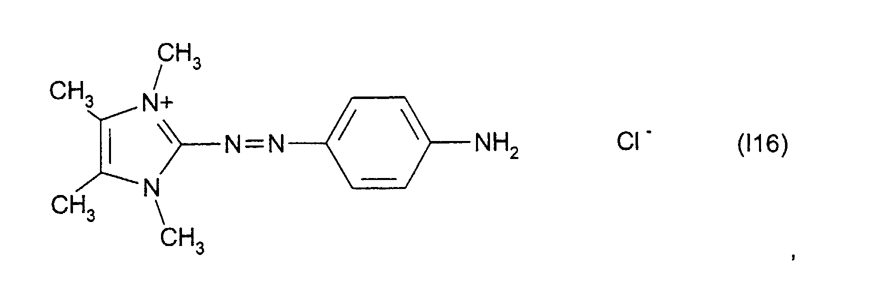 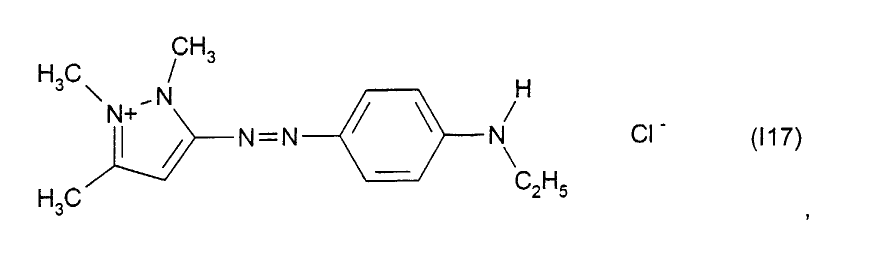 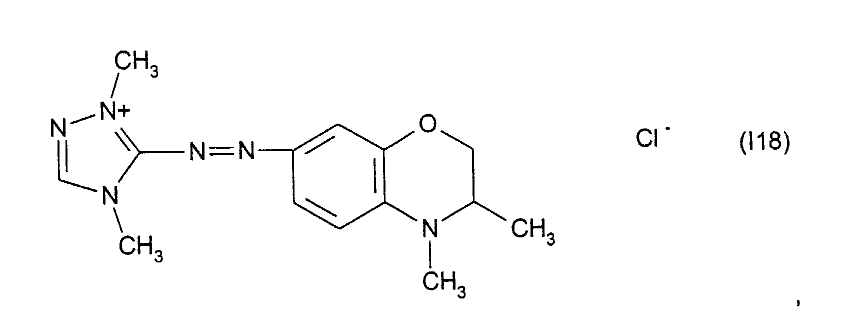 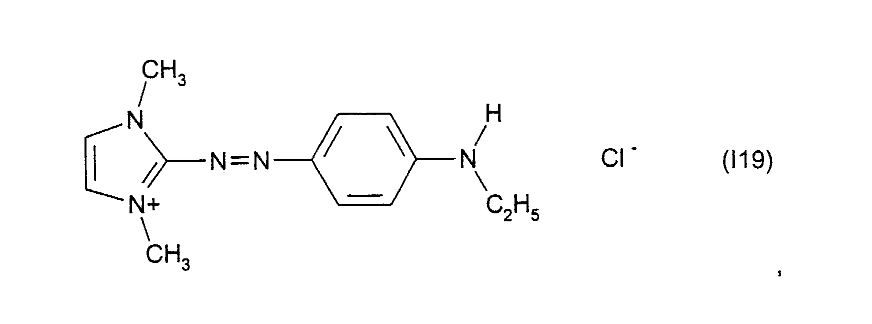 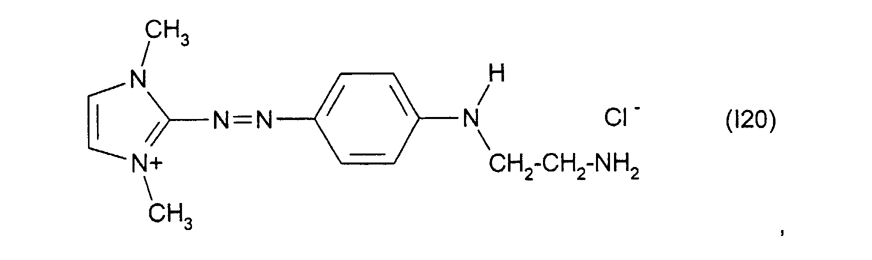 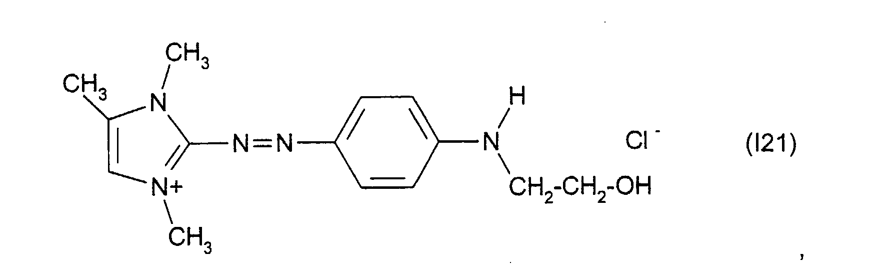 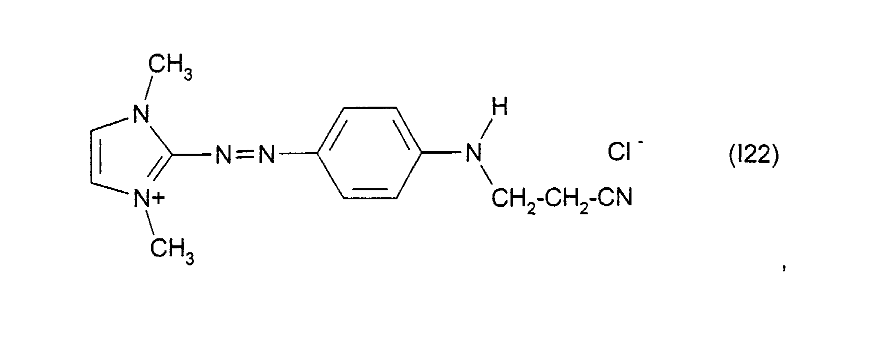 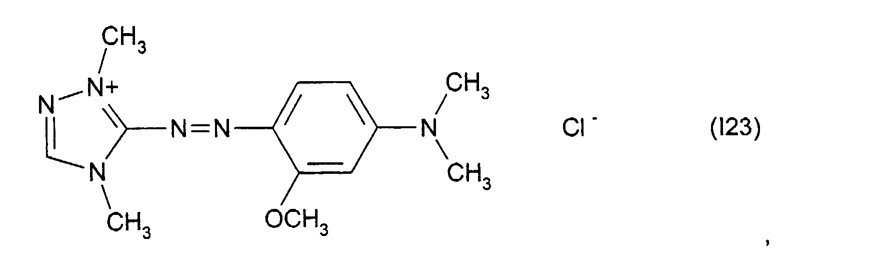 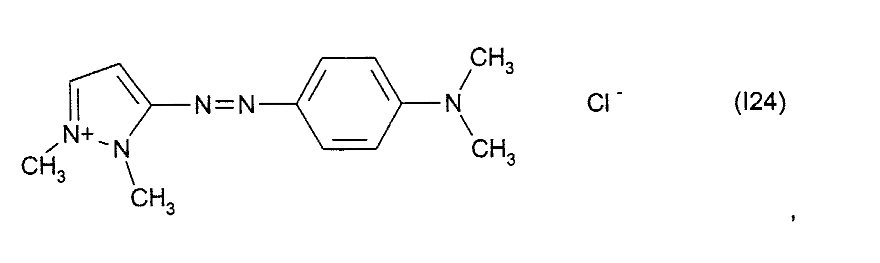 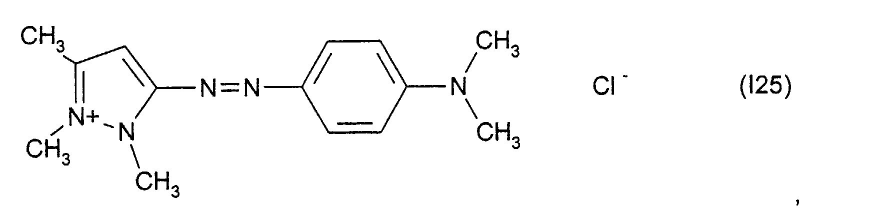 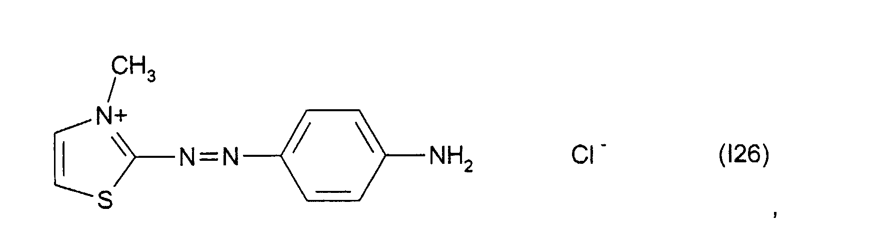 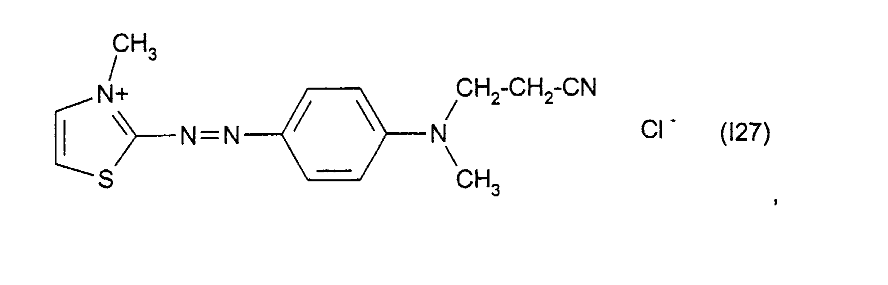 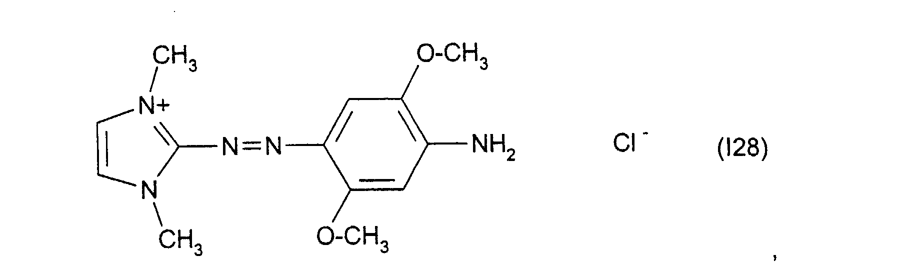 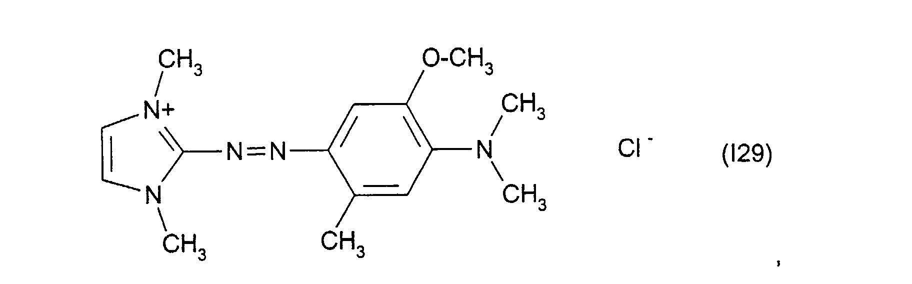 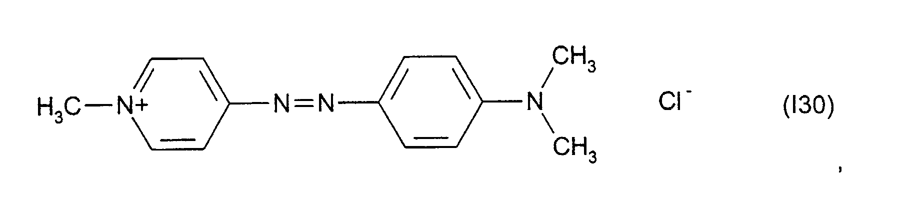 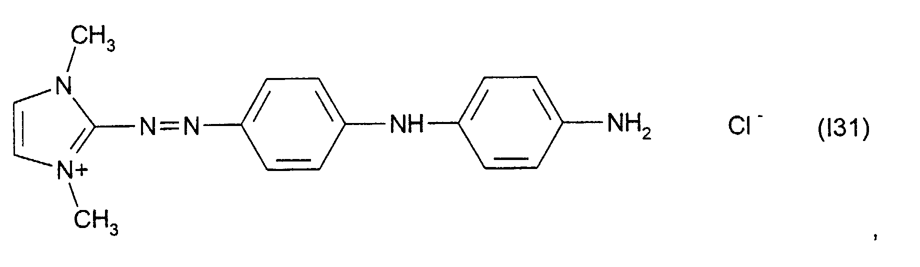 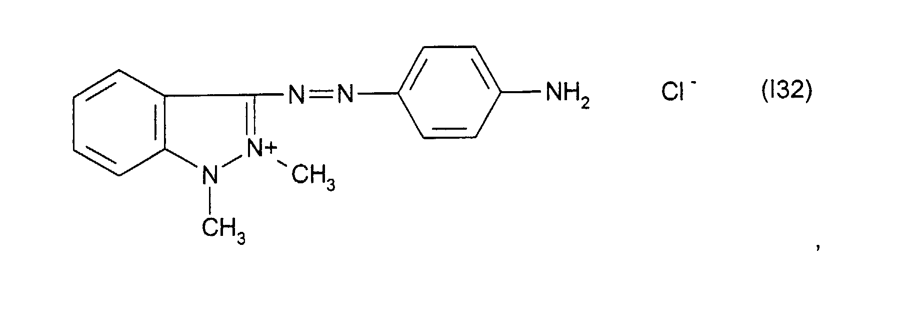 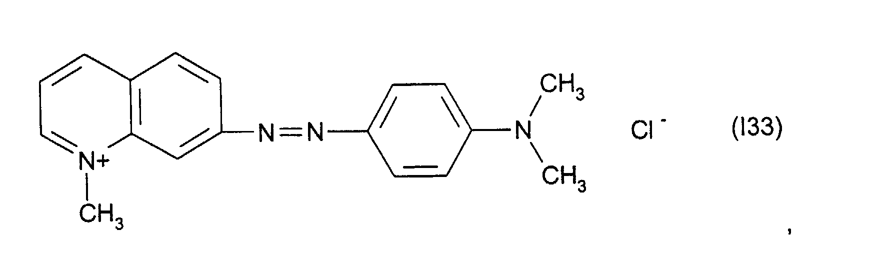 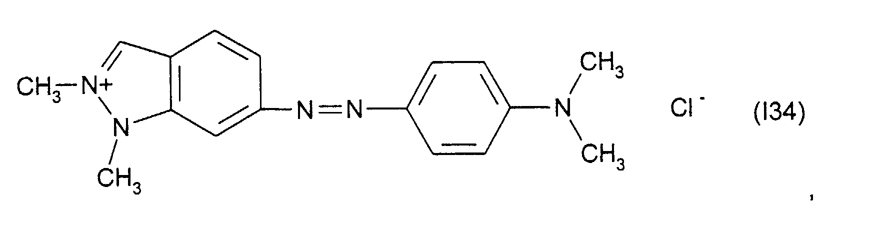 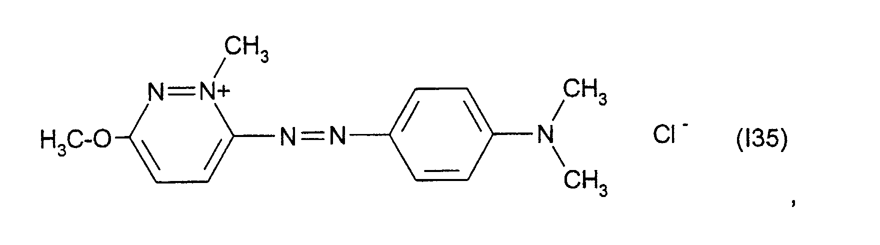 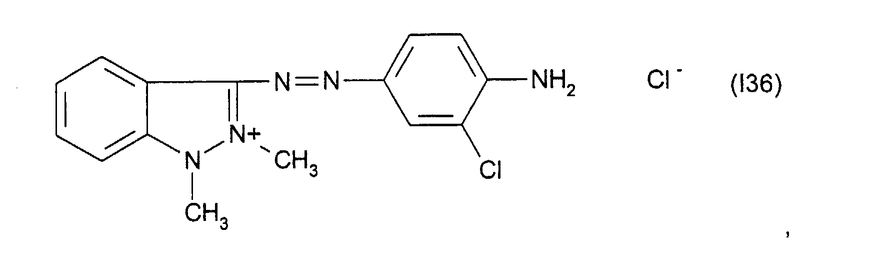 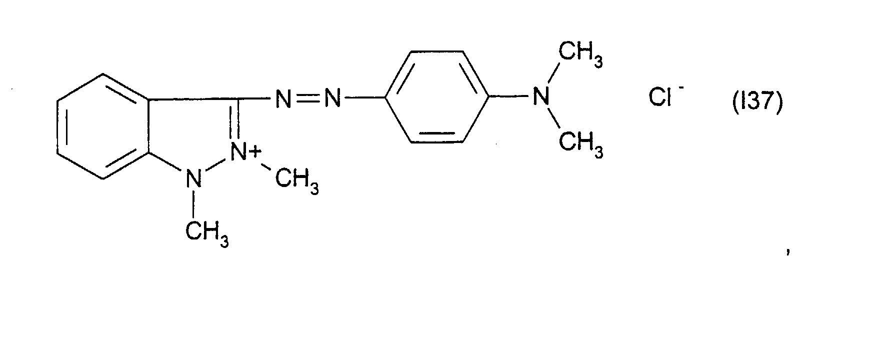 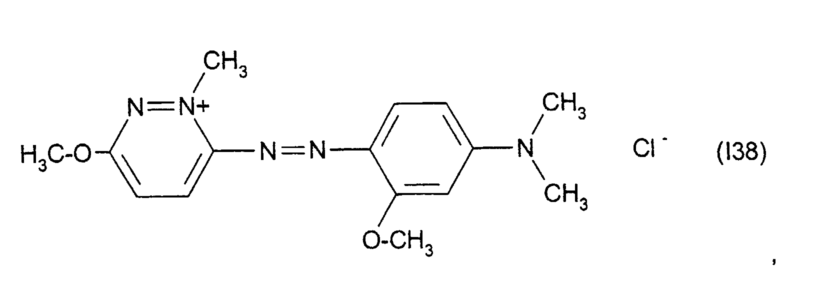 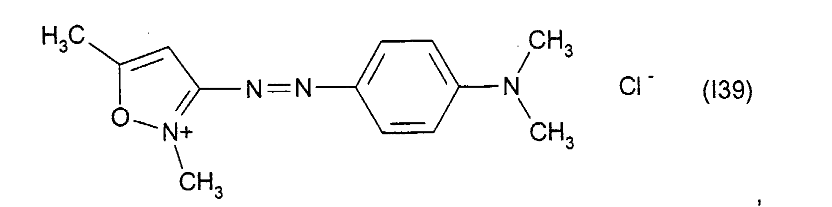 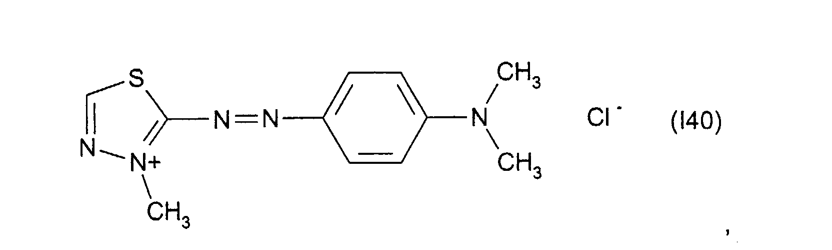 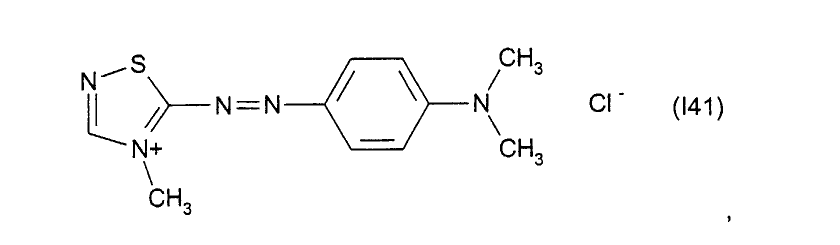 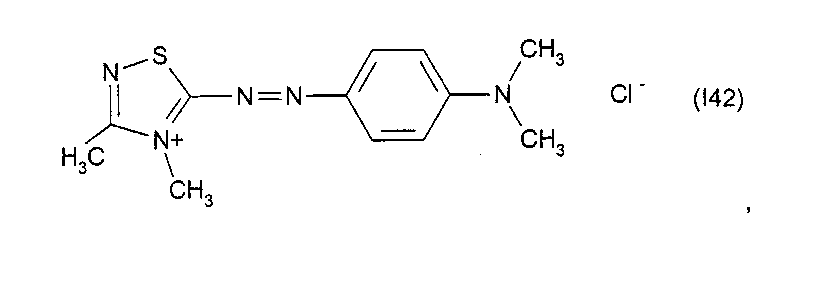 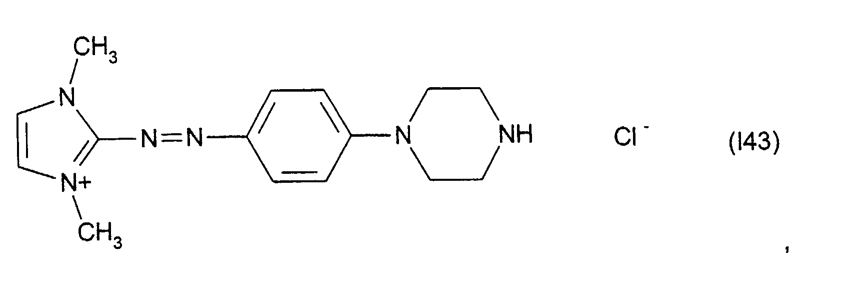 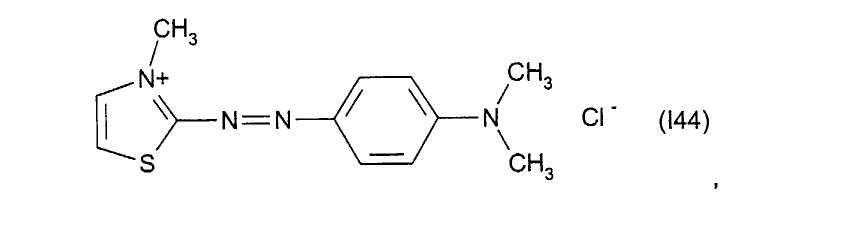 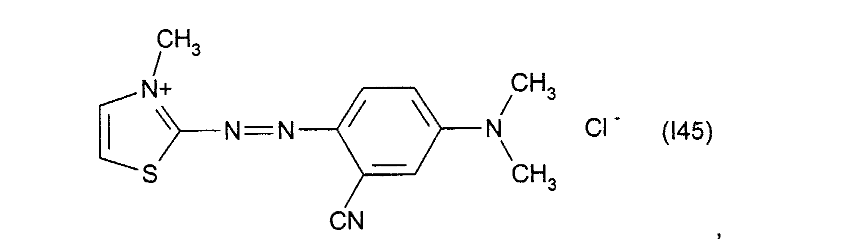 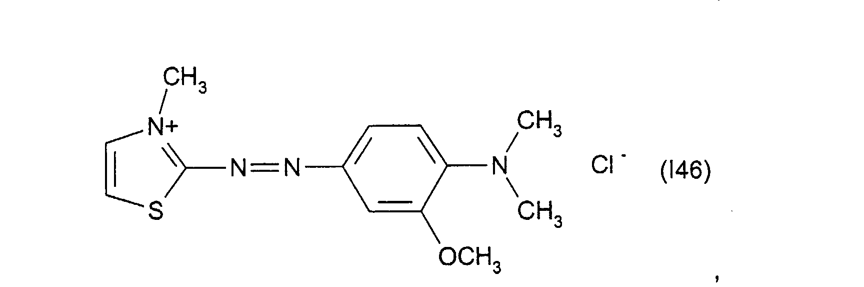 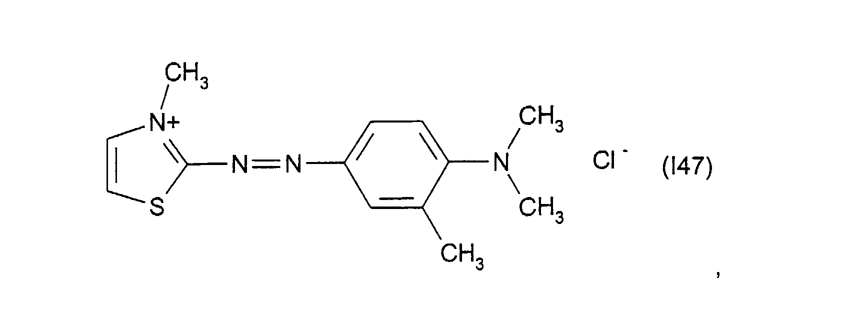 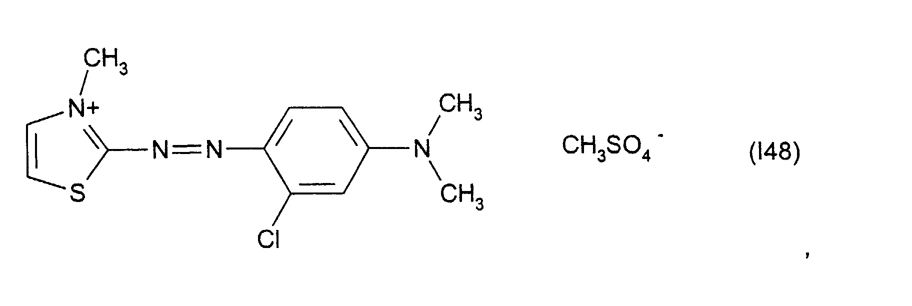 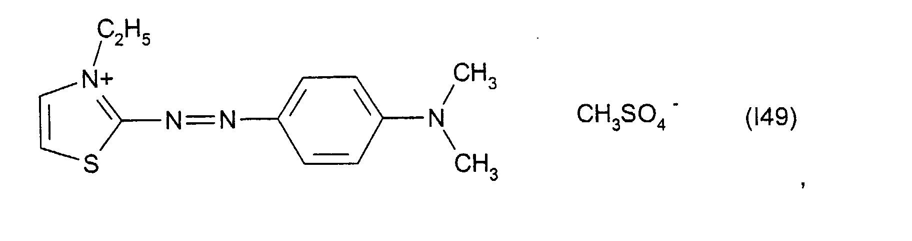 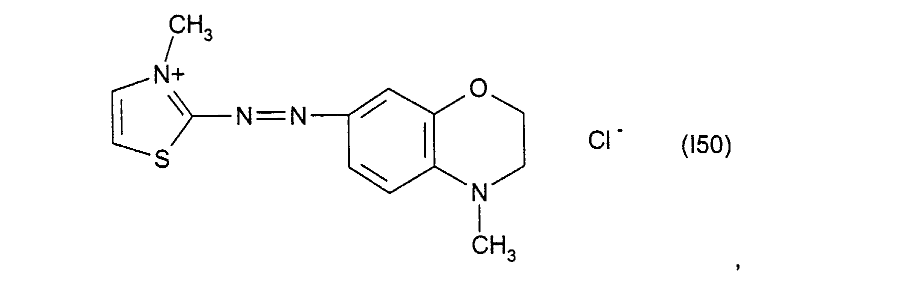 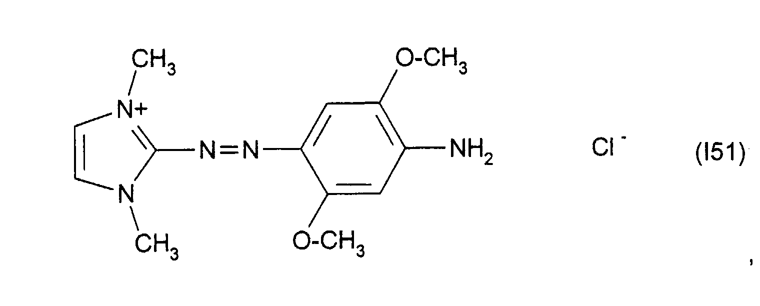 et

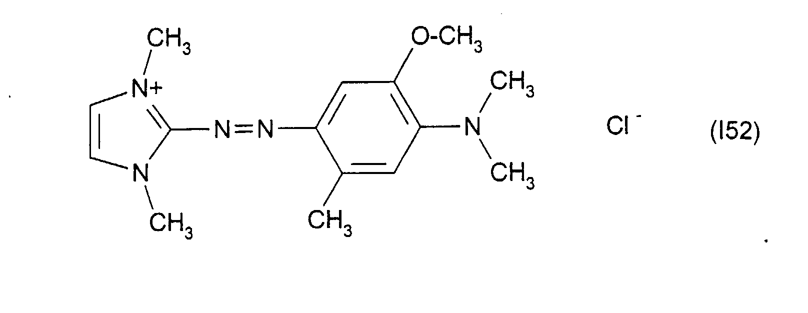

8. Composition selon la revendication 6, caractérisée par le fait que les colorants directs cationiques de formule (II) sont choisis parmi les composés répondant aux structures (II1) à (II12) suivantes : 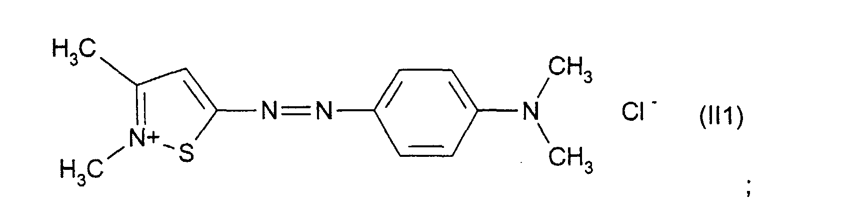 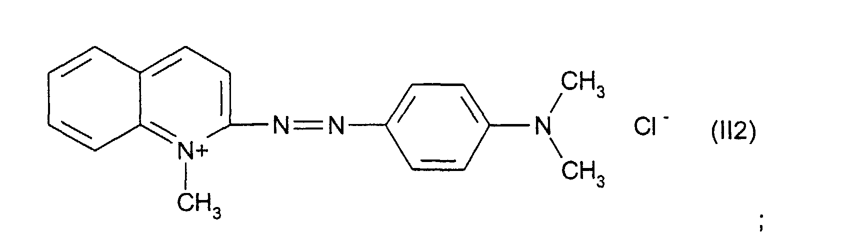 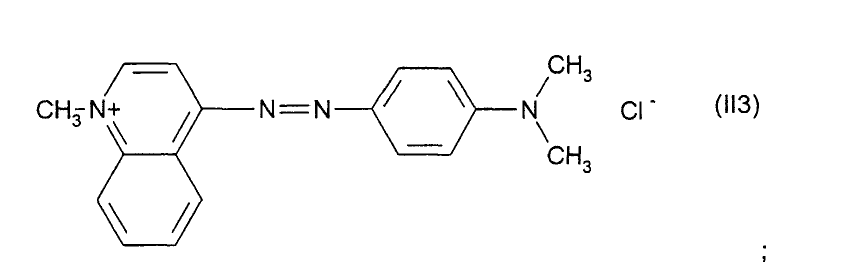 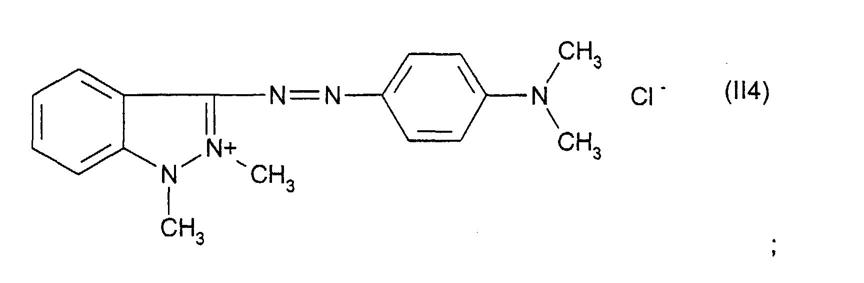 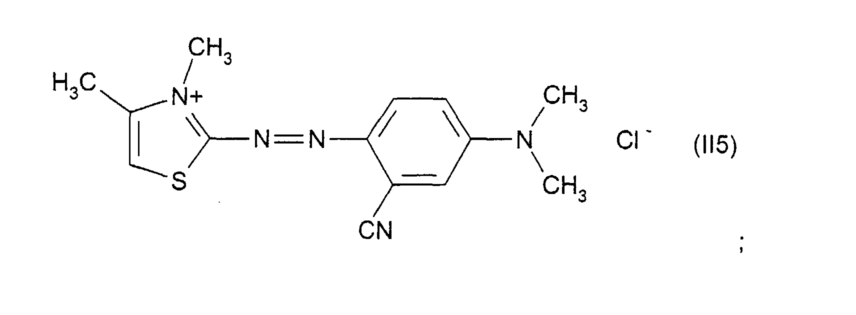 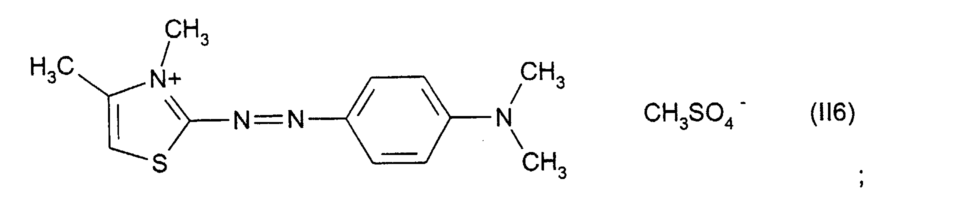 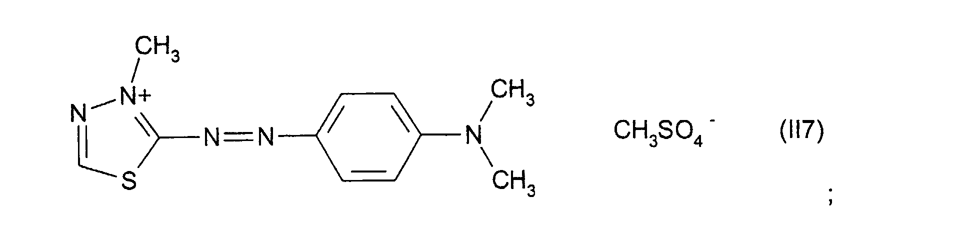 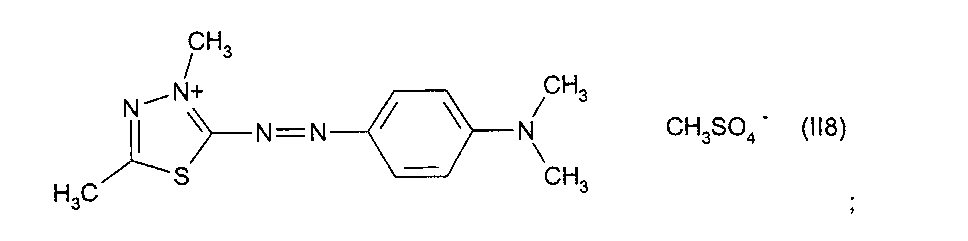 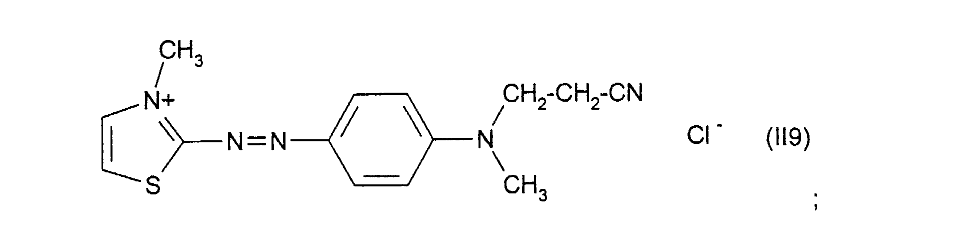 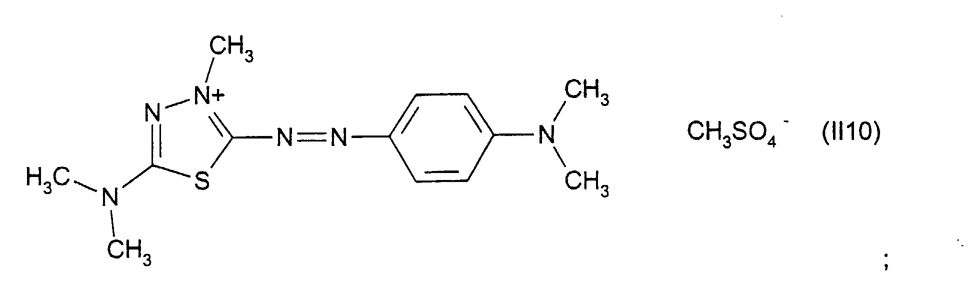 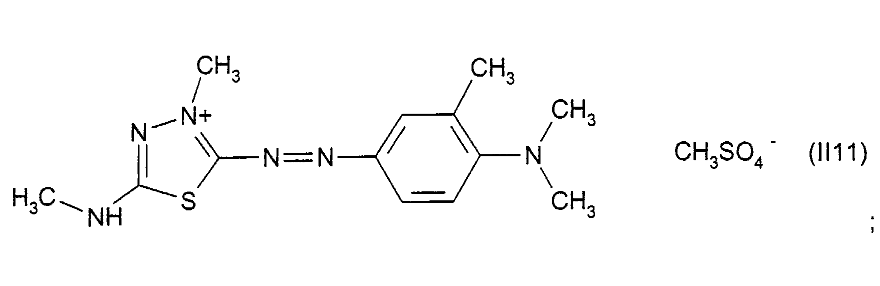 et

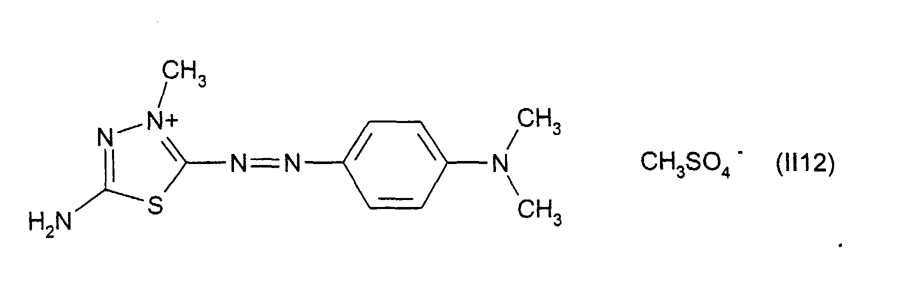

9. Composition selon la revendication 6, caractérisée par le fait que les colorants directs cationiques de formule (III) sont choisis parmi les composés répondant aux structures (III1) à (III18) suivantes : 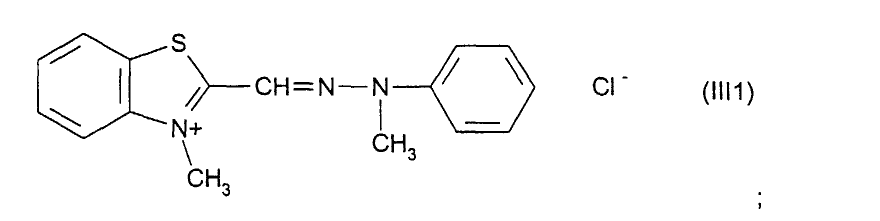 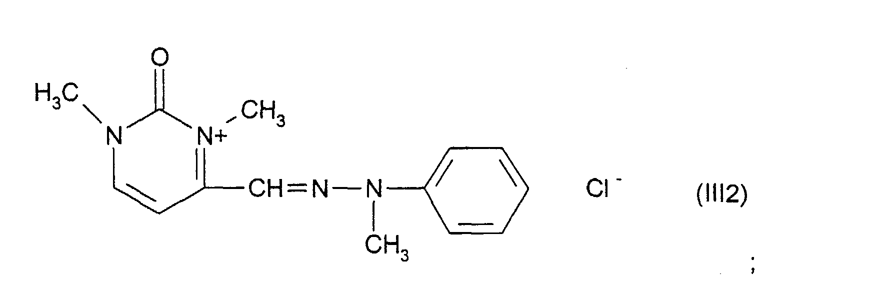 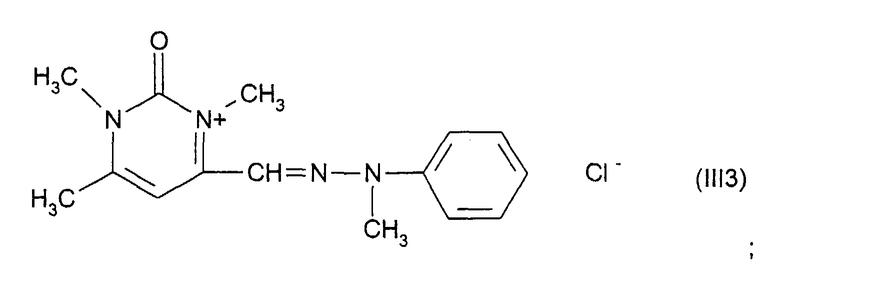 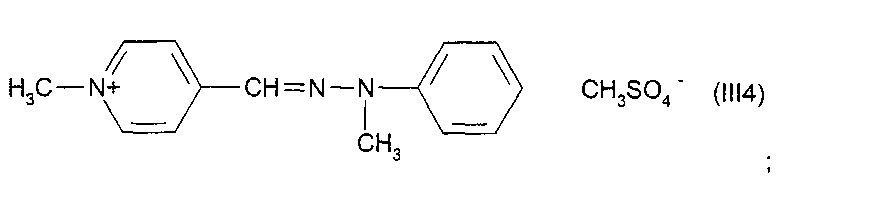 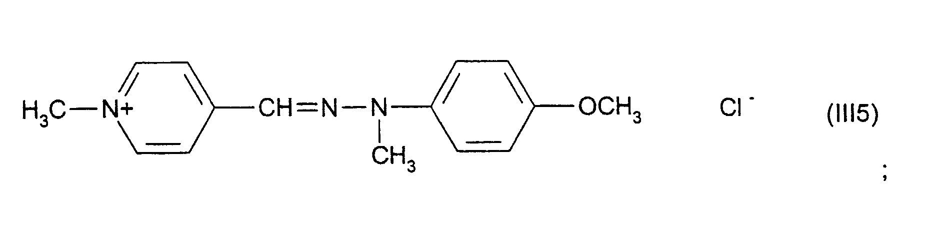 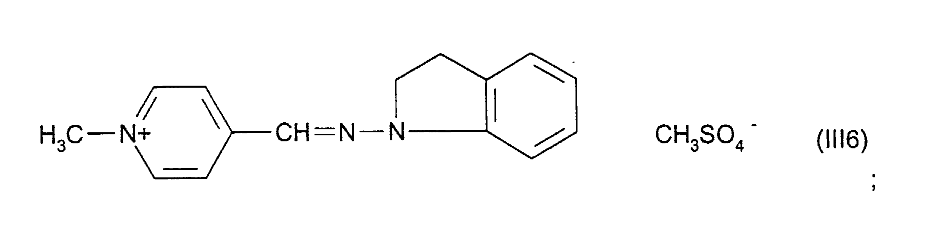 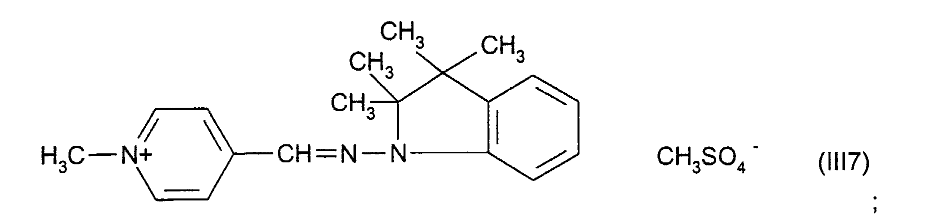 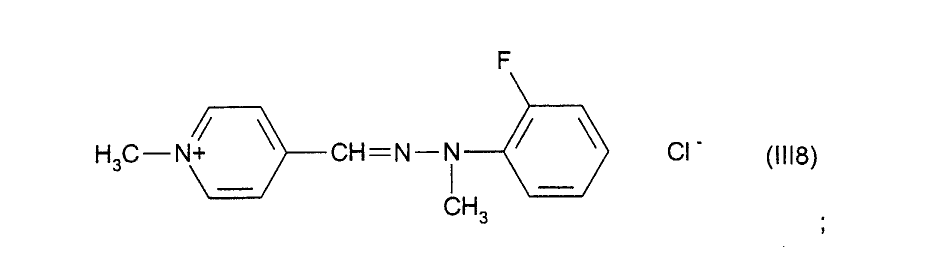 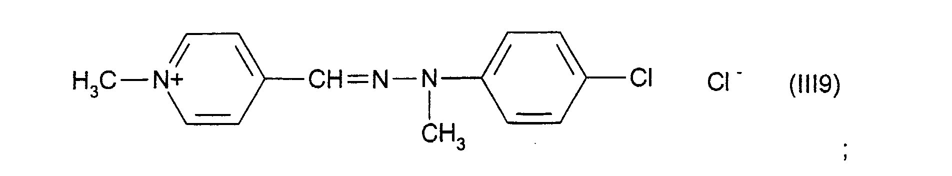 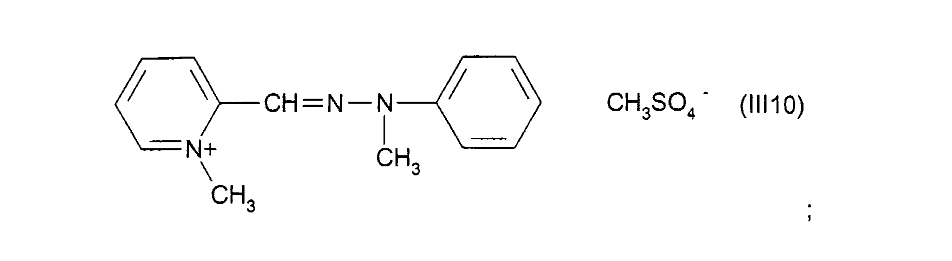 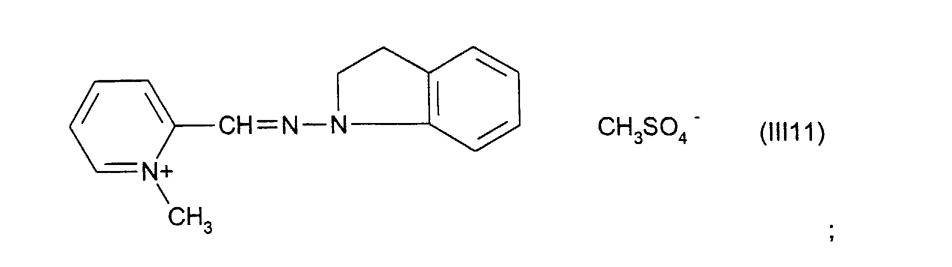 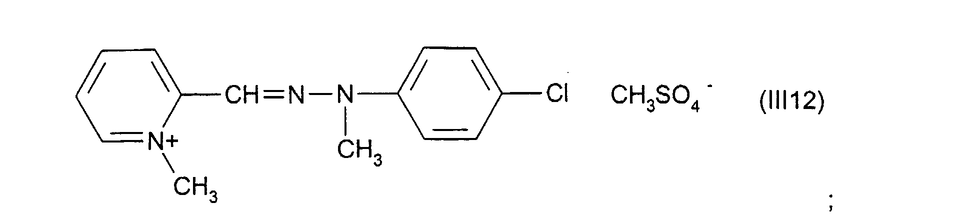 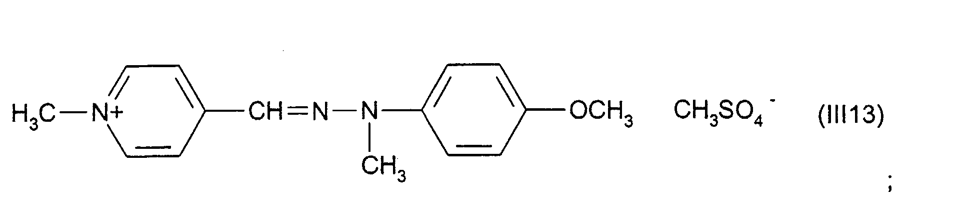 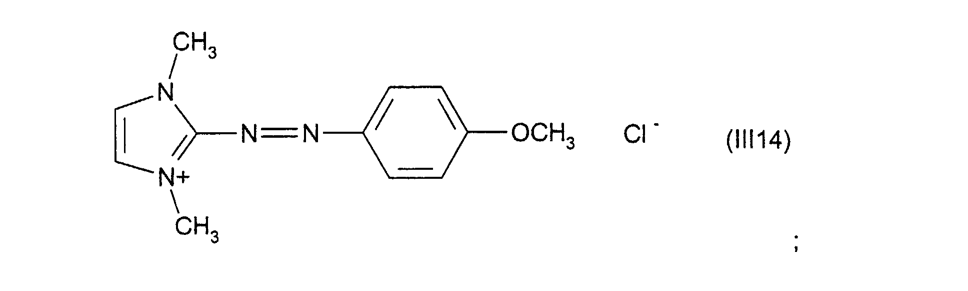 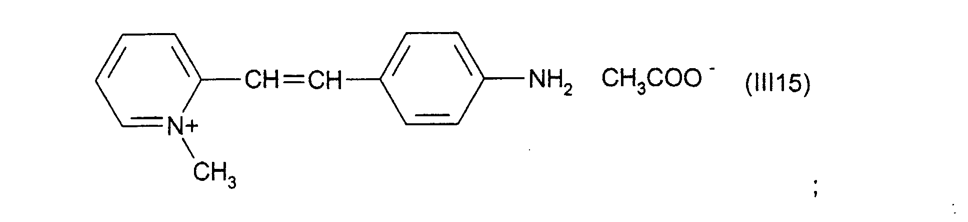 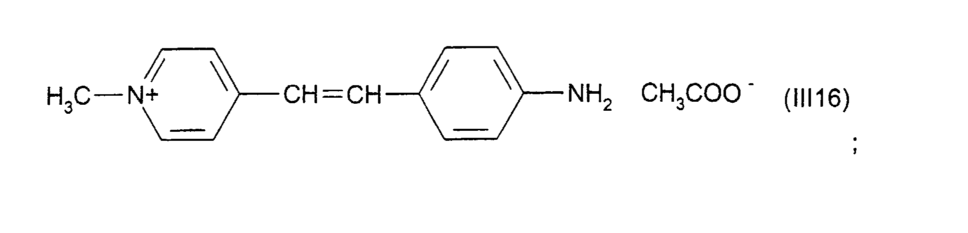 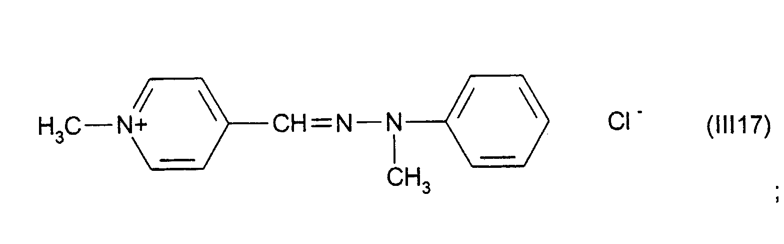 et

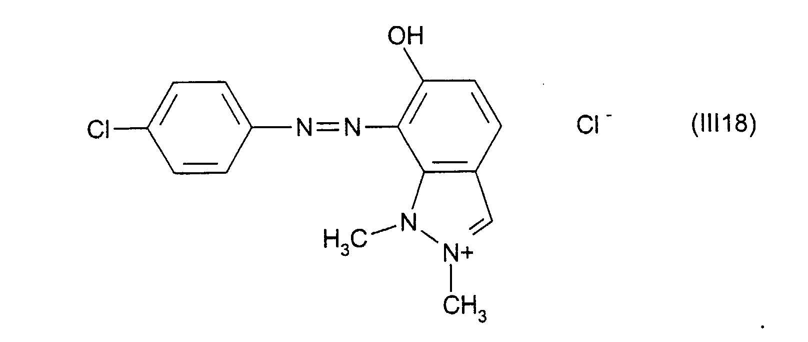

10. Composition selon la revendication 6, caractérisée par le fait que les colorants directs cationiques de formule (III') sont choisis parmi les composés répondant aux structures (III'1) à (III'3) suivantes : 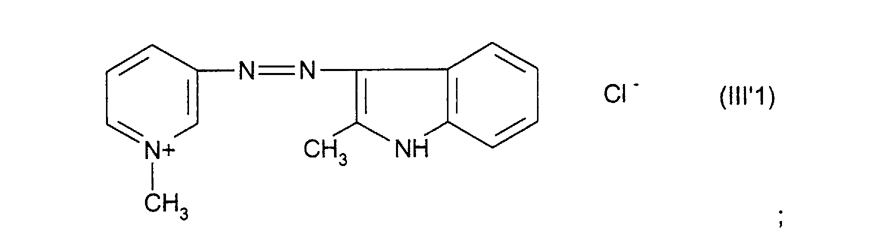 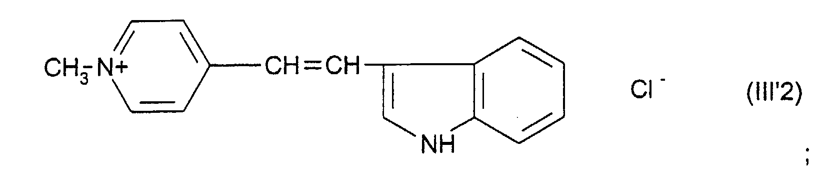 et

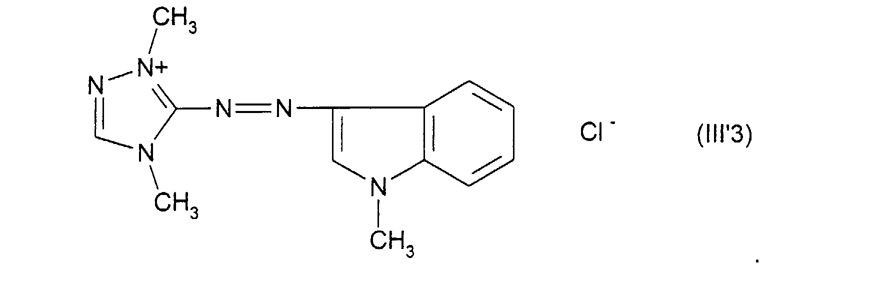

11. Composition selon la revendication 6, caractérisée par le fait que les colorants directs cationiques de formule (IV) sont choisis parmi les composés répondant aux structures (IV)₁ à (IV)₇₇ suivantes : 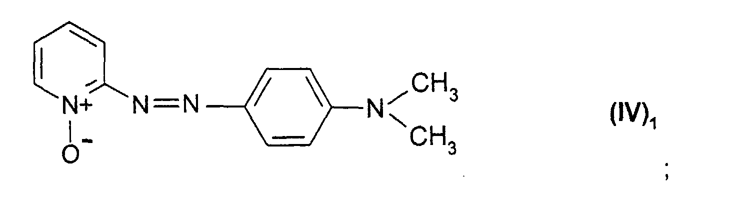 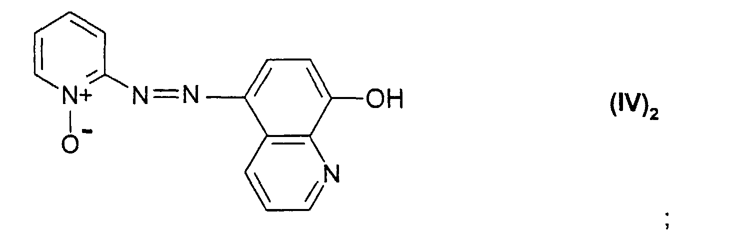 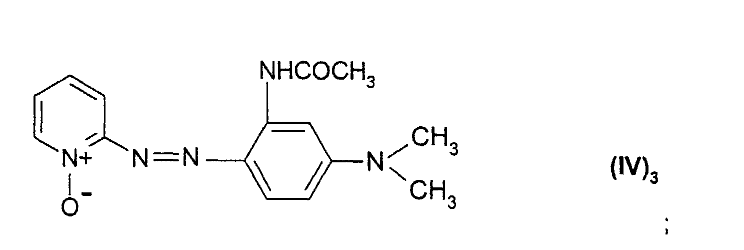 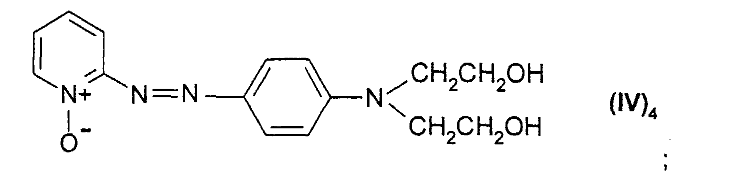 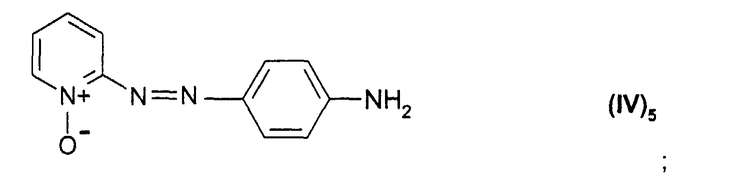 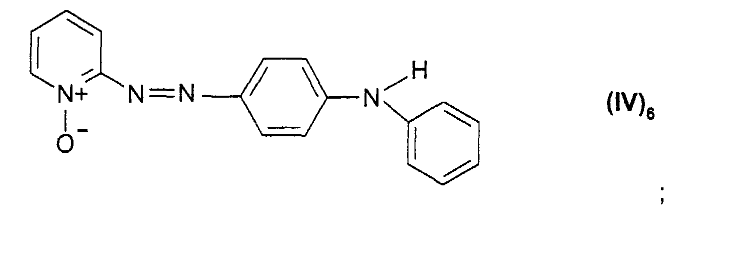 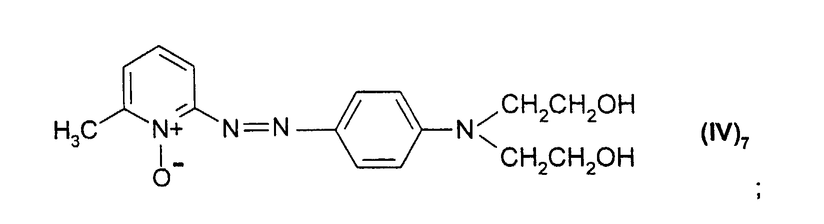 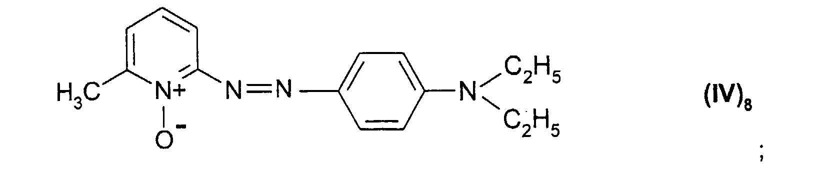 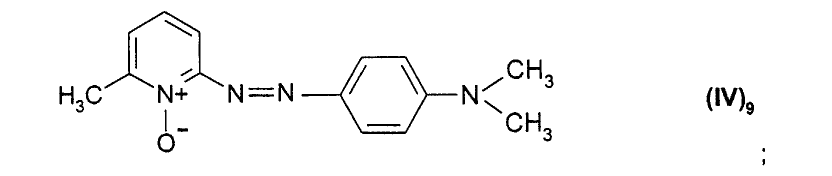 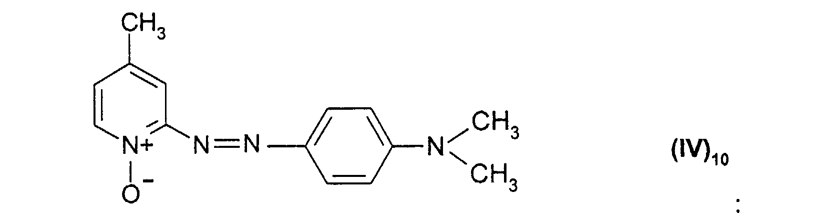 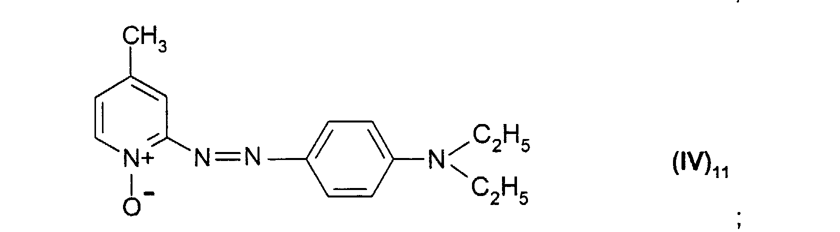 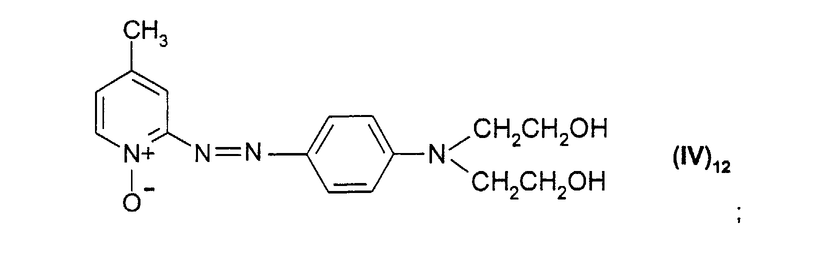 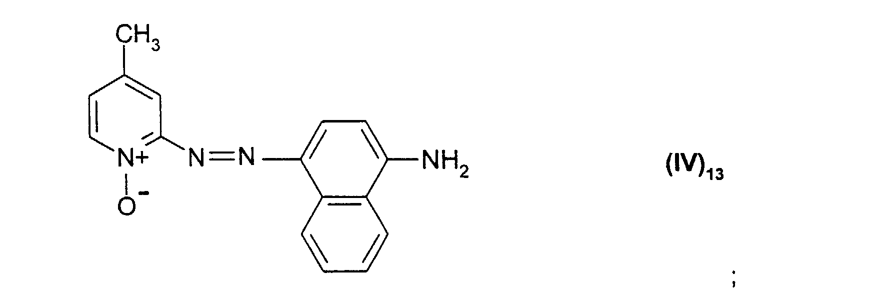 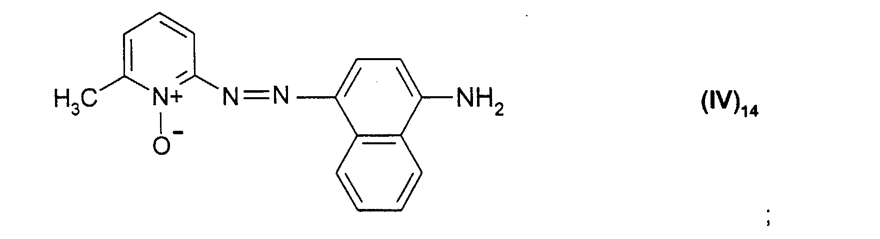 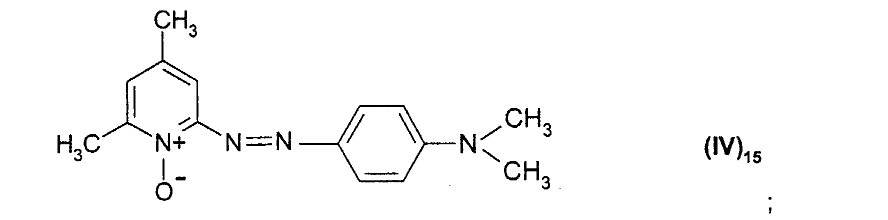 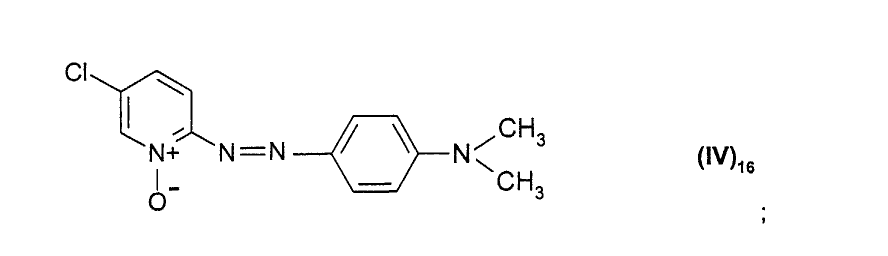 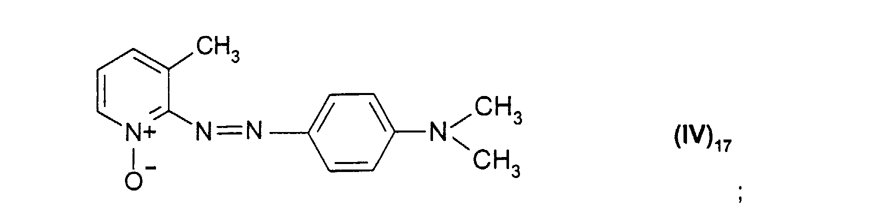 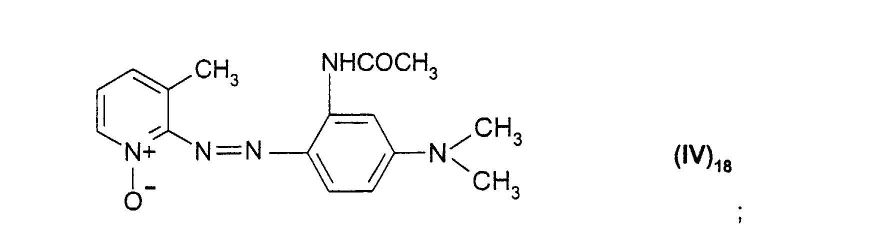 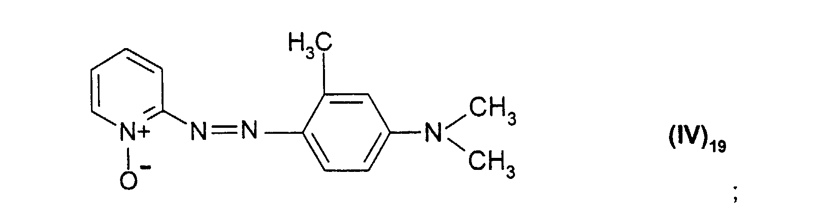 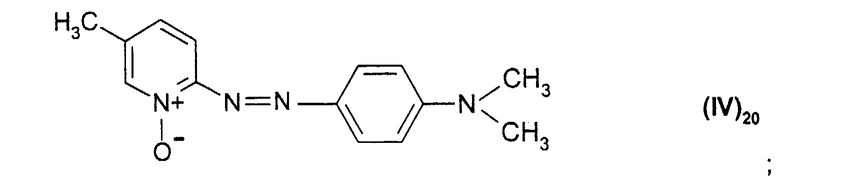 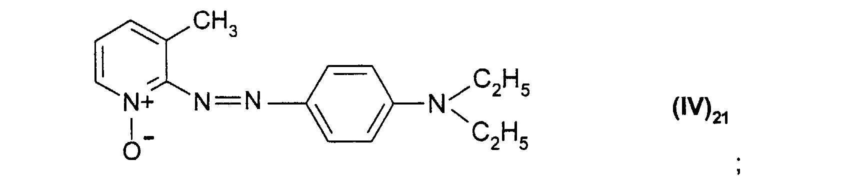 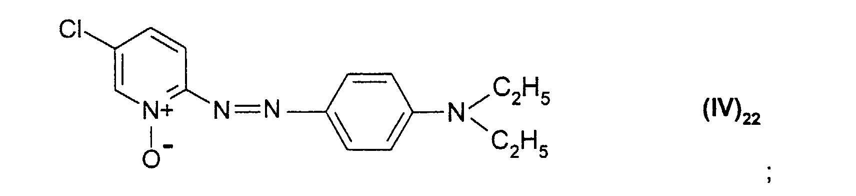 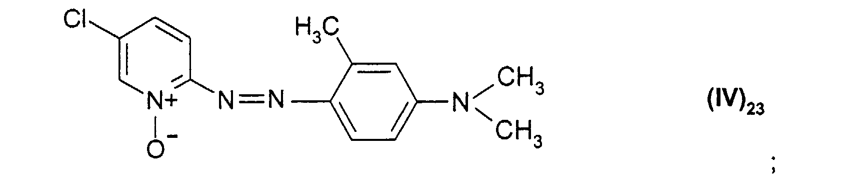 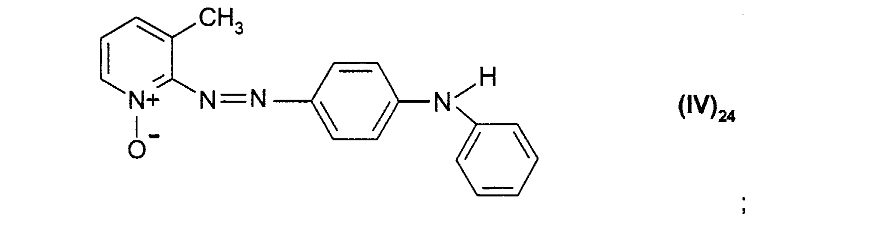 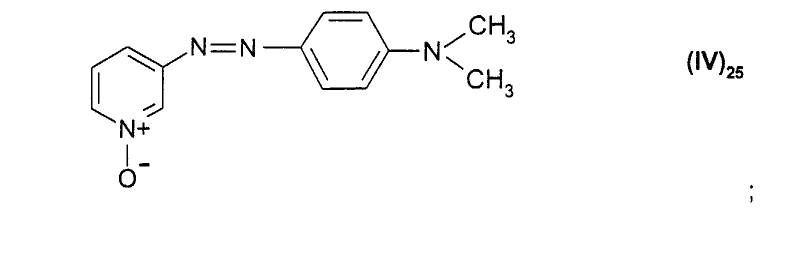 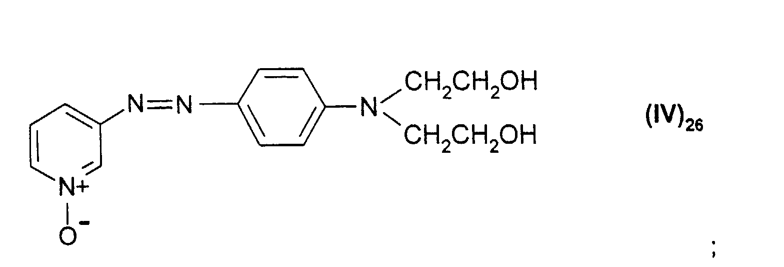 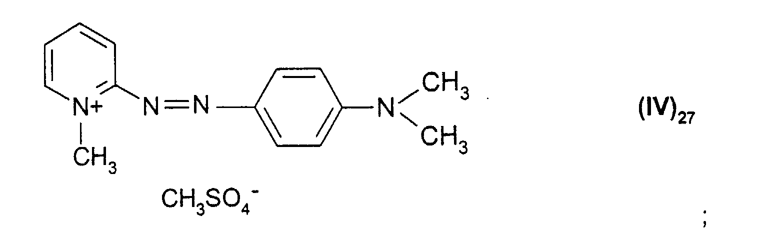 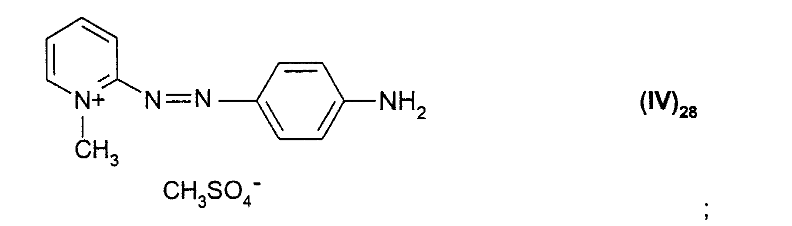 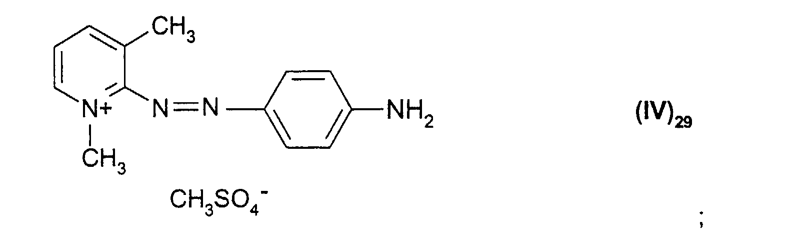 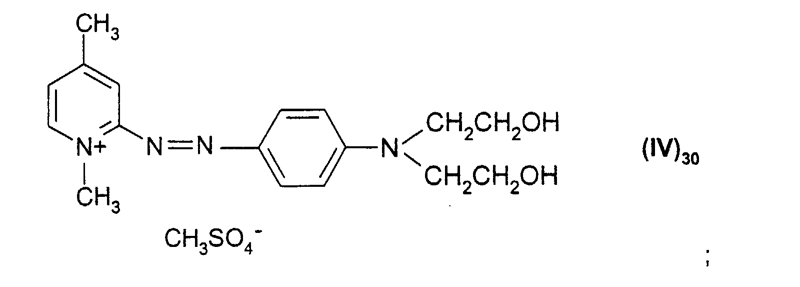 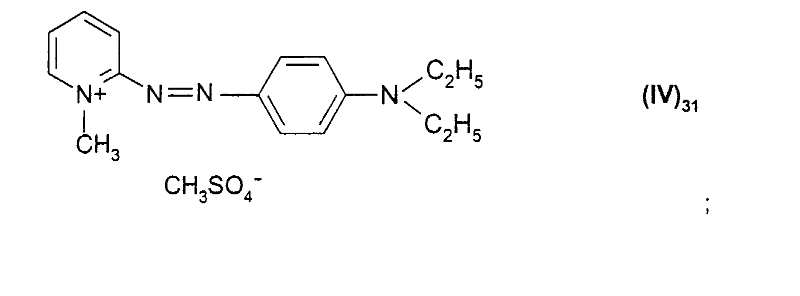 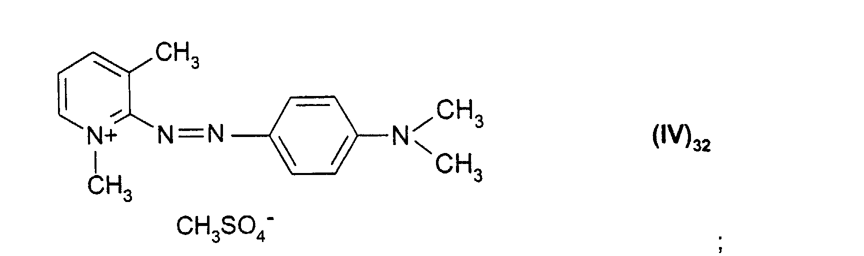 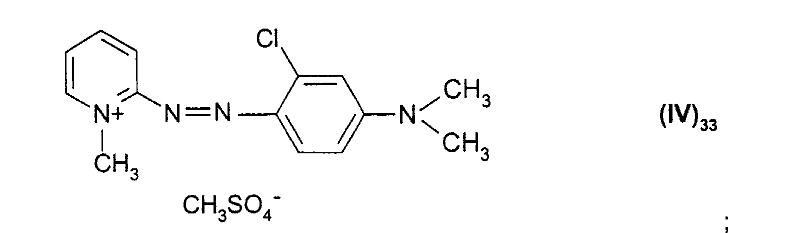 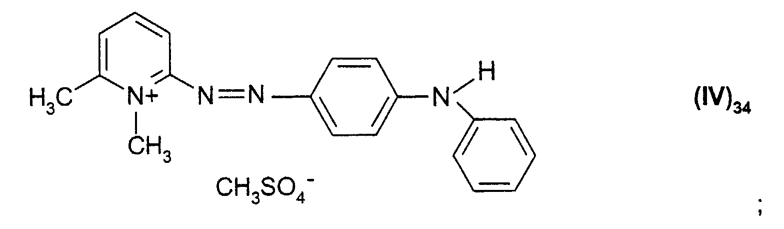 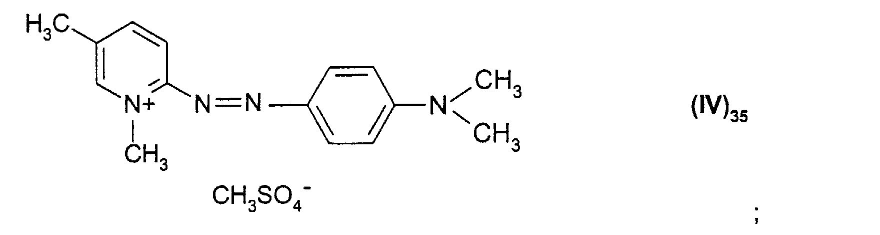 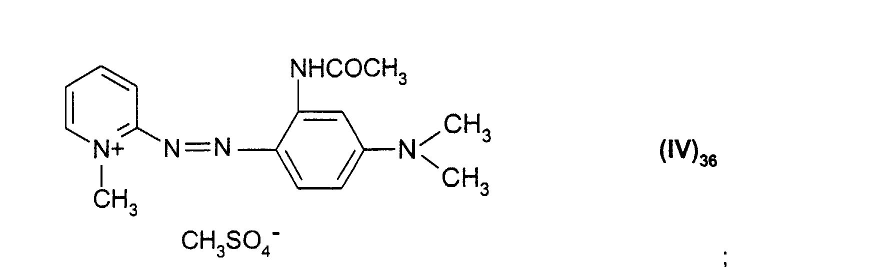 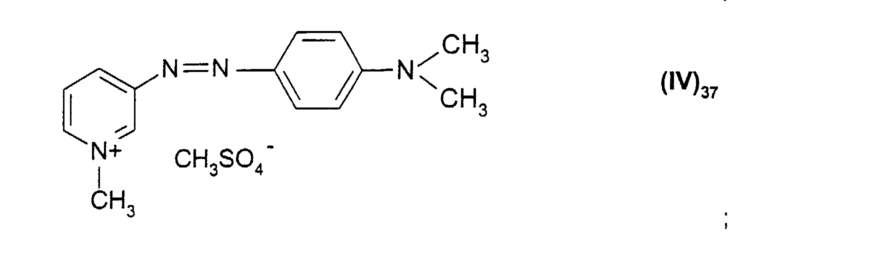 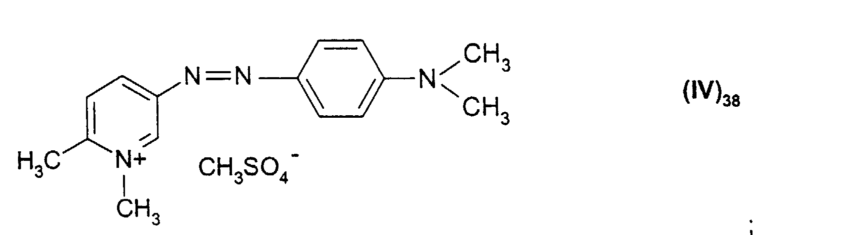 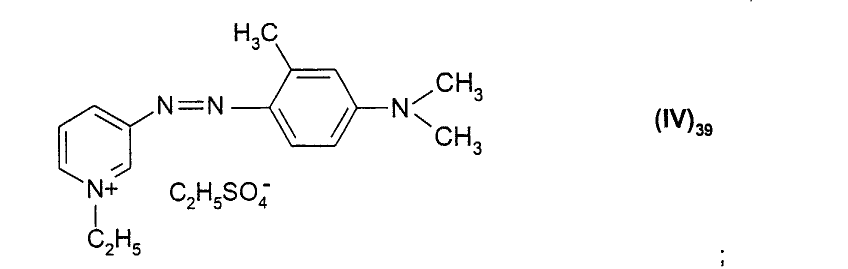 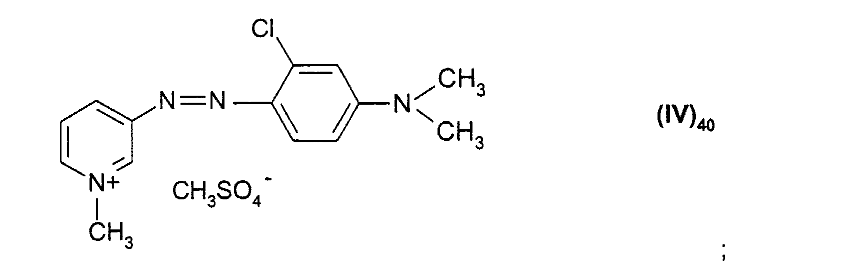 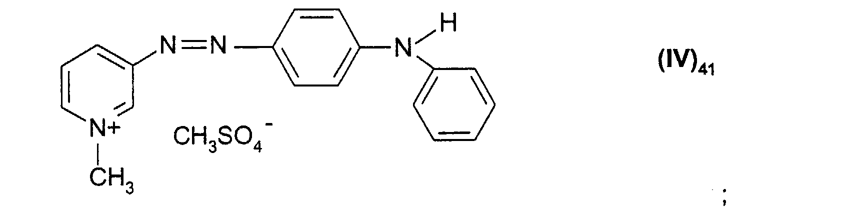 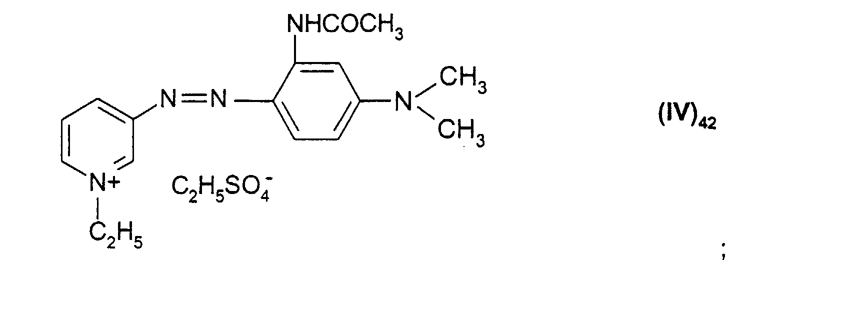 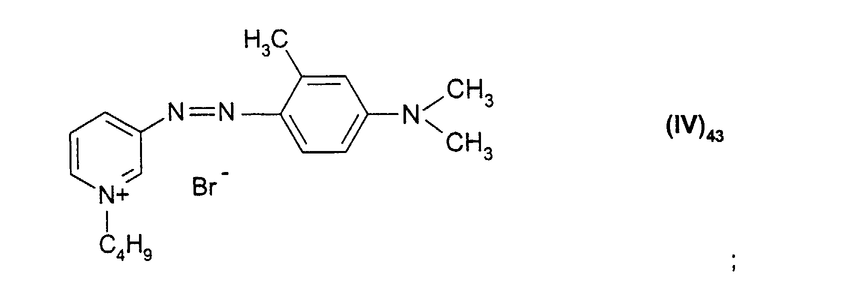 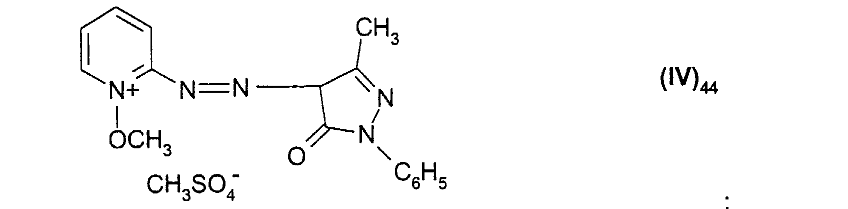 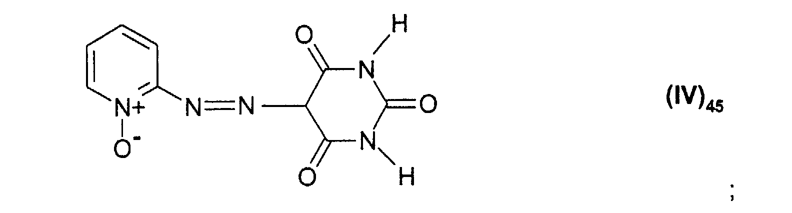 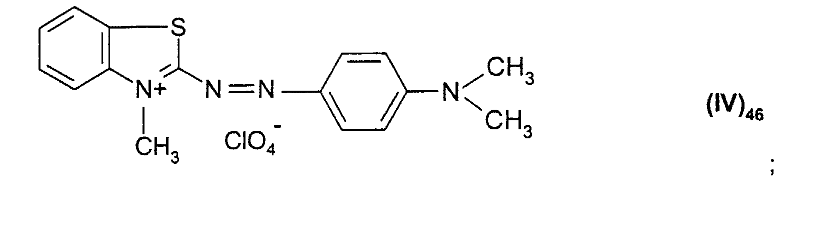 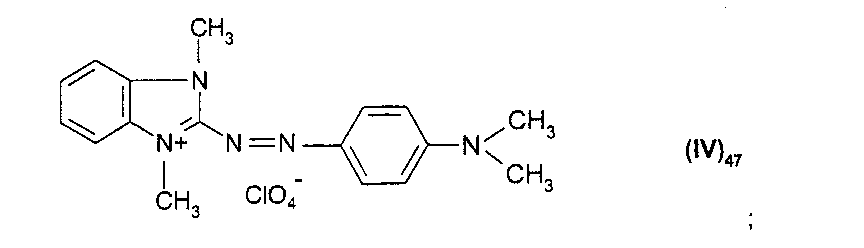 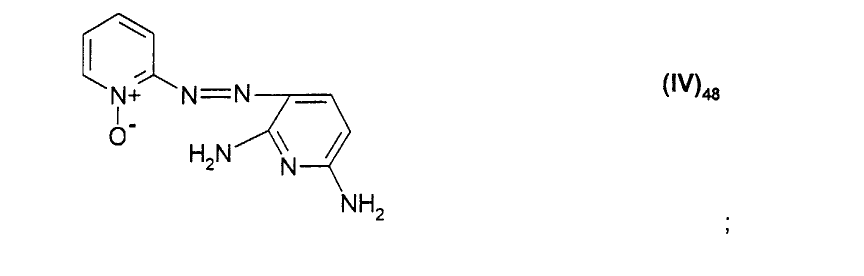 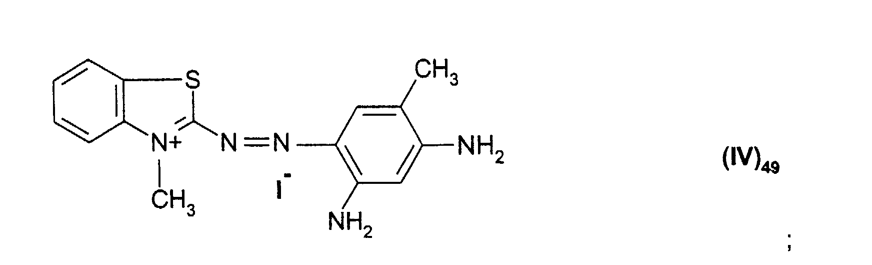 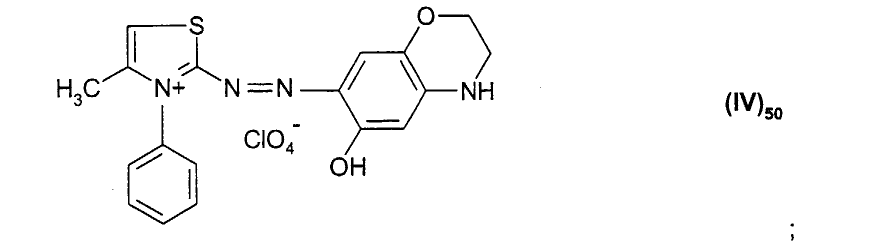 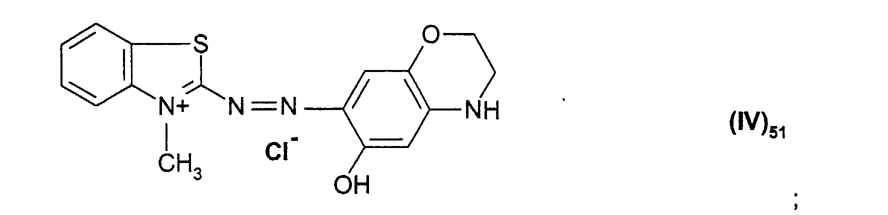 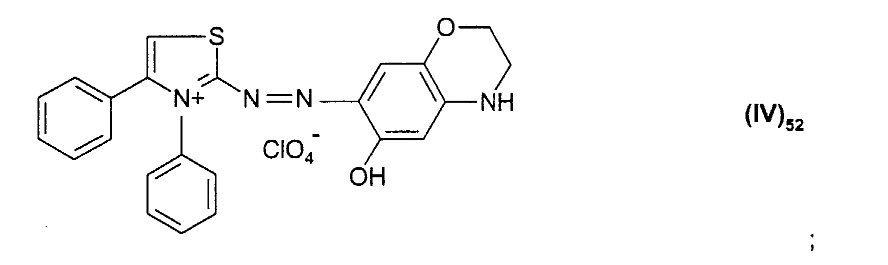 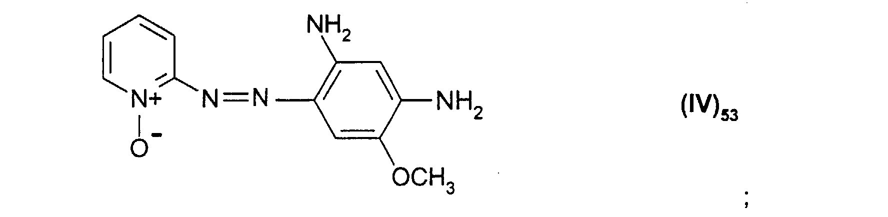 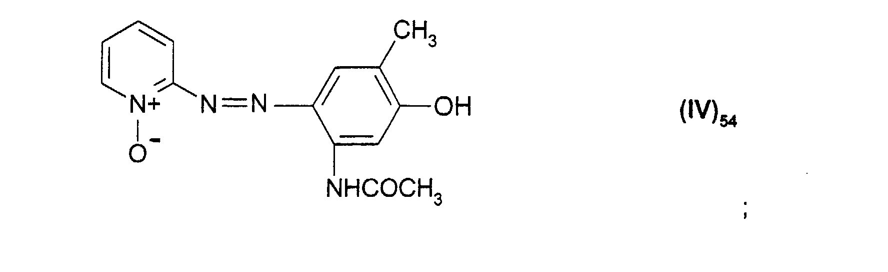 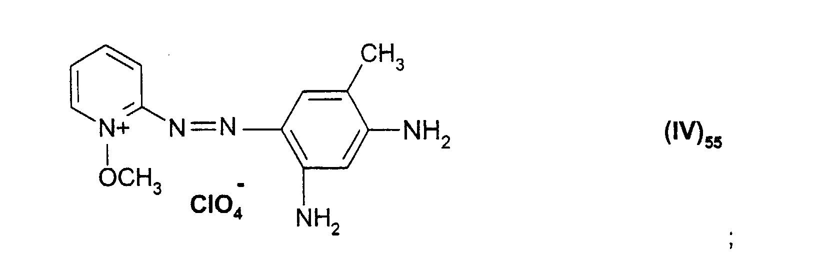 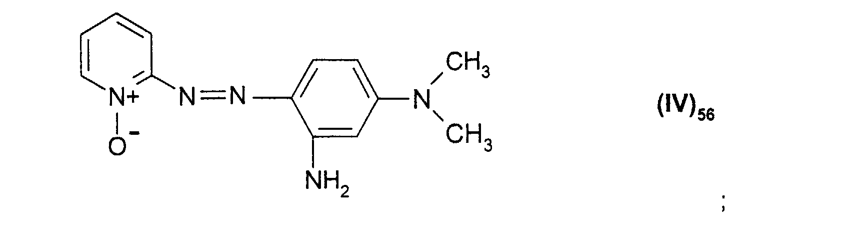 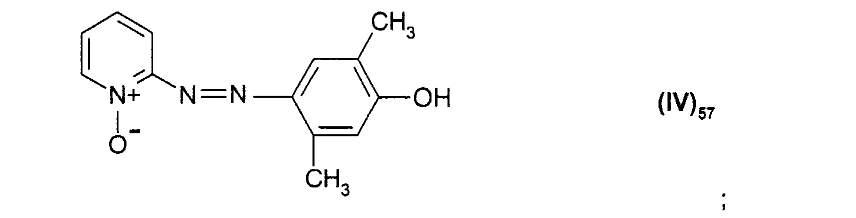 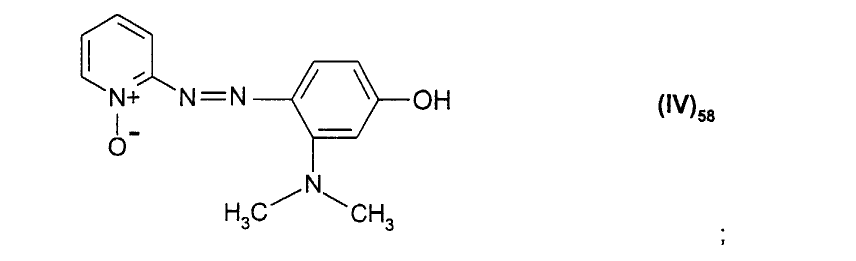 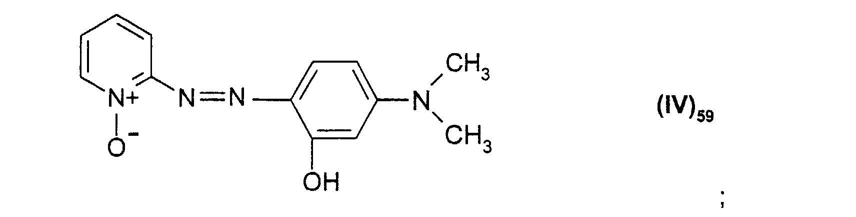 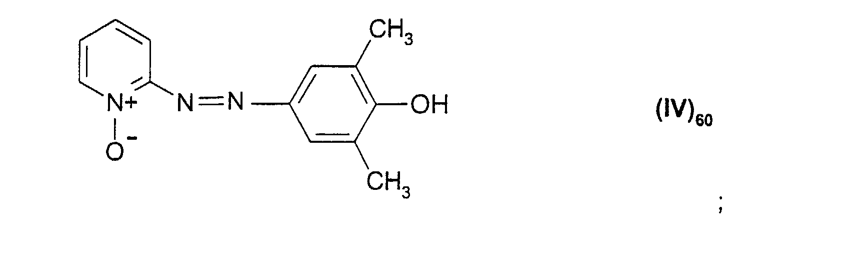 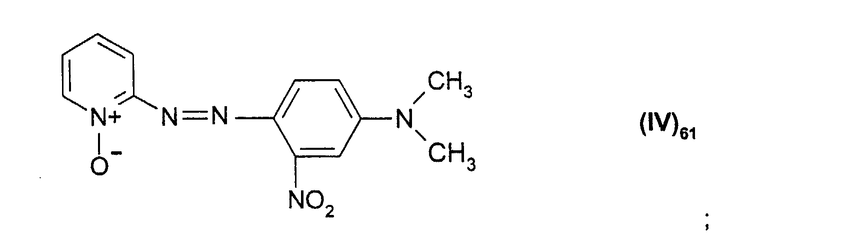 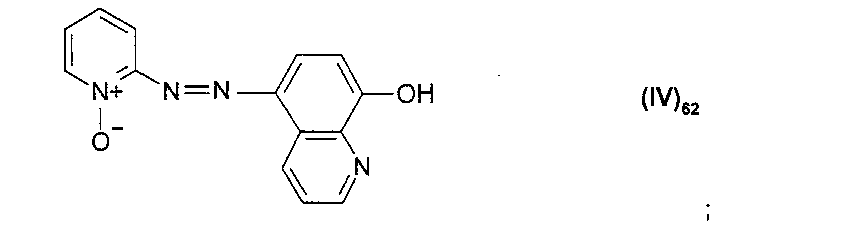 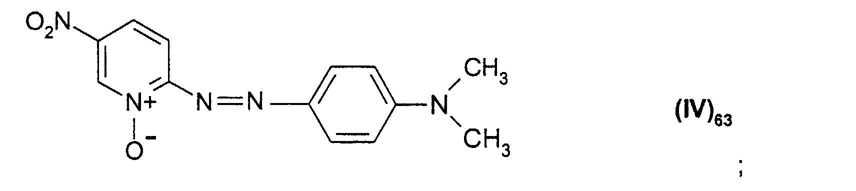 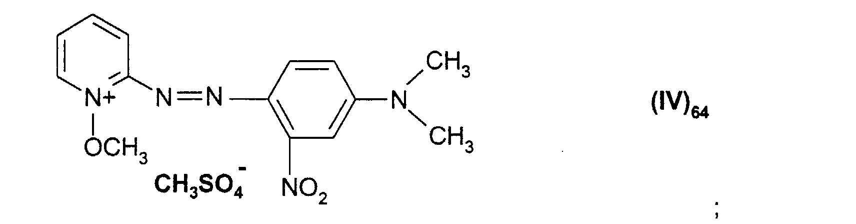 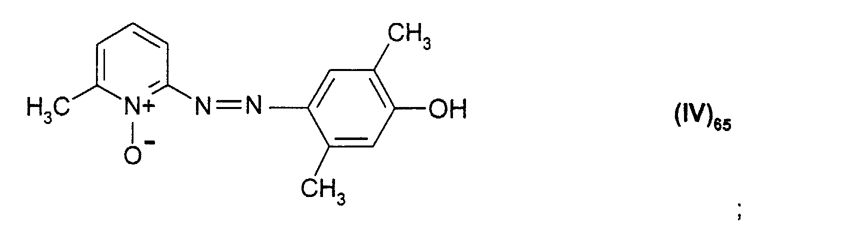 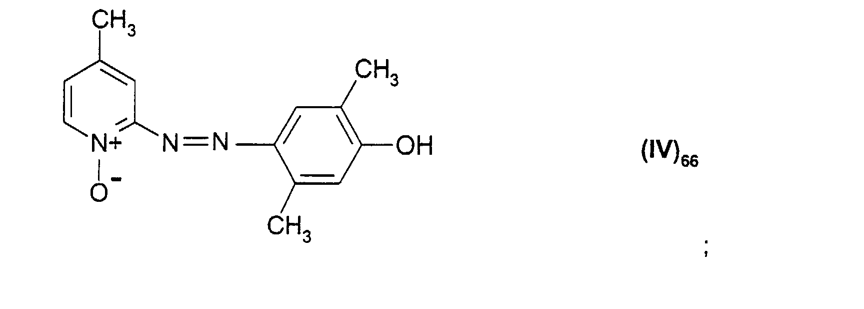 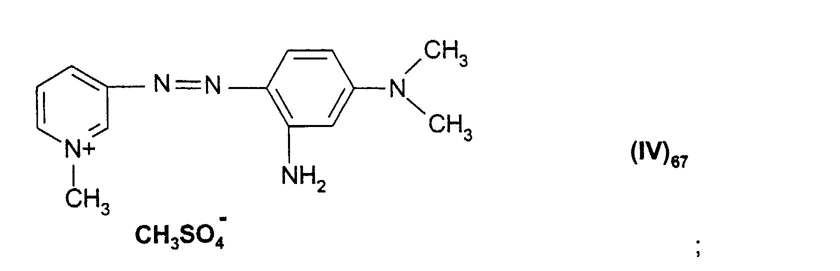 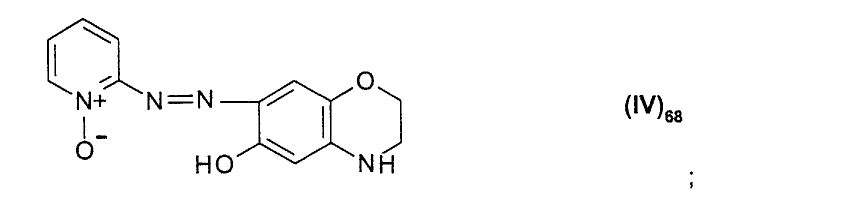 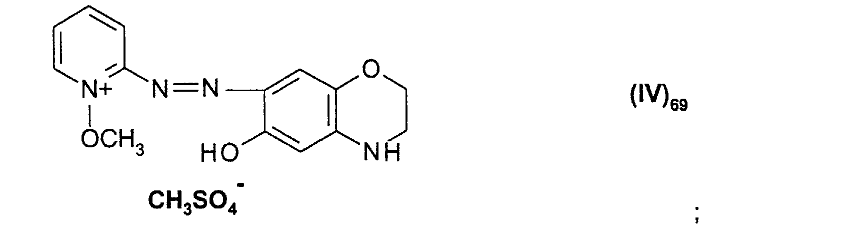 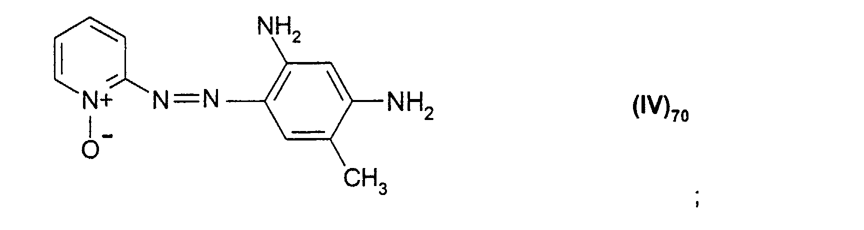 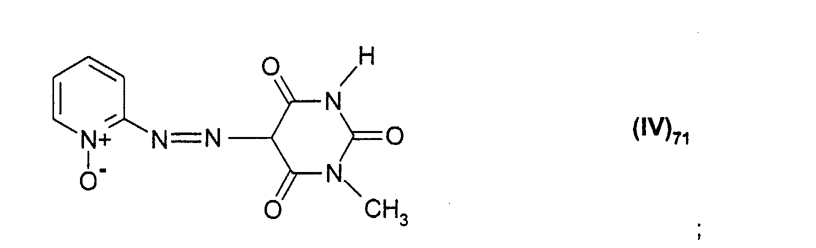 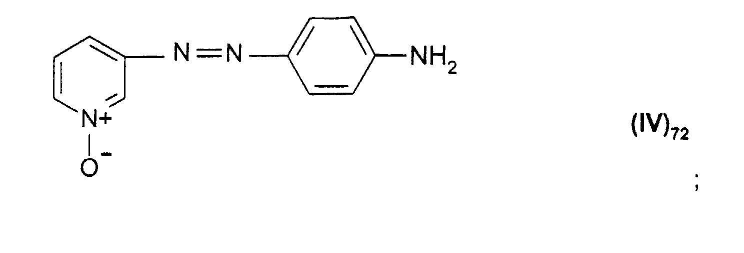 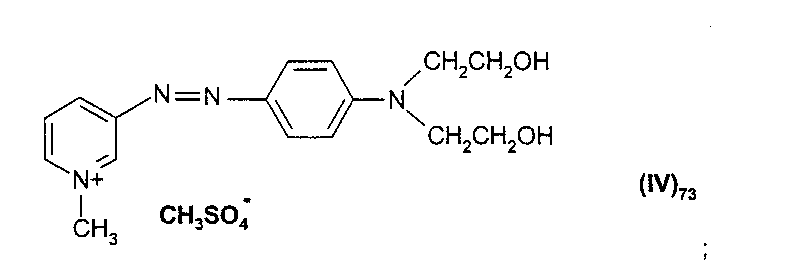 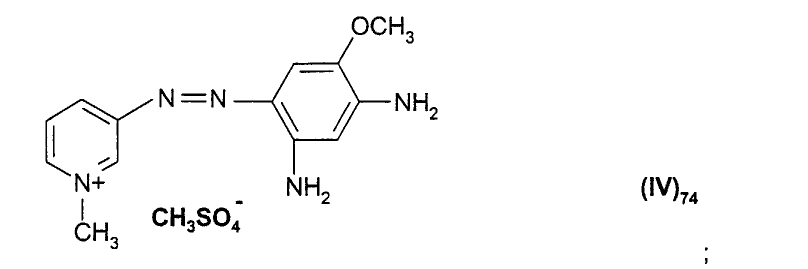 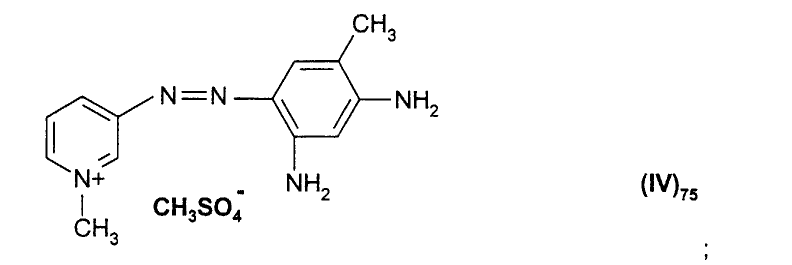 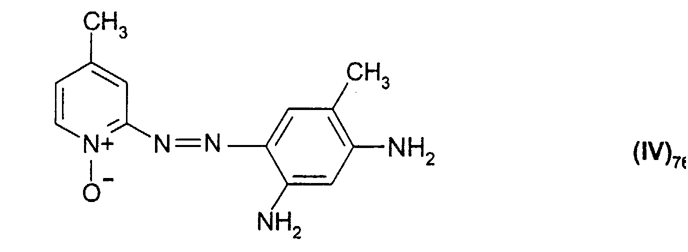 et

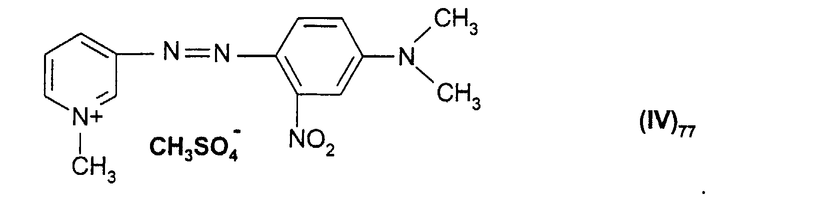

12. Composition selon l'une quelconque des revendications précédentes, caractérisée par le fait que la ou les pyrazolo-[1,5-a]-pyrimidines et/ou le ou leurs sels d'addition avec un acide ou avec une base représentent de 0,0005 à 12 % en poids du poids total de la composition tinctoriale.

13. Composition selon la revendication 12, caractérisée par le fait que la ou les pyrazolo-[1,5-a]-pyrimidines et/ou le ou leurs sels d'addition avec un acide ou avec une base représentent de 0,005 à 6 % en poids du poids total de la composition tinctoriale.

14. Composition selon l'une quelconque des revendications précédentes, caractérisée par le fait que le ou les colorants directs cationiques représentent de 0,001 à 10 % en poids du poids total de la composition tinctoriale.

15. Composition selon la revendication 14, caractérisée par le fait que le ou les colorants directs cationiques représentent de 0,005 à 5 % en poids du poids total de la composition tinctoriale.

16. Composition selon l'une quelconque des revendications précédentes, caractérisée par le fait qu'elle renferme un ou plusieurs coupleurs choisis parmi les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

17. Composition selon l'une quelconque des revendications précédentes, caractérisée par le fait qu'elle renferme une ou plusieurs bases d'oxydation additionnelles différentes d'une pyrazolo-[1,5-a]-pyrimidine et/ou un ou plusieurs colorants directs non cationiques.

18. Composition selon la revendication 17, caractérisée par le fait que la ou les bases d'oxydation additionnelles sont choisies parmi les paraphénylènediamines, les bis-phénylalkylènediamines, les para-aminophénols, les ortho-aminophénols et les bases hétérocycliques.

19. Composition selon l'une quelconque des revendications précédentes, caractérisée par le fait que les sels d'addition avec un acide sont choisis parmi les chlorhydrates, les bromhydrates, les sulfates, les citrates, les succinates, les tartrates, les lactates et les acétates, et que les sels d'addition avec une base sont choisis parmi ceux obtenus avec la soude, la potasse, l'ammoniaque ou les amines.

20. Procédé de teinture d'oxydation des fibres kératiniques et en particulier des fibres kératiniques humaines telles que les cheveux caractérisé par le fait qu'on applique sur lesdites fibres au moins une composition tinctoriale telle que définie à l'une quelconque des revendications 1 à 19, et que l'on révèle la couleur à pH acide, neutre ou alcalin à l'aide d'un agent oxydant non enzymatique qui est ajouté juste au moment de l'emploi à la composition tinctoriale ou qui est présent dans une composition oxydante appliquée simultanément ou séquentiellement.

21. Procédé selon la revendication 20, caractérisé par le fait que l'agent oxydant est choisi parmi le peroxyde d'hydrogène, le peroxyde d'urée, les bromates de métaux alcalins, les peracides et les persels.

22. Dispositif à plusieurs compartiments, ou "kit" de teinture à plusieurs compartiments, dont un premier compartiment renferme une composition tinctoriale telle que définie à l'une quelconque des revendications 1 à 19 et un second compartiment renferme une composition oxydante contenant un agent oxydant non enzymatique.
